# EUROPEAN PATENT SPECIFICATION

(11) **EP 4 058 060 B1**
(45) Date of publication and mention of the grant of the patent: **19.02.2025**
(21) Application number: 20804531.0
(22) Date of filing: 11.11.2020
(51) Int. Cl.: A61K 39/395, A61P 37/00

(54) **TYPE I INTERFERON INHIBITION IN SYSTEMIC LUPUS ERYTHEMATOSUS**
TYP-I-INTERFERON-HEMMUNG BEI SYSTEMISCHEM LUPUS ERYTHEMATODES
INHIBITION DE L'INTERFÉRON DE TYPE I DANS LE LUPUS ÉRYTHÉMATEUX DISSÉMINÉ

(30) Priority: 11.11.2019 US 201962933672 P; 29.05.2020 US 202063031848 P; 08.10.2020 US 202063089345 P
(43) Date of publication of application: 21.09.2022
(60) Divisional application: 24205849.3
(73) Proprietor: Astrazeneca AB, 151 85 Södertälje (SE)
(72) Inventor: BERGLIND, Anna, 151 85 Södertälje (SE); ABREU, Gabriel, 151 85 Södertälje (SE); PINEDA, Lilia, Wilmington, Delaware 19850 (US); TUMMALA, Raj, Wilmington, Delaware 19850 (US); ASKANASE, Anca, New York, NY 10032 (US); RICHEZ, Christophe, 33076 Bordeaux (FR); MORAND, Eric, Clayton, Victoria 3168 (AU); BRUCE, Ian, Manchester M13 9PT (GB); BROHAWN, Philip, Cambridge Cambridgeshire CB2 0AA (GB); FURIE, Richard, Hempstead, NY 11549 (US); BAE, Sang-Cheol, Seoul 04763 (KR); TANAKA, Yoshiya, Kitakyushu, 807-8555 (JP); WERTH, Victoria, Philadelphia, Pennsylvania 19104 (US); VOLLENHOVEN, Ronald Van, 1007 MB Amsterdam (NL); ISENBERG, David, London WC1E 6JF (GB); VITAL, Ed, Leeds LS7 4SA (GB); KAHLENBERG, Michelle, Ann Arbor, Michigan 48109-5422 (US); KALYANI, Rubana, Gaithersburg, Maryland 20878 (US); KALUNIAN, Kenneth, La Jolla, California 92037 (US)
(74) Representative: AstraZeneca Intellectual Property
(86) International application number: PCT/EP2020/081770
(87) International publication number: WO 2021/094378

(56) References cited:
- WO-A1-2017/031288
- WO-A1-2020/165437
- BROHAWN PZ ET AL: "Type I interferon gene signature test-low and -high patients with systemic lupus erythematosus have distinct gene expression signatures", LUPUS, vol. 28, no. 13, 29 October 2019 (2019-10-29), GB, pages 1524 - 1533, XP093057738, ISSN: 0961-2033, Retrieved from the Internet <URL:http://journals.sagepub.com/doi/full-xml/10.1177/0961203319885447> DOI: 10.1177/0961203319885447
- RICHARD FURIE ET AL: "Anifrolumab, an Anti-Interferon-[alpha] Receptor Monoclonal Antibody, in Moderate-to-Severe Systemic Lupus Erythematosus : ANIFROLUMAB IN MODERATE-TO-SEVERE SLE", ARTHRITIS & RHEUMATOLOGY (HOBOKEN), vol. 69, no. 2, 28 January 2017 (2017-01-28), US, pages 376 - 386, XP055652780, ISSN: 2326-5191, DOI: 10.1002/art.39962
- RICHARD FURIE ET AL: "A Phase 3 Randomized Controlled Trial of Anifrolumab in Patients with Moderate to Severe Systemic Lupus Erythematosus", 23 October 2019 (2019-10-23), XP055767135, Retrieved from the Internet <URL:https://acrabstracts.org/abstract/a-phase-3-randomized-controlled-trial-of-anifrolumab-in-patients-with-moderate-to-severe-systemic-lupus-erythematosus/> [retrieved on 20210120]
- ERIC MORAND ET AL: "Efficacy and Safety of Anifrolumab in Patients with Moderate to Severe Systemic Lupus Erythematosus: Results of the Second Phase 3 Randomized Controlled Trial", 23 October 2019 (2019-10-23), XP055767128, Retrieved from the Internet <URL:https://acrabstracts.org/abstract/efficacy-and-safety-of-anifrolumab-in-patients-with-moderate-to-severe-systemic-lupus-erythematosus-results-of-the-second-phase-3-randomized-controlled-trial/> [retrieved on 20210120]

## Description

### FIELD OF THE DISCLOSURE

The disclosure relates to compositions for use in the treatment of Systemic Lupus Erythematosus (SLE). Specifically, the disclosure relates to methods comprising administering to a subject in need thereof a type I IFN receptor inhibitor, namely anifrolumab.

### BACKGROUND

Systemic lupus erythematosus (SLE) is an autoimmune disease that causes significant morbidity and mortality¹. Despite intense SLE clinical trial activity, only one drug, belimumab, has received regulatory approval in the last 60 years. Many factors have contributed to drug development failures in SLE, including trial design challenges, heterogeneous patient populations, and a lack of robust endpoints.

Clinical manifestations of SLE include, but are not limited to constitutional symptoms, alopecia, rashes, serositis, arthritis, nephritis, vasculitis, lymphadenopathy, splenomegaly, hemolytic anemia, cognitive dysfunction and other nervous system involvement. These disease manifestations cause a significant burden of illness and can lead to reduced physical function, loss of employment, lower health-related quality of life (QoL) and a lifespan shortened by 10 years. Increased hospitalizations and side effects of medications including chronic oral corticosteroids (OCS or glucocorticoids and other immunosuppressive treatments add to disease burden in SLE.

Treatment of SLE is challenging because of the limited efficacy and poor tolerability of standard therapy². All of the therapies currently used for the treatment of SLE have well known adverse effect profiles and there is a medical need to identify new targeted therapies, particularly agents that may reduce the requirement for corticosteroids and cytotoxic agents.

**The challenge of finding a treatment for SLE**

The clinical development of a new drug is a lengthy and costly process with low odds of success. For molecules that enter clinical development, less than 10% will eventually be approved by health regulatory authorities³. Furthermore, the clinical development of biotherapeutics, especially monoclonal antibodies, is not quicker or cheaperthan small molecule drugs. The early clinical development of biotherapeutics is much lengthier than for small molecules.

Phase II trials are conducted in a small number of volunteers who have the disease of interest. They are designed to test safety, pharmacokinetics, and pharmacodynamics. A phase II trial may offer preliminary evidence of drug efficacy. However, the small number of participants and primary safety concerns within a phase II trial usually limit its power to establish efficacy. A Phase III trial is required to demonstrate the efficacy and safety of a clinical candidate. Critically, many clinical candidates that have shown promise at Phase II do not succeed at Phase III. More than 90% of novel therapeutics entering Phase I trials fail during clinical development, primarily because of failure in efficacy or safety. The probability of success at phase III, following successful Phase II, is less than 50% ⁴.

The process of drug development is particularly difficult for SLE. This is because SLE is an especially complex and poorly understood disease compared to diseases such as rheumatoid arthritis (RA) and ankylosing spondylitis. Not only is our understanding of the genetics of SLE rudimentary, but our insight into pathogenesis of most of the clinical manifestations are still relatively limited compared to other disease.

The complexity of SLE presents those wishing to develop new therapeutics with the problem of a patient population with extensive inhomogeneity ⁵. This makes protocol design for clinical trials in SLE even more difficult, for example, as regards to the choice of inclusion criteria and primary and secondary endpoints. It is further difficult to predict the disease course in each patient. This inevitably increases the background noise that reduces the statistical power of a trial. A high placebo response rate limits the range in which the tested new drug can show an efficacy signal, making clinical trials even more difficult to conduct and interpret.

The difficulty in developing effective therapeutics for SLE leads to an even higher failure rate of therapeutics in this area in clinical trials, compared to therapeutics for other indications. There has been only 1 new treatment (belimumab) for SLE approved by the US Food and Drug Administration (FDA) and the European Medicines Agency (EMA) in the approximately 50 years since hydroxychloroquine was approved for use in discoid lupus and SLE. However, belimumab is not approved everywhere, and the uptake has been modest. Many agents currently used to treat SLE, such as azathioprine, cyclophosphamide, and mycophenolate mofetil/mycophenolic acid, have not been approved for the disease. Furthermore, these drugs all have well-documents safety issues, and are not effective in all patients, for all manifestations of lupus. Antimalarial agents (e.g. hydroxychloroquine) and corticosteroids may be used to control arthralgia, arthritis, and rashes. Other treatments include nonsteroidal anti-inflammatory drugs (NSAIDs); analgesics for fever, arthralgia, and arthritis; and topical sunscreens to minimize photosensitivity. It is often difficult to taper subjects with moderate or severe disease completely off OCS, which cause long-term morbidity and may contribute to early cardiovascular mortality. Even small daily doses of 5 to 10 mg prednisone used long-term carry increased risks of side effects such cataracts, osteoporosis, and coronary artery disease.

Nonsteroidal anti-inflammatory drugs (NSAIDs) are commonly used for the symptomatic management of arthralgia, mild arthritis, myalgia, serositis and fever in patients with SLE. They do not have any immunosuppressive properties. NSAIDs can only be used for short periods of time and are not suitable for patients with renal involvement, hypertension and established heart disease. NSAIDs can cause fluid retention, renal impairment and interstitial nephritis.

Mycophenolate mofetil (MMF) is a specific inhibitor of inosine monophosphate dehydrogenase. MMF impairs *de novo* purine synthesis. Inosine monophosphate dehydrogenase is an essential pathway in activated lymphocytes. MMF thus inhibits both T and B lymphocyte proliferation and reduces antibody synthesis. A randomised blinded multicentre international trial of 370 SLE patients with active nephritis randomly assigned to receive MMF (2-3 g a day) or standard intravenous cyclophosphamide therapies for 6 months, the efficacy of MMF was similar to cyclophosphamide, which had been standard of care before the study. However, there were significantly different responses in African American, Hispanic and Latin American individuals⁶.

The development of novel therapeutics for the treatment of SLE has thus proved extremely difficult. There are many examples of clinical candidates that showed promise at Phase II but failed to show efficacy and/or safety in subsequent Phase or Phase III trials.

### Tabalumab

Tabalumab (LY2127399) is a human IgG4 monoclonal antibody that binds both soluble and membrane-bound B-cell activating factor (BAFF). The efficacy and safety of tabalumab was assessed in two 52-week, phase III, multicenter randomized, double-blind, placebo-controlled trial in patients with moderate-to-severe SLE (ILLUMINATE-1 and ILLUMINATE-2). The primary endpoint was proportion of patients achieving SLE Responder Index 5 (SRI-5) response at week 52.

In ILLUMINATE-1 (NCT01196091), the primary endpoint was not met. Key secondary efficacy endpoints (OCS sparing, time to severe flare, worst fatigue in the last 24 hours) also did not achieve statistical significance, despite pharmacodynamic evidence of tabalumab biological activity (significant decreases in anti-dsDNA, total B-cells, and immunoglobulins)⁷.

The primary endpoint was met in ILLUMINATE-2 (NCT01205438) in the higher dose group (tabalumab 120mg every 2 weeks). However, no secondary endpoints were met, including OCS sparing⁸. Following ILLUMINATE-1 and ILLUMINATE-2, tabalumab development was suspended given the small effect size and inability to meet other important clinical endpoints.

### Blisibimod

Blisibimod is a fusion protein composed of four BAFF-binding domains fused to the N-terminal Fc fragment of human IgG1 Ig. Blisibimod for the treatment of SLE had promising Phase II results but was unsuccessful in Phase III.

In a phase 2 double-blind, randomized, placebo-controlled clinical trial (PEARL-SC), patients with serologically active SLE and SELENA-SLEDAI score ≥6 points were randomized to 3 different doses of blisibimod or placebo (NCT01162681). At week 24, the highest dose group (200 mg once weekly) had a significantly higher SRI-5 response rate than the placebo group⁹.

However, in a subsequent placebo-controlled, phase III randomized, double-blind study (CHABLIS-SC1) conducted on seropositive SLE patients with persistent high disease activity (SELENA-SLEDAI ≥10 points) the primary endpoint (SRI-6) was not met (NCT01395745). The secondary end points (SRI-4 and SRI-8) were also not reached ¹⁰.

### Atacicept

Atacicept (TACI-Ig) is a fully human recombinant fusion protein that neutralizes both BAFF and APRIL. The efficacy of atacicept for the treatment of SLE was evaluated in two phase II/III placebo randomized controlled trials (APRIL-LN and APRIL-SLE). The APRIL-LN trial compared renal response to atacicept versus placebo plus standard of care (newly initiated MMF and glucocorticoids) in patients with SLE nephritis. The trial was discontinued after serious adverse events were reported.

In APRIL-SLE the primary end point, defined as a significantly decreased proportion of patients who developed a new flare from BILAG A or BILAG B domain scores, was not met in the lower dose (75mg) arm (NCT00624338). Treatment of patients with the higher dose (150mg) arm was discontinued due to serious AEs¹¹.

### Abetimus

Abetimus (LJP 394) comprises four synthetic oligodeoxynucleotides attached to a triethyleneglycol backbone, where more than 97% of these oligonucleotides are derived from dsDNA. The drug was designed to neutralize anti-dsDNA antibodies. In a double-blind, placebo-controlled study in SLE patients, treatment with LJP 394 in patients with high-affinity antibodies to its DNA epitope prolonged the time to renal flare, decreased the number of renal flares¹². However, in a subsequent Phase III trial (NCT00089804) using higher doses of abetimus, with a primary endpoint of time to renal flare, study and further drug development was discontinued when interim analysis failed to show efficacy¹³.

### Rituximab

Rituximab is a chimeric anti-CD20 monoclonal antibody. Rituximab is an effective treatment in a number of autoimmune disease, including rheumatoid arthritis and ANCA vasculitis. A small number of uncontrolled trials in lupus nephritis suggested that rituximab could also be potentially effective in patients wit lupus nephritis. Efficacy and safety of rituximab was assessed in a randomized, double-blind, placebo-controlled phase III trial in patients with lupus nephritis treated concomitantly with mycophenolate mofetil (MMF) and corticosteroids (LUNAR) (NCT00282347). Rituximab therapy did not improve clinical outcomes after 1 year of treatment¹⁴.

The efficacy and safety of rituximab in patients with moderate to severe SLE was evaluated in a multicenter placebo randomized controlled phase II/III trial (EXPLORER). The study randomized patients with baseline active SLE (defined as ≥1 new BILAG A scores or ≥2 BILAG B scores) to rituximab or placebo. The primary endpoint was the proportion of rituximab versus placebo-treated patients achieving a complete clinical response (CCR), partial clinical response (PCR), or no response at week 52. The primary endpoint was not met, with similar rates of complete and partial responses in rituximab and placebo arms at 52 weeks. Differences in time to first moderate or severe flare and change in HRQOL were also not significant¹⁵.

### Abatacept

Abatacept is a CTLA-4 fusion protein that binds to CD80/86 on the surface of antigen presenting cells and blocks signaling through CD-28 required for T-cell activation. In preclinical studies abatacept was demonstrated to have immunomodulatory activity in the NZB/NZW murine model of lupu¹⁶. Abatacept for treatment of non-renal SLE was been evaluated in a phase lib, randomized, double-blind, placebo-controlled trial¹⁷ (NCT00119678). The primary end point was the proportion of patients with new flare (adjudicated) according to a score of A/B on the British Isles Lupus Assessment Group (BILAG) index after the start of the steroid taper. The primary and secondary end points were not met.

### Epratuzumab

Epratuzumab is a monoclonal antibody that modulates B-cell activity by binding CD22 on the surface of mature B-cells. Epratuzumab initially demonstrated efficacy in treating SLE at phase II trial but this was not confirmed in a follow-up second phase Ilb trial or the subsequent phase III trial.

Two phase IIb trials assessed the efficacy of epratuzumab with a BILAG-based primary endpoint in patients with moderate-to-severe SLE (ALLEVIATE 1 and 2). A trend towards clinical efficacy was observed and the primary end point was met by more patients treated with epratuzumab than placebo. Epratuzumab treatment also led to improvements in Health-related quality of life (HRQOL) and mean glucocorticoid dose¹⁸.

In another phase IIb trial (EMBLEM), patients with moderate-to-severe SLE were randomized to one of five epratuzumab doses or placebo. BICLA response at 12 weeks, the primary endpoint, was greater with all doses of epratuzumab than placebo, but the effect was not statistically significant.

In the subsequent multicenter phase III trials EMBODY 1 and EMBODY 2, patients with moderate-to-severe SLE, the primary efficacy endpoint, BICLA response at 48 weeks, was not met. No significant differences were seen in secondary endpoints such as total SLEDAI-2K score, PGA, or mean glucocorticoid dose ¹⁹.

### PF-04236921

PF-04236921 is a monoclonal antibody that binds soluble IL-6, a cytokine that is elevated in SLE patients. The efficacy of PF-0436921 was evaluated in a phase II RCT of patients with active SLE (BUTTERFLY) (NCT01405196). Patients were randomized to receive either subcutaneous PF-04236921 10mg, 50mg, or 200mg or placebo every 8 weeks; the 200mg dose arm was discontinued early because of 3 deaths. The primary efficacy endpoint was SRI-4 response at 24 weeks, with BICLA as a secondary endpoint. The primary endpoint was not met²⁰.

### Type I IFN inhibition and anifrolumab

Type I interferons (IFN) are cytokines that form a crucial link between innate and adaptive immunity and are implicated in SLE by genetic susceptibility data and upregulated interferon-stimulated gene expression in the majority of SLE patients²¹.

Attempts to target the type I IFN pathway in order to treat SLE have not previously been successful. Rontalizumab is a humanised IgG1 anti-interferon α (anti-IFN-α) monoclonal antibody. A phase II study to the efficacy and safety of rontalizumab in patients with moderate-to-severe SLE was preformed (ROSE) (NCT00962832). Patients with active SLE were randomised (2:1) to 750 mg intravenous rontalizumab every 4 weeks or placebo (Part 1), and 300 mg subcutaneous rontalizumab every 2 weeks or placebo (Part 2). The primary and secondary end points of this trial were not met in all patients²². The development of Rontzlizumab was therefore discontinued.

Sifalimumab is an anti-interferon-a monoclonal antibody. The efficacy and safety of sifalimumab were assessed in a phase lib, randomised, double-blind, placebo-controlled study of adults with moderate to severe active systemic lupus erythematosus (SLE) (NCT01283139) met primary and some secondary end points, but the treatment effects were modest. Furthermore, the complement levels and anti-dsDNA levels were not significantly affected by sifalimumab.

Anifrolumab (MEDI-546) a monoclonal antibody which binds to IFNAR. In a phase II clinical trial, anifrolumab reduced disease activity compared to placebo in patients with moderate to severe SLE ²³. The safety and efficacy of anifrolumab to treat SLE was subsequently assessed in two phase III clinical trials, TULIP I and TULIP II. However, TULIP I failed to meet its primary endpoint^{24,25}.

### Conclusion

There is a huge unmet need for an SLE therapy with a better efficacy and safety profile the currently available therapies ^{26,27}. As described above, a large number and broad range of different biologics have been proposed and subjected to clinical trials, but these trials have failed to meet clinical meaningful endpoints in pivotal studies. Initial promise at Phase II of many proposed therapeutics was not translated into significant and meaningful clinical effect in subsequent pivotal Phase III clinical trials.

Therefore, there remains the need for safe and effective treatment of SLE that has proven clinical benefit, for example in a phase III double-blind, randomized, placebo controlled trial²⁸. The present invention solves one or more of the above-mentioned problems, by providing a safe and efficacious treatment for SLE wherein the treatment safety and efficacy has been demonstrated.

### SUMMARY

The present invention relates to a type I IFN receptor (IFNR) inhibitor for use in a method of treating systemic lupus erythematosus (SLE) in a subject in need thereof, the method comprising administering to the subject a therapeutically effective amount of a type I interferon (IFN) receptor (IFNR) inhibitor, wherein the IFNR inhibitor reduces SLE disease activity in the subject, wherein at baseline the subject has a low type I IFN gene signature (IFNGS), wherein the IFNGS of the subject at baseline is measurable by a 4-gene quantitative polymerase chain reaction-based test, wherein the test measures IFI27, IFI44, IFI44L and RSAD2 expression in the whole blood of the subject, and wherein the IFNR inhibitor is anifrolumab.

The ability of the IFNR inhibitor to reduce SLE disease activity in a subject may have been demonstrated in a phase III clinical trial, particularly a double-blind, randomized, placebo-controlled trial.

The present invention is based on the surprising results of the TULIP II phase III double-blind, randomized, placebo-controlled trial of anifrolumab. TULIP-2 surprisingly met its primary and secondary points. Compared with placebo, anifrolumab treatment resulted in significantly greater rates of improvement across a broad range of composite and organ-specific disease activity measures as well as in the achievement and maintenance of low disease activity, corticosteroid tapering and a trend toward flare rate reduction. TULIP II is the first successful phase III trial in SLE since the trial for belimumab program more than 10 years ago. Not only did TULIP II meet its primary end point, but anifrolumab was demonstrated to have a surprisingly early and sustained effect in SLE patient, particularly as compared to the only approved biologic for the treatment of SLE, belimumab. Furthermore, administration with anifrolumab permitted tapering of OCS dose in SLE patients without a worsening in SLE disease activity. Treatment with belimumab does not support OCS tapering ²⁹.

The successful TULIP II trial follows earlier reporting of the failure of the preceding TULIP I trial (NCT02446912). TULIP-1 failed to meet its primary end point ((SRI)4). However, analysis of the TULIP I data demonstrated efficacy of anifrolumab in pre-specified secondary end points, including BICLA response. Following a careful analysis of the TULIP-1 data and before unblinding of TULIP-2 trial data, the present inventors changed the primary outcome measure for TULIP-2 from SRI4 to BICLA in a protocol amendment. BICLA, in contrast to SRI4, reflects presence of disease activity related to a lupus manifestation rather than severity of a manifestation. The use of BICLA allowed for the assessment of incremental response rather than requiring more complete resolution of a disease manifestation. Reducing SLE disease activity in the subject in the method of the invention may comprise one or more of the following:
- a BILAG-Based Composite Lupus Assessment (BICLA) response in the subject,
- reducing the subject's Cutaneous Lupus Erythematosus Disease Area and Severity Index (CLASI) score compared to the subject's CLASI score pre-treatment,
- reducing the subject's tender and swollen joint count compared to the subject's tender and swollen joint count pre-treatment,
- the subject having a maximum of 1 BILAG-2004 B score following treatment,
- the subject having a BILAG-2004 score of C or better following treatment,
- the subject having an improvement in at least one patient reported outcome (PRO) compared to pre-treatment,
- reducing the subject's SLE flare rate compared to the subject's flare rate pre-treatment.

The method may comprise measuring the subject's BILAG score before and after administration of the IFNAR inhibitor. The BICLA response may be sustained in the subject for at least 52 weeks. The method may comprise measuring PROs in the subject before and after administration of the IFNAR inhibitor. The PRO's may comprise the subject's Functional Assessment of Chronic Illness Therapy-Fatigue (FACIT-F), Short Form 36 Health Survey version 2 (SF-36-v2), mental component summary (MCS), and/or SF-36, physical component summary (PCS) score.

The BICLA response may comprises reduction of the subject's BILAG-2004 A and B domain scores to B/C/D and C/D, respectively.

Reducing the subject's CLASI score compared to the subject's CLASI score pre-treatment may comprise a reduction in the subject's CLASI-A score compared to the subject's CLASI-A score pre-treatment. Reducing the SLE disease activity in the subject may comprise reducing the anti-dsDNA levels in the subject. Reducing SLE disease activity in the subject may comprise a BILAG-Based Composite Lupus Assessment (BICLA) response. The method may comprise reducing the OCS dose administered to the subject compared to the OCS dose administered to the subject pre-treatment

The OCS may comprise prednisone, prednisolone and/or methylprednisolone.

Reducing SLE disease activity in the subject may comprise a BILAG-Based Composite Lupus Assessment (BICLA) response by at least week 4 of treatment.

Reducing SLE disease activity may comprise a BILAG-Based Composite Lupus Assessment (BICLA) response by at least week 8 of treatment.

Reducing SLE disease activity in the subject may comprise at least a 50% improvement in the tender joint count and swollen joint count in the subject compared to the tender joint and swollen count in the subject pre-treatment value.

Reducing SLE disease activity in the subject may not comprise an improvement in the Systemic Lupus Erythematosus Responder Index (SRI)4 score.

Reducing SLR disease activity in the subject may not comprise an improvement in the Systemic Lupus Erythematosus Responder Index (SRI)4 score following 52 weeks of treatment.

Reduction in the subject's CLASI score may be achieved by at least week 8 of treatment.

Reduction in the subject's CLASI score may be achieved following 12 weeks of treatment.

Reducing SLE disease activity in the subject may comprise at least 50% reduction in the subject's CLASI score compared to the subject's CLASI score pre-treatment.

Reducing SLE disease activity in the subject may comprise reduction of the subject's CLASI-A score following 12 weeks of treatment.

The subject may have a CLASI-A score of ≥10 pretreatment.

Reducing SLE disease activity in the subject may comprise the subject's BILAG-2004 score being C or better after 24 weeks of treatment.

Reducing SLE disease activity in the subject may comprise the subject having a maximum of 1 BILAG-2004 B score after 24 weeks of treatment.

Reducing SLE disease activity in the subject may comprise a reduction in the subject's BILAG-based annualized flare rate compared to the subject's BILAG-based annualized flare rate pre-treatment. Reducing SLE disease activity in the subject may comprise preventing flares in the subject.

A flare may be defined as ≥1 new BILAG-2004 A or ≥2 new (worsening) BILAG-2004 B domain scores compared to the subject's scores one month previously.

Reducing SLE disease activity in the subject may comprise a reduced flare rate in the subject compared to the flare rate pre-treatment, wherein the method comprises reducing OCS dose administration to the subject compared to the OCS dose administered to the subject pre-treatment.

The method may comprise selecting the subject for treatment, wherein the subject is selected for having active SLE. The method may comprise selecting the subject for treatment, wherein the subject is selected for having moderate to severe SLE. The subject may be selected for having SLE that is unresponsive to OCS treatment.

The subject may be an adult. The subject may be a human adult. The subject may be a human adult with moderate to severe SLE. The subject may be a human adult with active SLE.

The present disclosure relates to a method for reducing OCS dose in a subject having an autoimmune disease comprising administering to the subject a therapeutically effective amount of a type I IFN receptor inhibitor and reducing the OCS dose administered to the subject compared to the OCS dose administered to the subject pre-treatment, wherein the IFNR inhibitor reduces disease activity in the subject.

The present disclosure also relates to a method for preventing OCS-associated organ damage in a subject having an autoimmune, wherein the subject is receiving OCS pre-treatment, the method comprising administering to the subject a therapeutically effective amount of a type I IFN receptor inhibitor and reducing the OCS dose administered to the subject compared to the OCS dose administered to the subject pre-treatment, wherein the IFNR inhibitor reduces disease activity in the subject.

The subject may be receiving an OCS dose of about ≥ 10 mg/day at the start of treatment. The OCS may be reduced to about ≤ 7.5 mg/day. The OCS may be reduced to ≤ 7.5 mg/day. The OCS dose may be reduced to about 0 mg/day. The OCS dose may be reduced to 0 mg/day. The method may comprise sustaining the reduced OCS dose for at least 12 weeks.

The subject may have OCS associated organ damage. The OCS may comprise prednisone, prednisolone and/or methylprednisolone. The autoimmune disease may be SLE or moderate to severe SLE. The subject may be selected for having SLE that is unresponsive to OCS treatment.

The type I IFN receptor inhibitor may be administered intravenously.

Type I IFN receptor inhibitor is an antibody as defined in claim 1.

The antibody may be anifrolumab.

The method may comprise administering 300 mg anifrolumab, optionally wherein anifrolumab as an intravenous (IV) infusion over a 30-minute period. Anifrolumab may be administered as an IV infusion over a 30-minute period, every 4 weeks. Anifrolumab may be administered from a single-dose vial. Anifrolumab may be provided in a solution at a concentration of 150mg/mL. Anifrolumab may be administered every four weeks. Anifrolumab may be administered for at least 52 weeks.

The present disclosure also relates to a pharmaceutical composition for use in a method of treatment of the invention, wherein the pharmaceutical composition comprises the type I IFN receptor inhibitor.

The present disclosure also relates to the use of a pharmaceutical composition in the manufacture of a medicament for the treatment of SLE, wherein the pharmaceutical composition comprise a type I IFN receptor inhibitor.

The pharmaceutical composition may comprise anifrolumab at a concentration of 150 mg/mL.

The pharmaceutical composition may comprise
- 150 mg/mL anifrolumab;
- 50 mM lysine HCl;
- 130 mM trehalose dihydrate;
- 0.05% polysorbate 80;
- 25 mM histidine/histidine HCl,
wherein the pharmaceutical composition is at a pH of 5.9.

The present disclosure also relates to a unit dose for use in the method of the invention, wherein the unit dose comprises 300 mg anifrolumab.

The present invention also relates to a kit for use in the method of the invention, wherein the kit comprises:
- the unit dose of the invention, and
- a glass vial containing the unit dose.

The kit may comprise instructions for administration of the unit dose. The instructions may specify administration of the unit dose every 4 weeks. The instructions may specify that the subject has moderate to severe SLE.

The present disclosure also relates to an improved method for assessing the effect of a treatment for SLE on patient reported outcomes (PROs) of treatment in a subject, comprising measuring the subject's BICLA response, wherein the BICLA response corresponds to an improvement in patient reported outcomes (PRO) of treatment in the subject. The PROs may comprise the subject's Functional Assessment of Chronic Illness Therapy-Fatigue (FACIT-F), Short Form 36 Health Survey version 2 (SF-36-v2), mental component summary (MCS), and/or SF-36, physical component summary (PCS) score.

### BRIEF DESCRIPTION OF THE DRAWINGS

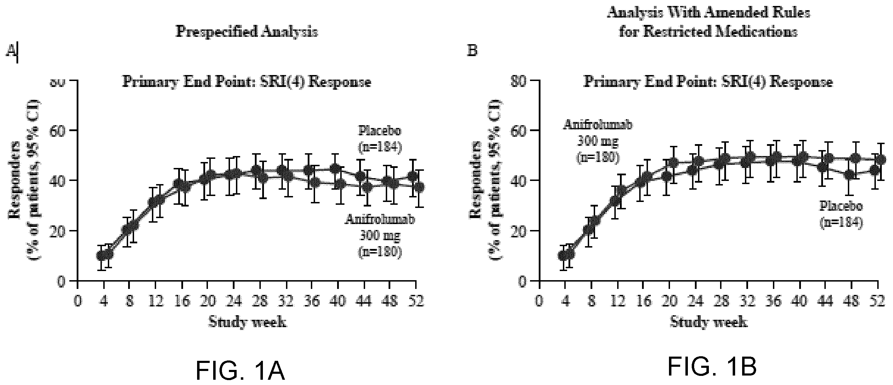

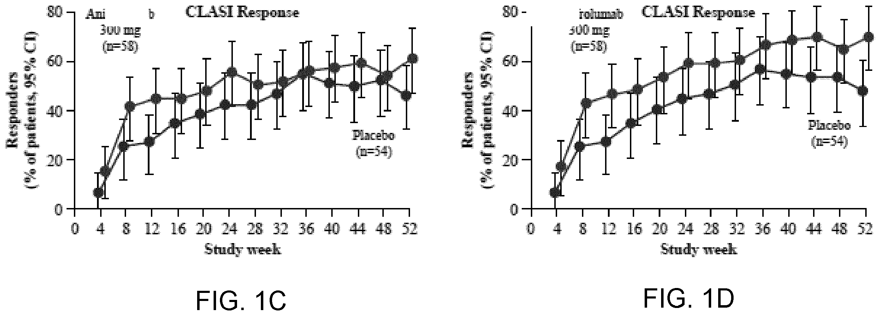

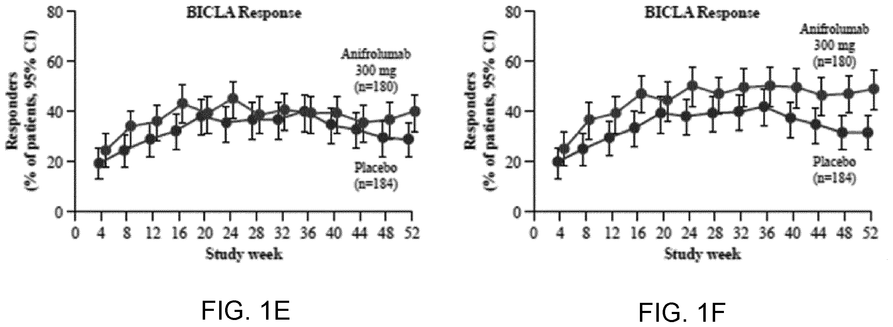

### FIG 1: Primary and selected secondary efficacy outcomes over time in TULIP-1

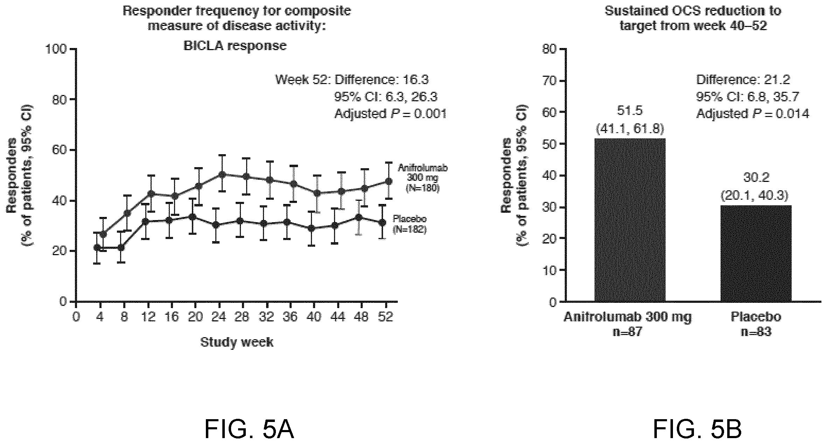

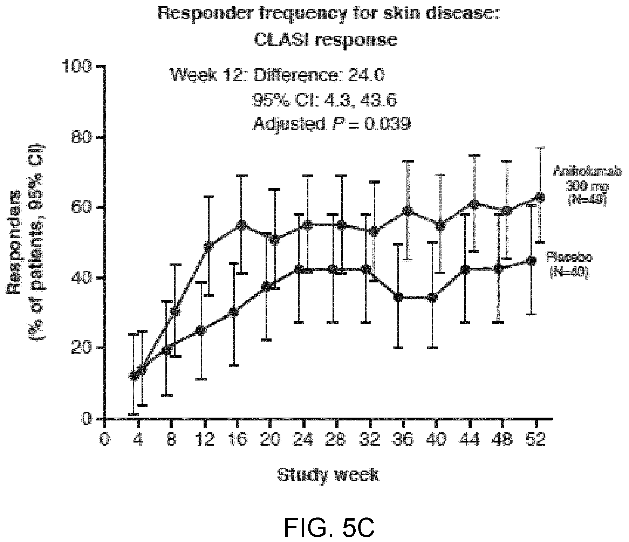

**FIG's 1A-1F** illustrate primary and selected secondary efficacy outcomes overtime in TULIP-1. **FIG 1A** and **FIG 1B** show the primary endpoint: Percentage of patients with SRI(4) response. **FIG 1C** and **FIG 1D** show percentage of patients with ≥50% reduction in CLASI activity score from baseline, in patients with CLASI score ≥10 at baseline. **FIG 1E** and **FIG 1F** show percentage of patients with BICLA response.

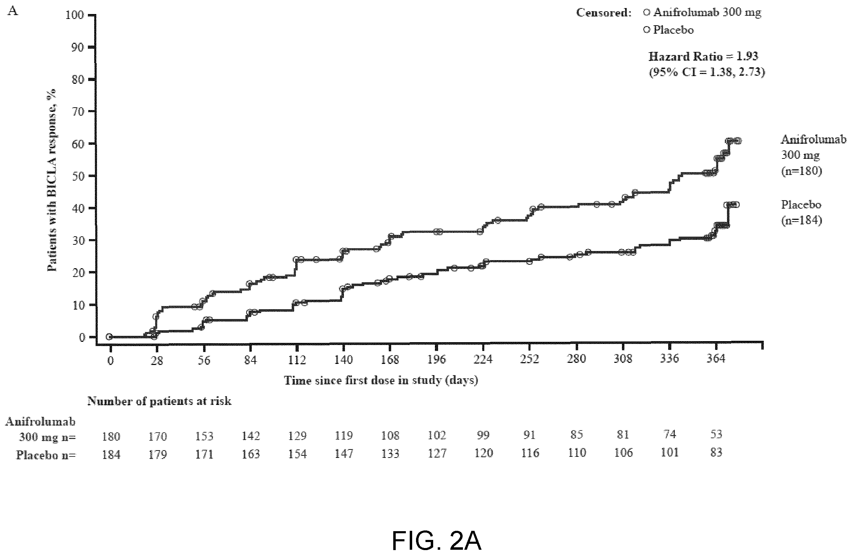

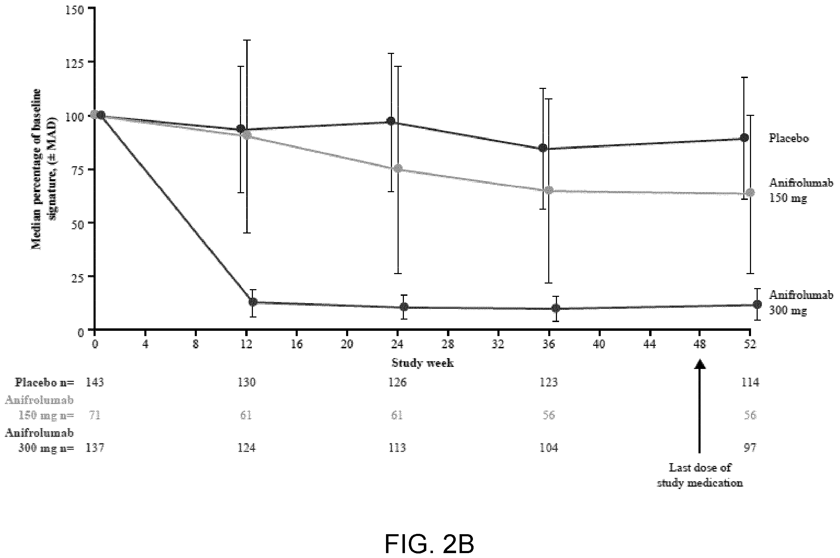

**FIG 2****: BICLA response and type I IFNGS over time in TULIP I**
**FIG's 2A-2B** illustrate the BICLA response and Type I IFNGS suppression over time in TULIP-1. **FIG 2A** shows the Kaplan-Meier plot of time to onset of BICLA response that was sustained to week 52. Patients without a BICLA response sustained up to week 52 are censored at the date of discontinuation of study treatment or week 52, whichever occurred earlier. **FIG 2B** shows the median percentage of baseline type I IFN pharmacodynamic (21-gene) signature at each visit among patients with high type I IFNGS (fold change >2) at baseline.

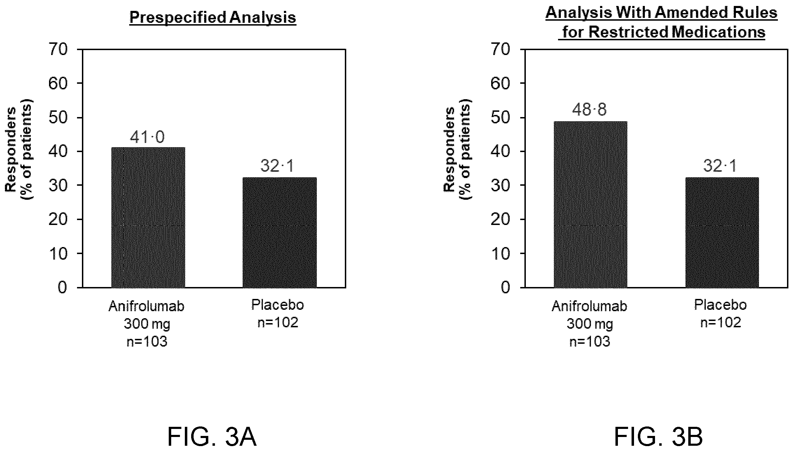

**FIG 3****: OCS reduction**
**FIG's 3A** and **3B** show the sustained oral corticosteroid dosage reduction to a target of ≤7.5 mg/day from Week 40 Through Week 52 in patients with corticosteroid dosage ≥10 mg/day at baseline. **FIG 3A** shows results using prespecified restricted medication rules for determining nonresponse and **FIG 3B** shows the analysis with the amended restricted medication rules.

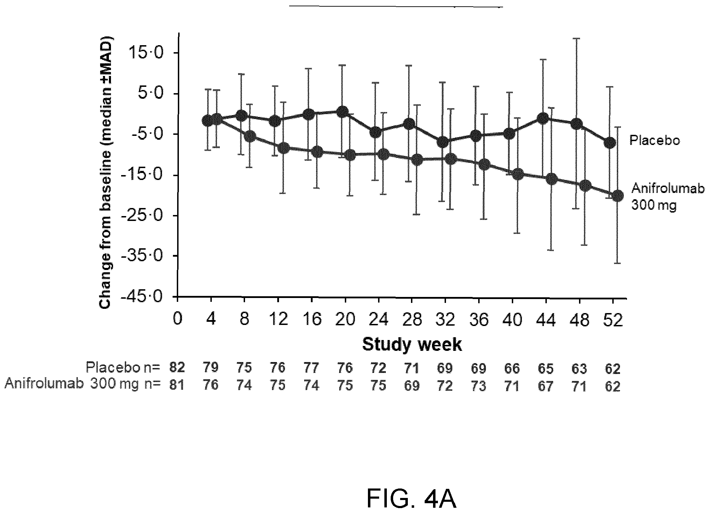

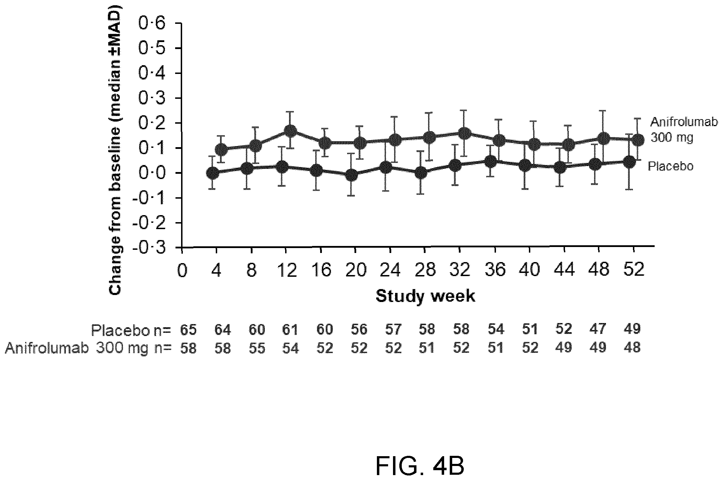

**FIG 4****: anti-dsDNA antibodies and C3 concentration in TULIP I**
**FIG's 4A-4B** illustrate the change from baseline over time in **(****FIG 4A****)** anti-dsDNA antibodies in patients with elevated anti-dsDNA antibodies at baseline and **(****FIG 4B****)** C3 concentration in patients with low C3 at baseline in TULIP-1.
**FIG 5****: Primary and key secondary efficacy outcomes over time for TULIP-2**
**FIG's 5A-5C** illustrate the primary and key secondary efficacy outcomes over time for TULIP-2. **FIG 5A** shows the primary endpoint: percentage (95% CI) of patients with BICLA response. **FIG 5B** shows the percentage (95% CI) of patients with OCS dosage reduction to ≤7.5 mg/day sustained from week 40 to 52 among patients with baseline dosage prednisone ≥10 mg/day or equivalent. **FIG 5C** shows the percentage (95% CI) of patients with ≥50% reduction in CLASI activity score from baseline among patients with CLASI score ≥10 at baseline.

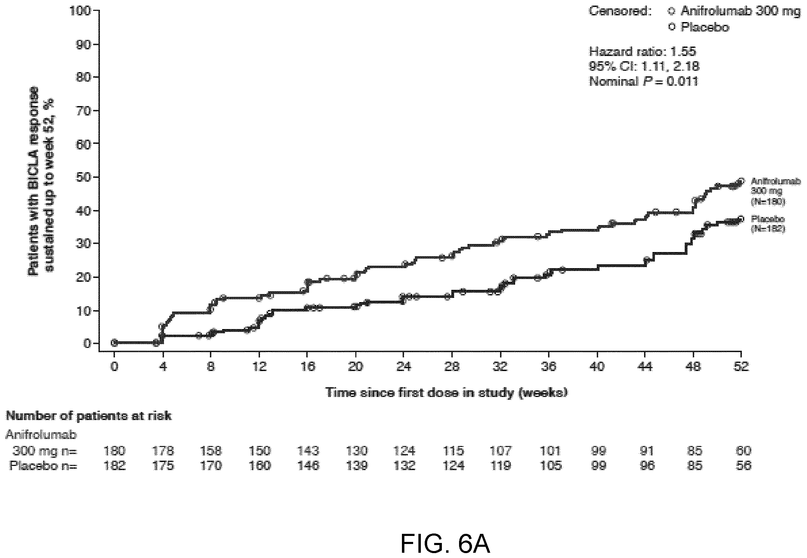

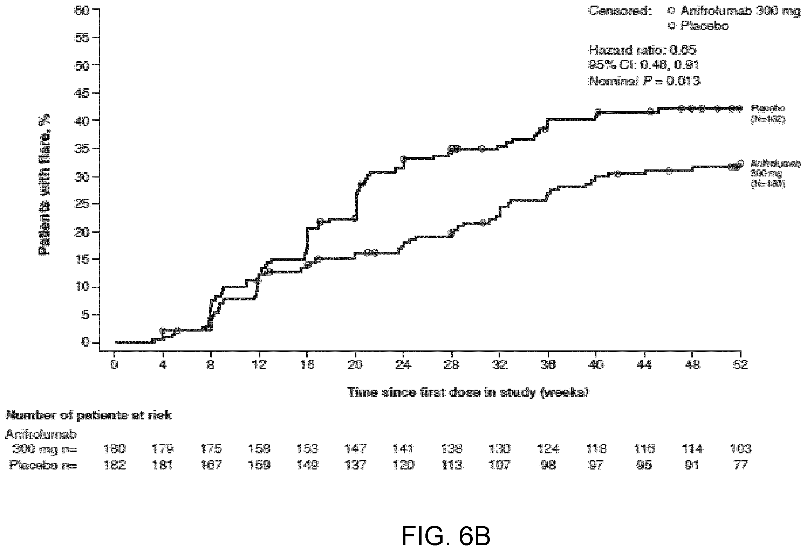

**FIG 6****: Time course of sustained BICLA response and time to flare in TULIP-2**
**FIG's 6A-6B** illustrate the time course of sustained BICLA response and time to flare in TULIP-2. **FIG 6A** shows the time to onset of BICLA response that was sustained to week 52. **FIG 6B** shows the time to first flare, with flare defined as either ≥1 new BILAG A or ≥2 new BILAG B items compared with the previous visit. Both outcomes were evaluated using a Cox proportional hazards model.

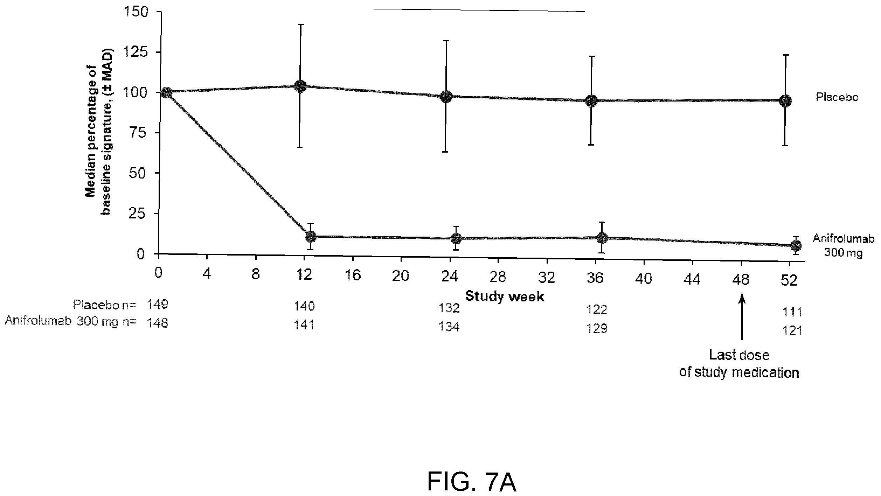

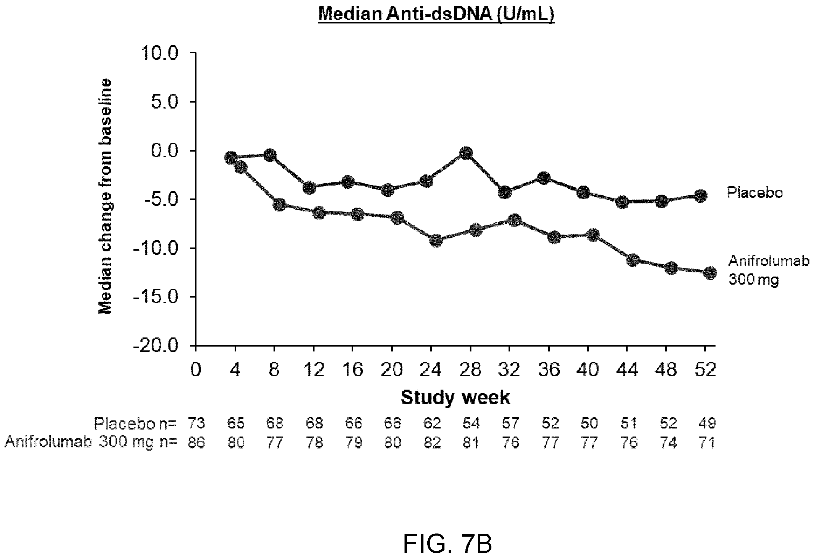

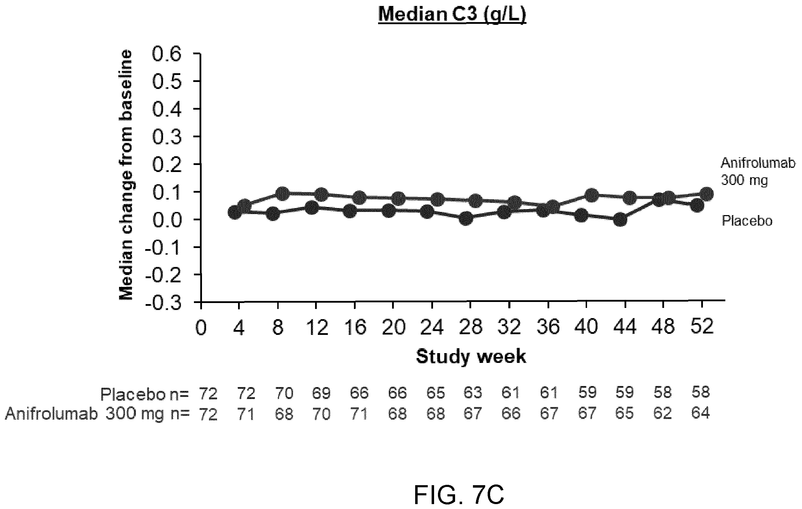

**FIG 7****: IFNGS and anti-dsDNA antibodies**
   The change from baseline over time in **(****FIG 7A****)** IFNGS and **(****FIG 7B****)** anti-dsDNA antibodies, and in patients with elevated anti-dsDNA antibodies at baseline in TULIP-2. **FIG 7C** shows the C3 concentration in patients with low C3 at baseline.

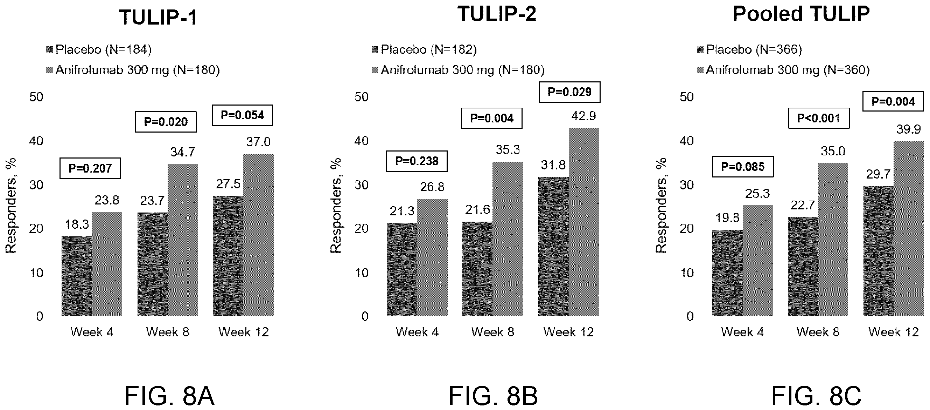

**FIG 8****: BICLA response at early time points**
**FIG's 8A-8C** illustrate the percentage of patients with a BICLA response at early time points in TULIP-1 **(****FIG 8A****),** TULIP-2 **(****FIG 8B****),** and pooled data from TULIP-1 and TULIP-2 **(****FIG 8C****).**

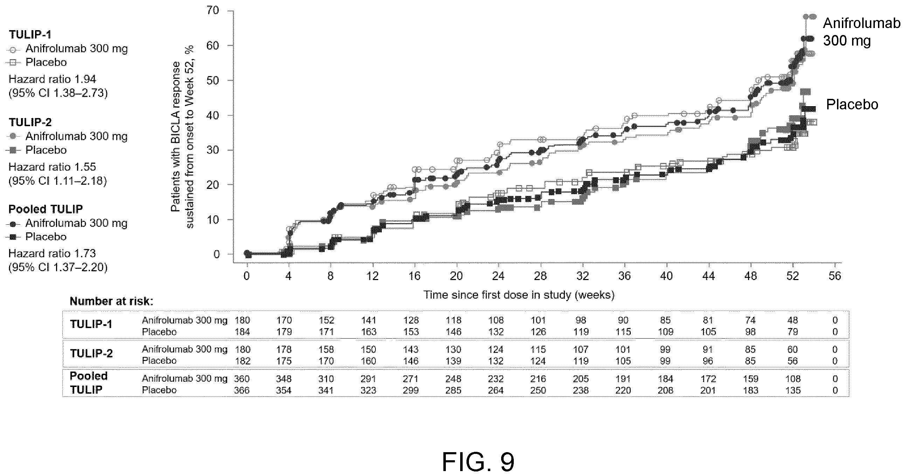

**FIG 9****: Percentage of patients with a BICLA response sustained from onset to 52 in TULIP-1, TULIP-2, and pooled data from TULIP-1 and TULIP-2.**
   BICLA, British Isles Lupus Assessment Group-based Composite Lupus Assessment; Cl, confidence interval; IFNGS, interferon gene signature; OCS, oral corticosteroid; SLEDAI-2K, Systemic Lupus Erythematosus Disease Activity Index 2000. In TULIP-1, TULIP-2, and pooled TULIP data, restricted medication rules were according to the TULIP-2 protocol. Hazard ratios and 95% Cls are estimated using a Cox regression model with treatment groups and the stratification factors (SLEDAI-2K at screening, Day 1 OCS dosage, and type I IFNGS test result at screening) as covariates.

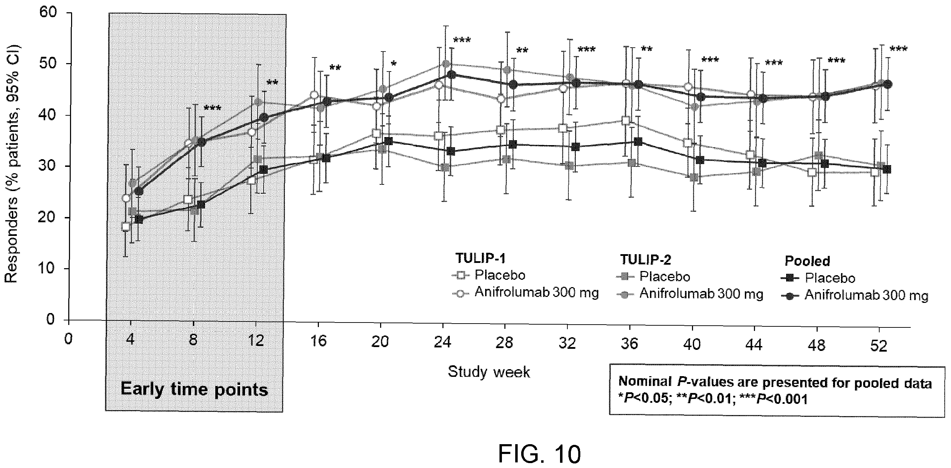

**FIG 10****: BICLA Response at All Time Points.**
   BICLA, British Isles Lupus Assessment Group-based Composite Lupus Assessment; IFNGS, interferon gene signature; OCS, oral corticosteroid; SLEDAI-2K, Systemic Lupus Erythematosus Disease Activity Index 2000. At early time points, P-values in TULIP-1 and TULIP-2 were 0.207 and 0.238 (Week 4), 0.020 and 0.004 (Week 8), and 0.054 and 0.029 (Week 12), respectively. In TULIP-1, TULIP-2, and pooled TULIP data, restricted medication rules were according to the TULIP-2 protocol. Responder rates are calculated using a stratified Cochran-Mantel-Haenszel approach, with stratification factors Day 1 OCS dosage, SLEDAI-2K, and type I IFNGS test result, both at screening. In the pooled analysis, an additional stratification factor is added for study. Vertical bars indicate 95% confidence intervals.

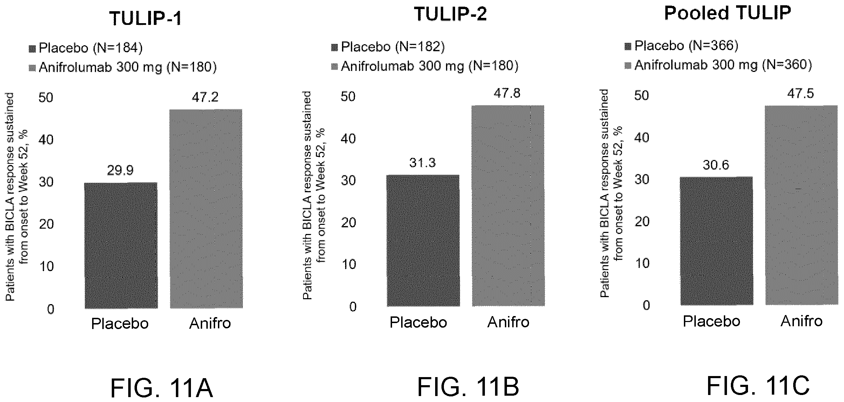

**FIG 11****: Percentage of patients with a BICLA response**
**FIG's 11A-11C** illustrate the percentage of patients with a BICLA response sustained from onset to week 52 in TULIP-1 **(****FIG 11A****),** TULIP-2 **(****FIG 11B****),** and pooled data from TULIP-1 and TULIP-2 **(****FIG 11C****).**

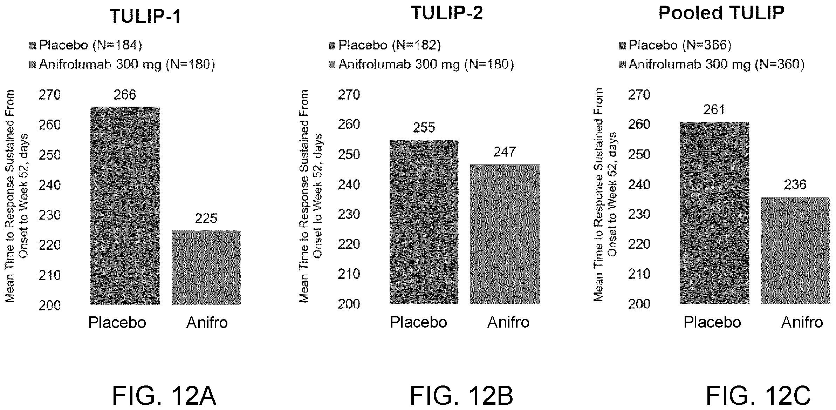

**FIG 12****: Time to BICLA response sustained**
**FIG's 12A-12C** illustrate the mean time to a BICLA response sustained from onset to week 52 in TULIP-1 **(****FIG 12A****),** TULIP-2 **(****FIG 12B****),** and pooled data from TULIP-1 and TULIP-2 **(****FIG 12C****).**

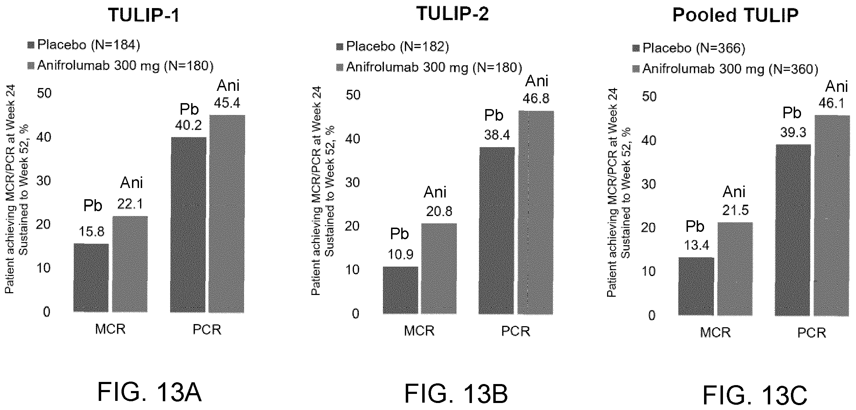

**FIG 13****: MCP and PCR**
**FIG's 13A-13C** illustrate the percentage of patients achieving a major clinical response or a partial clinical response at week 24 that was sustained to week 52 in TULIP-1 **(****FIG 13A****),** TULIP-2 **(****FIG 13B****),** and pooled data from TULIP-1 and TULIP-2 **(****FIG 12C****).** IFNGS, interferon gene signature; MCR, major clinical response; OCS, oral corticosteroid; PCR, partial clinical response; SLEDAI-2K, Systemic Lupus Erythematosus Disease Activity Index 2000. In TULIP-1, TULIP-2, and pooled TULIP data, restricted medication rules were according to the TULIP-2 protocol. Responder rates are calculated using a stratified Cochran-Mantel-Haenszel approach, with stratification factors Day 1 OCS dosage, SLEDAI-2K, and type I IFNGS test result, both at screening.

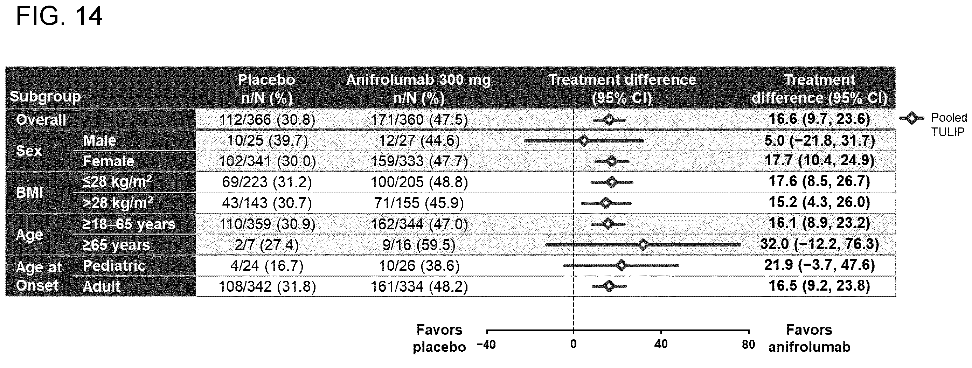

**FIG 14****: BICLA Response by Demographic Subgroup for Pooled TULIP.**
   BMI, body mass index; BICLA, British Isles Lupus Assessment Group-based Composite Lupus Assessment; Cl, Confidence interval; CMH, Cochran-Mantel-Haenszel. TULIP-1 data were analyzed incorporating the prespecified restricted medication rules. Differences in treatment estimates and associated 95% Cls were weighted and calculated using a stratified CMH approach.

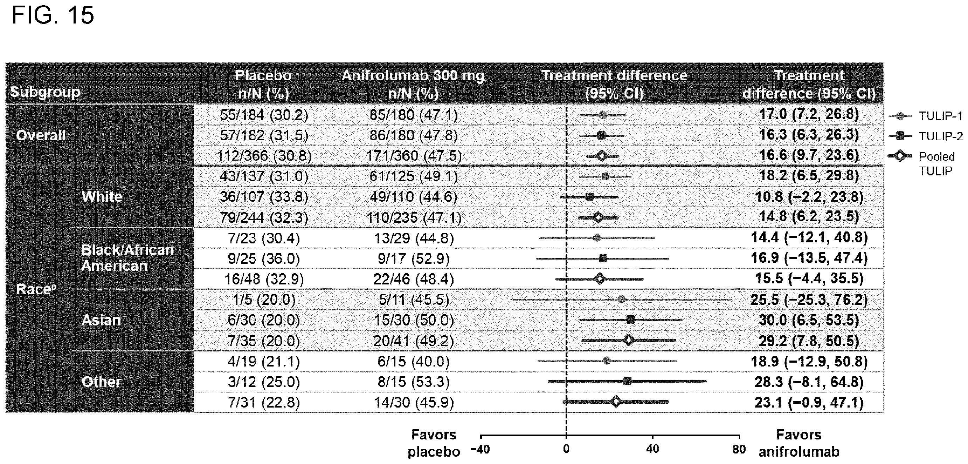

**FIG 15****: BICLA Response by Race.**
   BICLA, British Isles Lupus Assessment Group-based Composite Lupus Assessment; Cl, confidence interval; CMH, Cochran-Mantel-Haenszel. a8 patients from both the placebo and anifrolumab 300 mg arms had missing data from TULIP-1. TULIP-1 data were analyzed incorporating the prespecified restricted medication rules. Differences in treatment estimates and associated 95% Cls were weighted and calculated using a stratified CMH approach. Pooled TULIP data are subject to final confirmation.

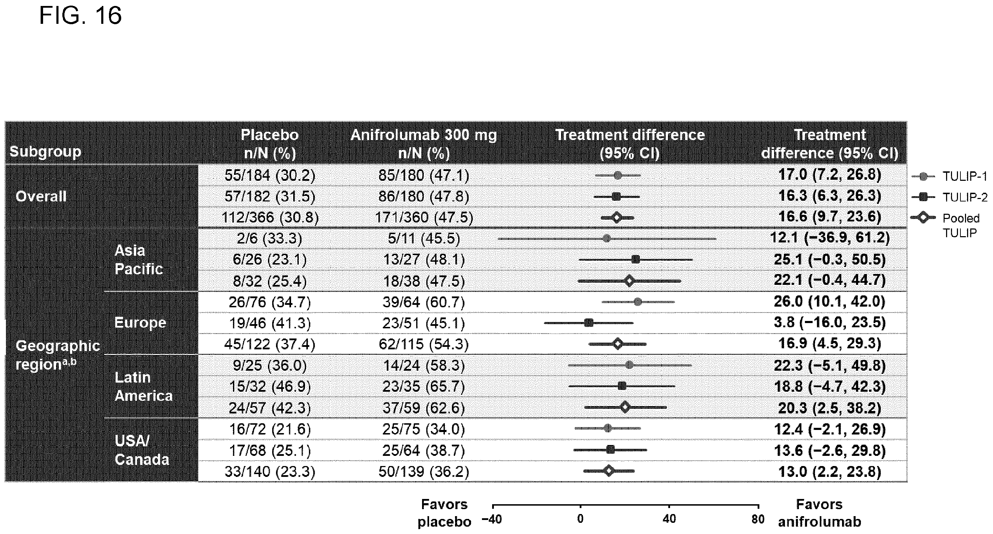

**FIG 16****: BICLA Response by Geographic Region.**
   BICLA, British Isles Lupus Assessment Group-based Composite Lupus Assessment; Cl, confidence interval; CMH, Cochran-Mantel-Haenszel. ^{a}Countries within each geographic region are defined on slide 7; ^{b}Treatment difference (95% CI) for Rest of World: TULIP-1, -6.7 (-65.3, 51.9); TULIP-2, 66.7 (6.6, 100.0); Pooled, 26.9 (-15.1, 69.0). TULIP-1 data were analyzed incorporating the prespecified restricted medication rules. Differences in treatment estimates and associated 95% Cls were weighted and calculated using a stratified CMH approach.

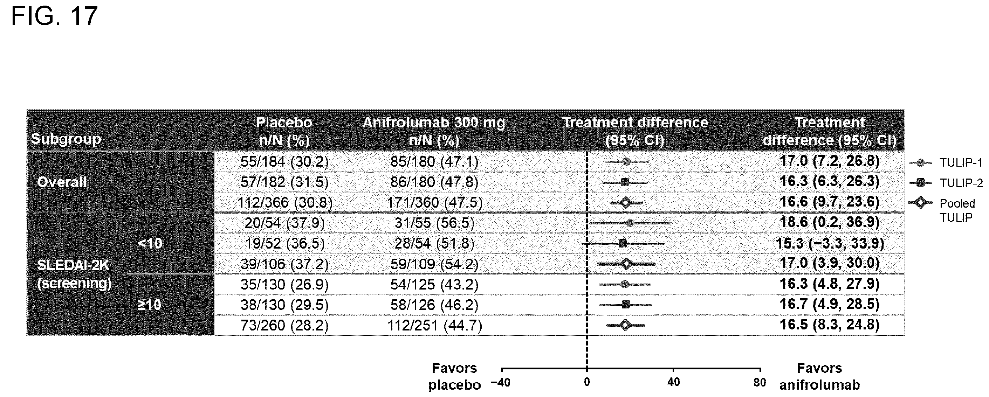

**FIG 17****: BICLA Response by SLEDAI-2K at Screening.**
   BICLA, British Isles Lupus Assessment Group-based Composite Lupus Assessment; Cl, confidence interval; CMH, Cochran-Mantel-Haenszel; SLEDAI-2K, Systemic Lupus Erythematosus Disease Activity Index 2000. TULIP-1 data were analyzed incorporating the prespecified restricted medication rules. Differences in treatment estimates and associated 95% Cls were weighted and calculated using a stratified CMH approach.

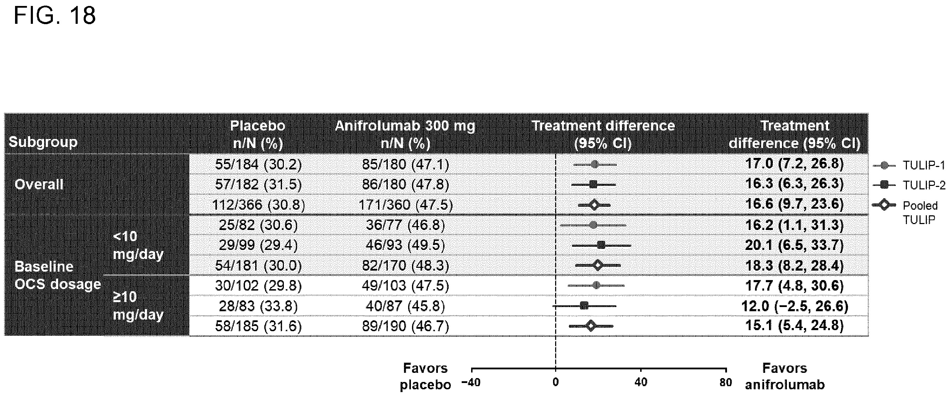

**FIG 18****: BICLA Response by Baseline Oral Corticosteroid Dosage.**
   BICLA, British Isles Lupus Assessment Group-based Composite Lupus Assessment; Cl, confidence interval; CMH, Cochran-Mantel-Haenszel; OCS, oral corticosteroid. TULIP-1 data were analyzed incorporating the prespecified restricted medication rules. Differences in treatment estimates and associated 95% Cls were weighted and calculated using a stratified CMH approach.

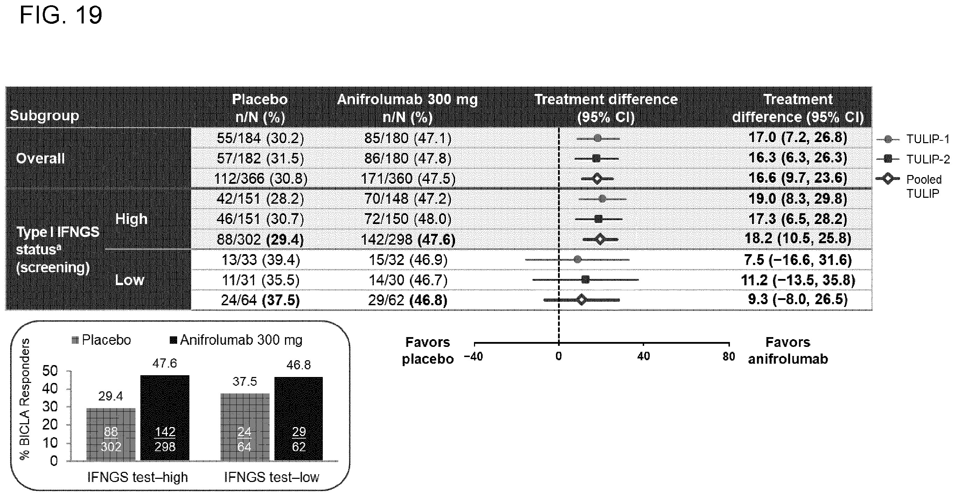

**FIG 19****: BICLA Response by Type I IFN Gene Signature Status (Screening)**
   BICLA, British Isles Lupus Assessment Group-based Composite Lupus Assessment; Cl, confidence interval; CMH, Cochran-Mantel-Haenszel; IFNGS, type I interferon gene signature; qPCR, quantitative polymerase chain reaction. ^{a}Type I IFNGS was classified as either high or low by central laboratory screening using a 4-gene qPCR-based test from whole blood. TULIP-1 data were analyzed incorporating the prespecified restricted medication rules. Differences in treatment estimates and associated 95% Cls were weighted and calculated using a stratified CMH approach.

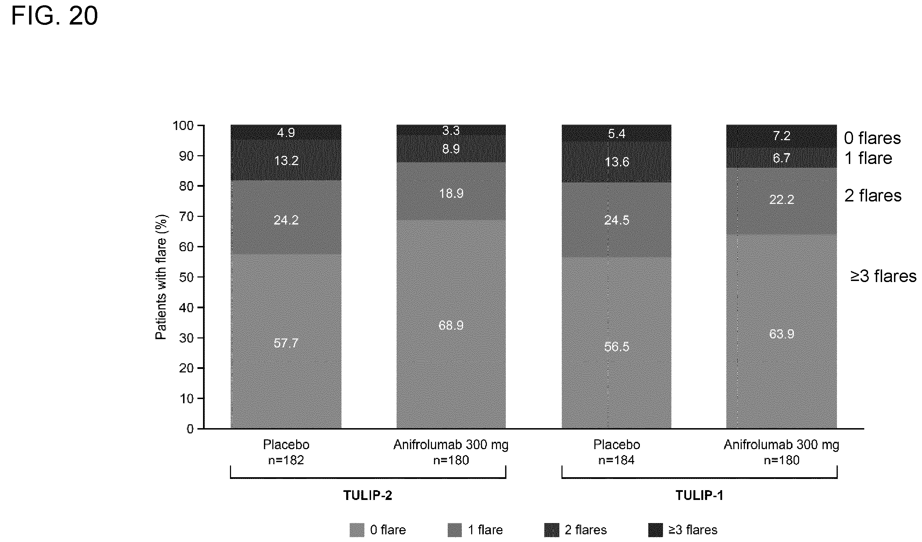

**FIG 20****: Flares assessed using BILIAG-2004 in patients treated with anifrolumab compared with placebo in TULIP-2 and TULIP-1.**
   BILAG: British Isles Lupus Assessment Group. Note: Flare defined as 1≤ new BILAG-2004 A or 2≤ new (worsening) BILAG-2004 B domain scores as compared with the prior month's visit.

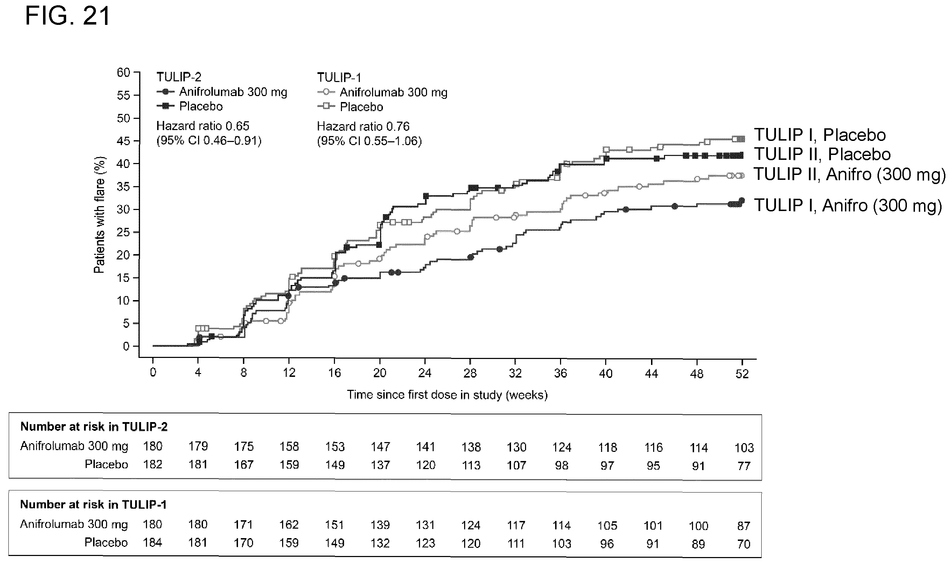

**FIG 21****: Time to final flare in TULIP-2 and TULIP-1**
   BILAG: British Isles Lupus Assessment Group. Note: Flare defined as 1≤. New BILAG-2004 A or 2≤ new (worsening) BILAG-2004 B domain scores as compared with the prior month's visit. Time to first flare is derived as data of first flare minus date of first administration of investigational product. If the patient did not have a flare, the time to flare is censored at the end of the exposure time.

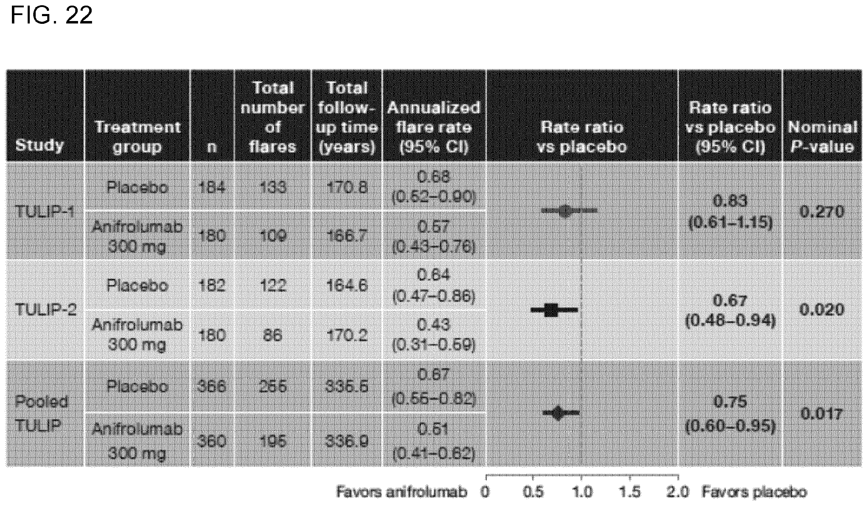

**FIG 22****: Annualized flare rates through week 52 in the TULIP trials.**

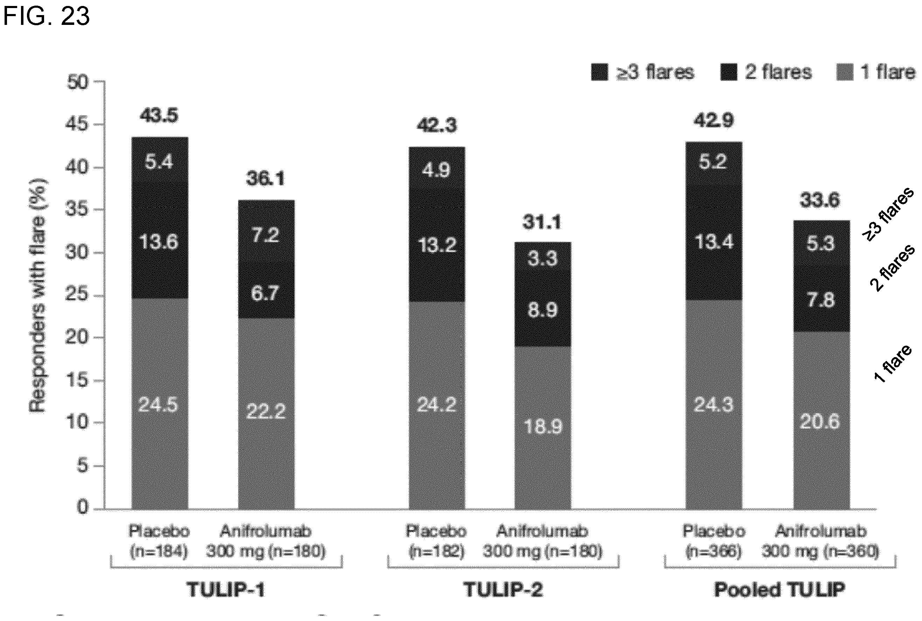

**FIG 23****: Percentage of patients with 1, 2, or three or more SLE flares through week 52 in the TULIP trials.**
   BILAG: British Isles Lupus Assessment Group; SLE, systemic lupus erythematosus. Flares were defined as 1≤ new BILAG-2004 A or 2≤ new BILAG-2004 B organ domain scores versus the prior visit.

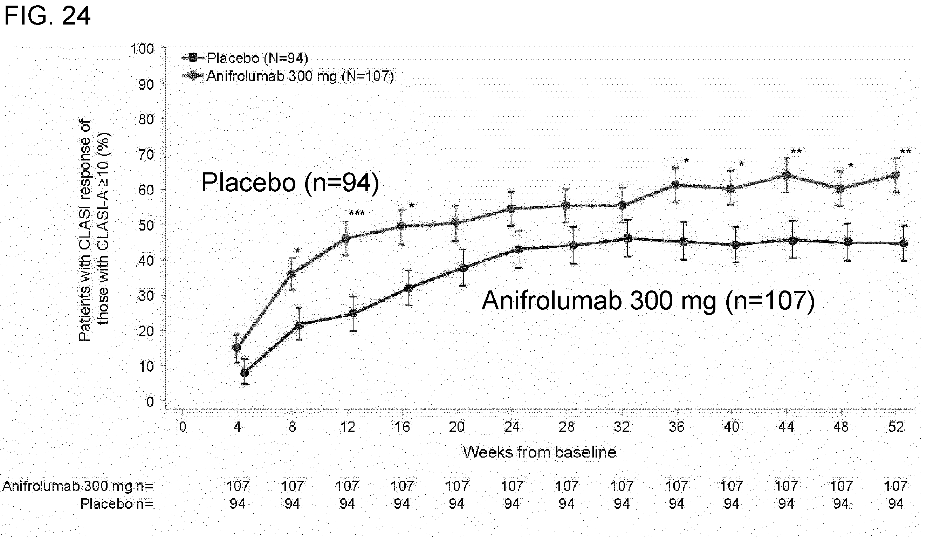

**FIG 24****: Percentage of Patients Achieving a CLASI-A Response by Time in Patients with SLE Receiving Anifrolumab and Placebo: TULIP-1 and TULIP-2 Pooled Data.**
   CLASI, Cutaneous Lupus Erythematous Disease Area and Severity Index; CLASI-A, CLASI activity score; n, number of patients in analysis; N, number of patients in treatment group; NA, not available; OCS, oral corticosteroids. A response is defined as 50%≤ reduction in CLASI activity score from baseline for patients with baseline CLASI-A 10≤. Responder rates are calculated using a stratified Cochran-Mantel-Haenszel approach, with stratification factors SLEDAI-2K score at screening. Day 1 OCS dosage, type I IFN gene signature test result at screening, and study (TULIP-1 and TULIP-2). Nominal P-values are presented, *P<0.05; **P<0.01; ***P<0.001.

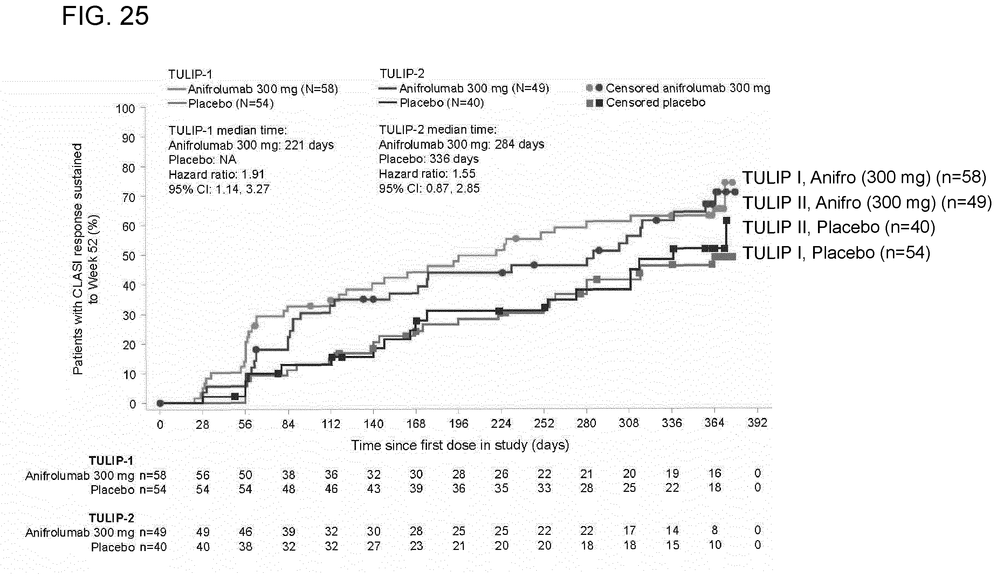

**FIG 25****: Time to CLASI-A Response Sustained to Week 52 in Patients with SLE Receiving Anifrolumab and Placebo: TULIP-1 and TULIP-2.**
   CLASI, cutaneous lupus erythematous disease area and severity index; CLASI-A, CLASI activity score; n, number of patients in analysis; N, number of patients in treatment group; NA, not available; OCS, oral corticosteroids. A response is defined as 50%≤ reduction in CLASI activity score from baseline for patients with baseline CLASI-A 10≤. Hazard ratios and 95% Cis were estimated using a Cox regression model with treatment groups with stratification (SLEDAI-2K score at screening. Day 1 OCS dosage, study, and type I IFN gene signature test result at screening) as covariates.

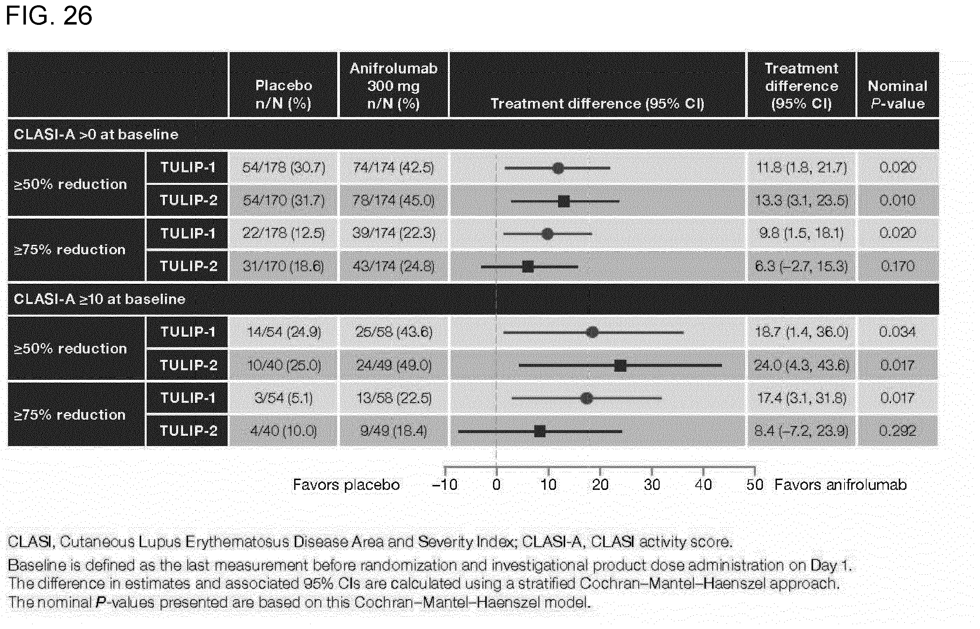

**FIG 26****: CLASI-A Response at Week 12 by Baseline CLASI-A at 50% and 75% Response Thresholds: TULIP-1 and TULIP-2**

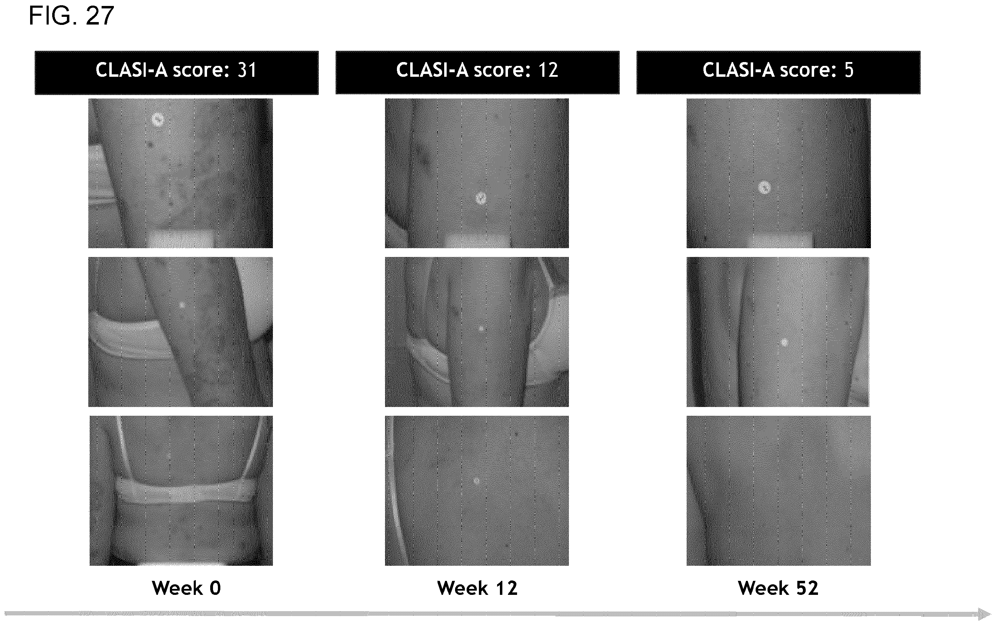

**FIG 27****: CLASI-A skin response: Example from one patient following treatment with anifrolumab (300mg).**
   CLASI-A, Cutaneous Lupus Erythematosus Disease Area and Severity Index activity score. A response is defined as ≥50% reduction in CLASI-A from baseline for patients with baseline CLASI-A ≥10. In total, 13 anifrolumab-treated patients from 5 sites participated in skin photography; 2 patients had a CLASI-A response at Week 12.

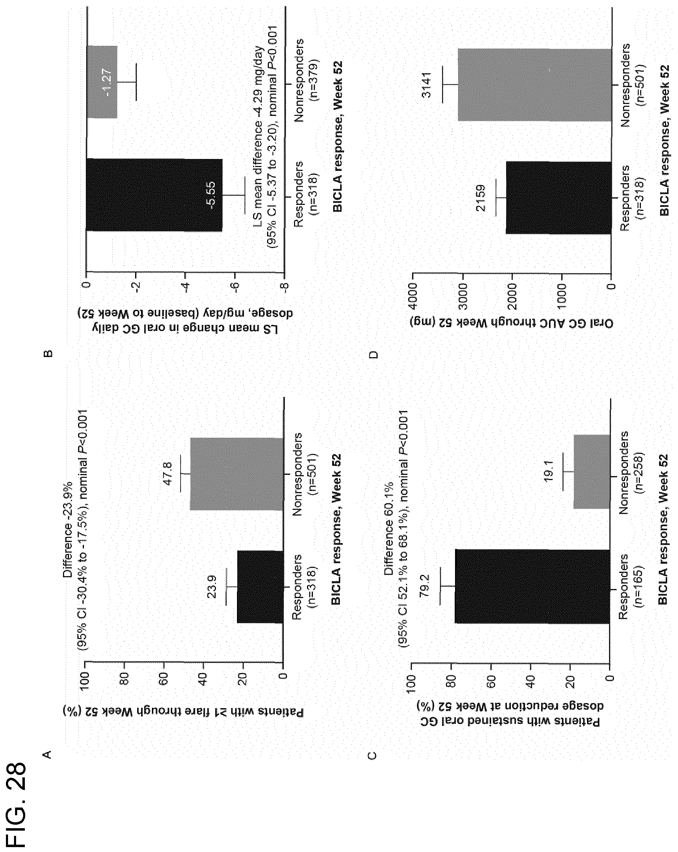

**FIG 28****: Flares and oral glucocorticoid use in BICLA responders vs nonresponders.**
**FIG 28A****:** Patients with ≥1 BILAG-2004 flare through Week 52. Error bars represent 95% Cl. **FIG 28B****:** LS mean change in oral glucocorticoid daily dosage from baseline to Week 52 in all patients regardless of baseline oral glucocorticoid dosage. Error bars represent 95% Cl. **FIG 28C****:** Patients achieving sustained oral glucocorticoid dosage reduction to ≤7.5 mg/day among patients receiving oral glucocorticoid ≥10 mg/day at baseline. Sustained oral glucocorticoid dosage reduction defined as oral glucocorticoid dose of ≤7.5 mg/day sustained from Weeks 40 to 52. Error bars represent 95% Cl. **FIG 28D****:** Oral glucocorticoid AUC through Week 52 for all patients regardless of baseline oral glucocorticoid dosage. Error bars represent SD. A-D, Rate difference, Cls, and nominal P values were calculated using a stratified Cochran-Mantel-Haenszel approach. AUC, area under the curve; BICLA, British Isles Lupus Assessment Group-based Composite Lupus Assessment; BILAG, British Isles Lupus Assessment Group; Cl, confidence interval; LS, least squares; SD, standard deviation.

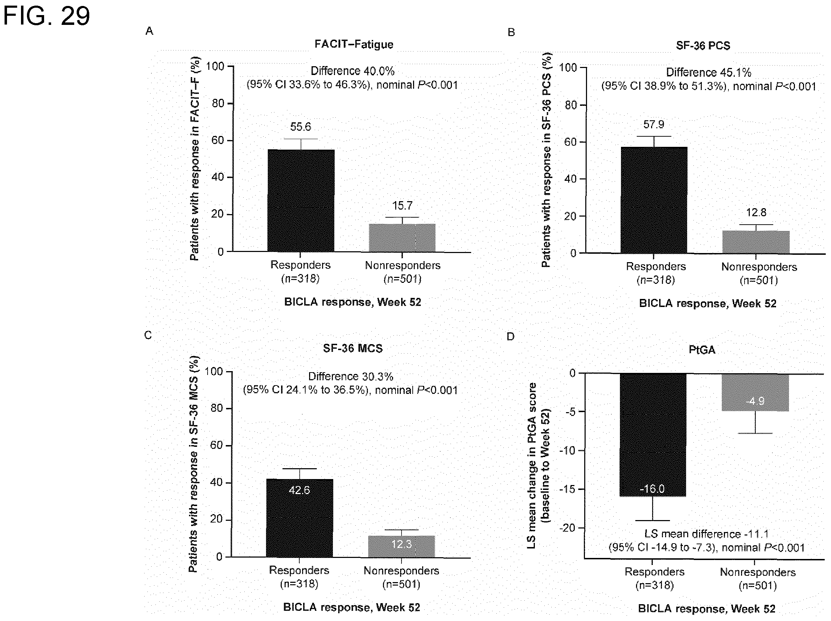

**FIG 29****: PRO response at Week 52 in BICLA responders** vs **nonresponders.**
   Patients with response in **(****FIG 29A****)** FACIT-F, defined as an improvement from baseline to Week 52 >3.4; **(****FIG 29B****)** SF-36 PCS, defined as an increase from baseline to Week 52 >3.4 in the PCS domain; and **(****FIG 29C****)** SF-36 MCS, defined as an increase from baseline to Week 52 >4.2 in the MCS domain. **FIGS 28A-****C:** Error bars represent 95% Cl. Response rates, Cls, and nominal P values were calculated using a stratified Cochran-Mantel-Haenszel approach. **FIG 28D****:** LS mean change from baseline to Week 52 in PtGA score. Error bars represent 95% Cl. LS mean difference, Cls, and nominal P values-calculated using mixed model repeated measures. BICLA, British Isles Lupus Assessment Group-based Composite Lupus Assessment; FACIT-F, Functional Assessment of Chronic Illness Therapy-Fatigue; MCS, mental component summary; PCS, physical component summary; PRO, patient-reported outcome; PtGA, Patient's Global Assessment; SF-36, Short Form 36 Health Survey.

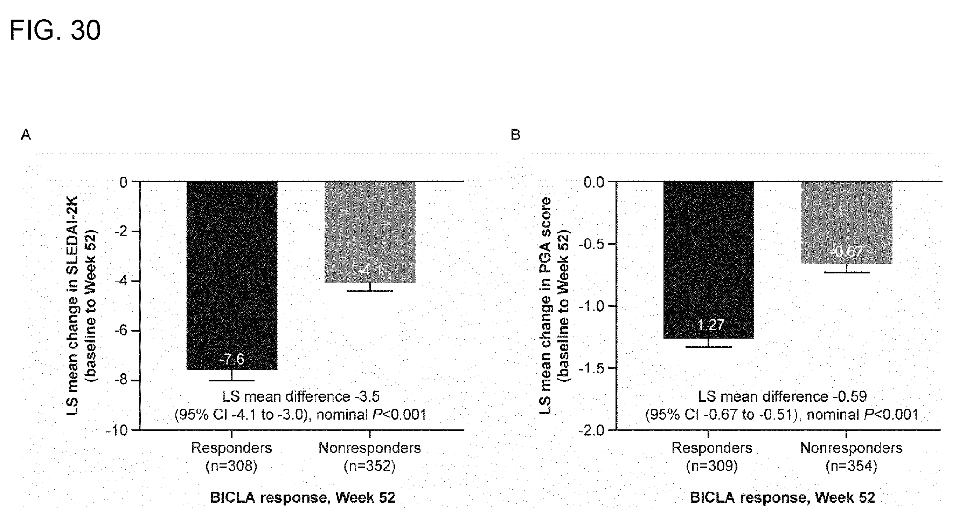

**FIG 30****: Change from baseline in SLEDAI-2K and PGA in BICLA responders** vs **nonresponders.**
   Change from baseline to Week 52 in **(****FIG 30A****)** SLEDAI-2K and **(****FIG 30B****)** PGA. Error bars represent 95% Cls. LS mean difference, Cls, and nominal *P* values calculated using mixed model repeated measures. BICLA, British Isles Lupus Assessment Group-based Composite Lupus Assessment; BILAG, British Isles Lupus Assessment Group; Cl, confidence interval; LS, least squares; PGA, Physician's Global Assessment; SLEDAI-2K, Systemic Lupus Erythematosus Disease Activity Index 2000.

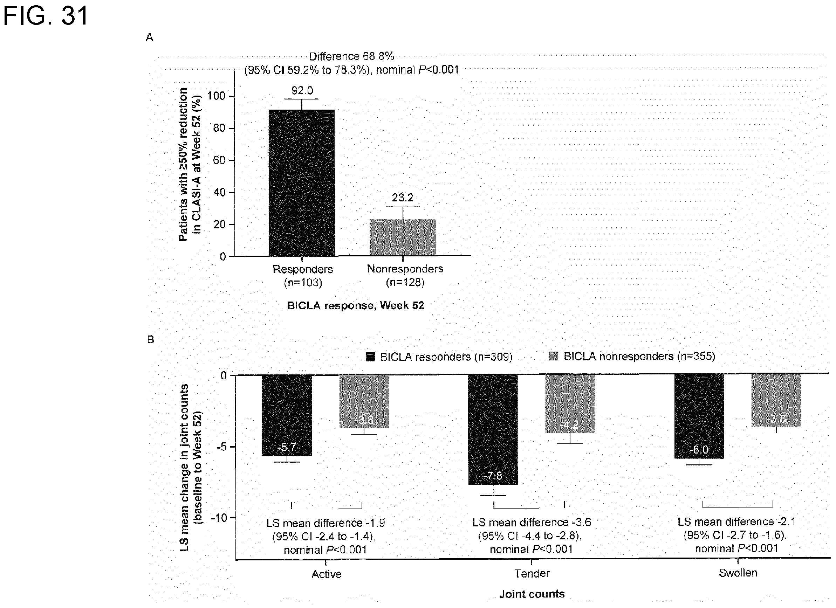

**FIG 31****: Patients with CLASI-A response at Week 52 (defined as** ≥**50% reduction from baseline to Week 52) among patients with a CLASI-A score ≥10 at baseline.**
   Response rates, Cls, and nominal P values were calculated using a stratified Cochran-Mantel-Haenszel approach. **FIG 31B****,** Change in LS mean joint count from baseline to Week 52 for active (defined as a joint with swelling and tenderness), tender, and swollen joints. Error bars represent 95% Cls. LS mean difference, Cls, and nominal P values calculated using mixed model repeated measures. BICLA, BILAG-based Composite Lupus Assessment; BILAG-2004, British Isles Lupus Assessment Group-2004; Cl, confidence interval; CLASI-A, Cutaneous Lupus Erythematosus Disease Area and Severity Index Activity; LS, least squares.: CLASI-A response and joint counts between BICLA responders and nonresponders. A,

### DETAILED DESCRIPTION

The present invention is defined in the claims.

The disclosure relates to methods and compositions for the treatment of Systemic Lupus Erythematosus (SLE). Specifically, the disclosure relates to methods comprising administering to a subject in need thereof anifrolumab.

As utilized in accordance with the present disclosure, unless otherwise indicated, all technical and scientific terms shall be understood to have the same meaning as commonly understood by one of ordinary skill in the art. Unless otherwise required by context, singular terms shall include pluralities and plural terms shall include the singular.

Without limiting the disclosure, a number of embodiments of the disclosure are described herein for purpose of illustration.

### Subject

The term "subject" is intended to include human and non-human animals, particularly mammals. The subject may be an adult human patient. The subject may be a patient with moderate to severe SLE.

### Treatment

The terms "treatment" or "treat" as used herein refer to both therapeutic treatment and prophylactic or preventative measures. Those in need of treatment include subjects having SLE as well as those prone to having SLE or those in which SLE is to be prevented. In some embodiments, the methods disclosed herein can be used to treat SLE.

### Administration

The terms "administration" or "administering" as used herein refer to providing, contacting, and/or delivering a compound or compounds by any appropriate route to achieve the desired effect. Administration may include, but is not limited to, oral, sublingual, parenteral (e.g., intravenous, subcutaneous, intracutaneous, intramuscular, intraarticular, intraarterial, intrasynovial, intrasternal, intrathecal, intralesional, or intracranial injection), transdermal, topical, buccal, rectal, vaginal, nasal, ophthalmic, via inhalation, and implants.

### Pharmaceutical composition

The terms "pharmaceutical composition" as used herein refers to a compound or composition capable of inducing a desired therapeutic effect when properly administered to a subject. In some embodiments, the disclosure provides a pharmaceutical composition comprising a pharmaceutically acceptable carrier and a therapeutically effective amount of at least one antibody of the disclosure. The terms "pharmaceutically acceptable carrier" or "physiologically acceptable carrier" as used herein refer to one or more formulation materials suitable for accomplishing or enhancing the delivery of one or more antibodies of the disclosure.

### Antigen-binding fragment

The term "antigen-binding fragment", refers to one or more fragments of an antibody that retain(s) the ability to specifically bind to the antigen. Examples of antigen-binding fragments include the following: Fab fragment, F(ab')2 fragment, Fd fragment, Fv fragment, dAb fragment, as well as a scFv.

### Systemic lupus erythematosus (SLE)

Systemic lupus erythematosus (SLE) is a chronic, multisystemic, disabling autoimmune rheumatic disease of unknown etiology. Systemic lupus erythematosus predominantly affects women of childbearing years with a recent review reporting the female-to-male ratio in the childbearing years to be about 12:1. Accurate data on the current incidence and prevalence of SLE are largely lacking, however there are numerous indications that SLE is more common in non-Caucasian populations; for example, in the United States of America (USA), SLE is more prevalent in African-Americans, Hispanics, and Asians than Caucasians. As a result, SLE prevalence varies from country to country. In addition, variability of SLE prevalence within countries appears to be dependent upon racial, genetic differences, complex socioeconomic factors and age; the incidence of disease in females is usually highest between 15-44 years of age.

Clinical manifestations of SLE can include constitutional symptoms, alopecia and rashes, serositis, inflammatory arthritis, renal disease, systemic vasculitis, lymphadenopathy, splenomegaly, hemolytic anemia, cognitive dysfunction and other central nervous system (CNS) involvement. These disease manifestations cause a significant burden of illness and can cause reduced physical function, loss of employment, lower health-related quality of life (HRQoL), and a lifespan shortened by about 10 years. Increased hospitalizations and side effects of medications including chronic oral corticosteroids (OCS) and other immunosuppressive treatments add to disease burden in SLE At this time, belimumab is the only new treatment for SLE that has been approved by the Food and Drug Administration (FDA) in about 50 years since hydroxychloroquine was approved for use in discoid lupus and SLE. The existing standard of care treatment for SLE (SOC SLE) otherwise consists of off-label medications. lupus erythematosus.

Different patterns of disease activity in SLE have been identified. Three patterns of SLE activity were apparent in a cohort study by Petri and colleagues: chronic active, relapsing-remitting, and long quiescent. The chronic active pattern was the most frequent pattern, representing about half of the total person-years followed in this SLE cohort study. The average disease activity was moderately severe for patients with chronic active disease, suggesting that significant morbidity occurs in this subset. Additionally, most patients with chronic active disease had an overlay of moderate or severe exacerbations during follow-up. A second, relapsing-remitting pattern of disease occurred in slightly more than a quarter of patients, and average disease activity tended to be mild during remitting disease periods. The least common pattern was long quiescent, which occurred in slightly less than a quarter of patients.

Although off-label therapy has improved in recent years, long-term prognosis remains poor. Compared to the general population, the overall mortality in SLE is increased with a standardized mortality ratio (SMR; defined as the ratio of the number of deaths observed to deaths expected) of 2.4 in a large international cohort of 9,457 patients followed for over 70,000 patient-years. In this study, mortality varied by geographic region, and was greater in females, patients less that 40 years of age (SMR = 10.7, 9.5-11.9 95% confidence interval [Cl]), and in patients with disease duration less than 1 year (SMR = 5.4, 4.7-6.3 95% CI). The major causes of death were circulatory disease (SMR = 1.7, 1.5-1.9 95% CI), infections (SMR = 5.0, 3.7-6.7 95% CI), and renal causes (SMR = 7.9, 5.5-11.0 95% CI). Other prospective studies have reported elevated mortality rates among SLE patients with severe and/or active disease.

There is substantial unmet medical need in the treatment of SLE, particularly in patients with moderate-to-severe disease. Belimumab is the only new treatment for SLE that has been approved by the FDA in about 50 years since hydroxychloroquine was approved for use in discoid lupus and SLE. Many agents currently used to treat SLE such as azathioprine, cyclophosphamide, and mycophenolate mofetil/mycophenolic acid have not been approved for the disease. Other available treatments include nonsteroidal anti-inflammatory drugs (NSAIDs) and analgesics for fever, arthralgia, and arthritis; and topical sunscreens to minimize photosensitivity. Antimalarial agents (eg, hydroxychloroquine) and corticosteroids may be added to control arthralgia, arthritis, and rashes. It is often difficult to taper patients with moderate or severe disease completely off corticosteroids, which cause long-term morbidity and may contribute to early cardiovascular mortality. Even low-dose prednisone, if used as a long-term treatment, carries increased risk for side effects.

### Clinical trials

### Phase 2/Phase II/pivotal studies

Phase II studies gather preliminary data on effectiveness. In Phase 2 studies, researchers administer the drug to a group of patients with the disease or condition for which the drug is being developed. Typically involving a few hundred patients, these studies aren't large enough to show whether the drug will be beneficial. Instead, Phase 2 studies provide researchers with additional safety data. Researchers use these data to refine research questions, develop research methods, and design new Phase 3 research protocols.

### Phase 3/Phase III/pivotal studies or trials

Researchers design Phase 3 studies to demonstrate whether or not a product offers a treatment benefit to a specific population. Sometimes known as pivotal studies, these studies involve 300 to 3,000 participants. Phase 3 studies provide most of the safety data. In previous studies, it is possible that less common side effects might have gone undetected. Because these studies are larger and longer in duration, the results are more likely to show long-term or rare side effects. Regulatory bodies such as the EMA and FDA usually require a phase III clinical trial demonstrating that the product is safe and at least as effective (if not better) than available medications, before approving a new medication. Phase III clinical trials usually fail, even if they follow a successful a phase II clinical trial.

### CLASI (Cutaneous Lupus Erythematosus Disease Area and Severity Index)

CLASI is a tool used to measure disease severity and response to treatment. A 4-point or 20% decrease in CLASI activity score is commonly viewed as a cut-off for classifying subjects as responders to treatment. In particular embodiments, treatment using anifrolumab results in at least 50% reduction of a subject's CLASI score compared to the subject's baseline score.

The CLASI is a validated index used for assessing the cutaneous lesions of SLE and consists of 2 separate scores: the first summarizes the inflammatory activity of the disease; the second is a measure of the damage done by the disease. The activity score takes into account erythema, scale/hypertrophy, mucous membrane lesions, recent hair loss, and nonscarring alopecia. The damage score represents dyspigmentation, scarring/atrophy/panniculitis, and scarring of the scalp. Subjects are asked if their dyspigmentation lasted 12 months or longer, in which case the dyspigmentation score is doubled. Each of the above parameters is measured in 13 different anatomical locations, included specifically because they are most often involved in cutaneous lupus erythematosus (CLE). The most severe lesion in each area is measured.

In particular embodiments, treatment using anifrolumab reduces a subject's CLASI score by at least week 8, week 12, week 24, week 36, week 48, or week 52 of treatment. In particular embodiments, treatment using anifrolumab reduces a subject's CLASI score by at least week 8. In particular embodiments, treatment using anifrolumab reduces a subject's CLASI score by at least week 12.

In particular embodiments provided herein is a method of treating systemic lupus erythematosus in a subject in need thereof, comprising administering to the subject a therapeutically effective amount of anifrolumab, wherein the treatment results in a reduction in the Cutaneous Lupus Erythematosus Disease Area and Severity Index (CLASI) score compared to a patient receiving placebo.

### Oral Corticosteroids

Oral corticosteroids include prednisone, cortisone, hydrocortisone, methylprednisolone, prednisolone and triamcinolone.

In particular embodiments provided herein is a method of treating systemic lupus erythematosus in a subject in need thereof, wherein the subject is being treated with oral corticosteroids, comprising administering to the subject a therapeutically effective amount of anifrolumab, wherein the treatment results in a reduction in oral corticosteroid dosage in the subject to at least ≤ 5.5 mg/day, ≤ 6.5 mg/day, ≤ 7.5 mg/day, or ≤ 8.5 mg/day. In particular embodiments, the reduction in oral corticosteroid dosage in the subject is reduced to at least ≤ 7.5 mg/day. In particular embodiments, the treatment results in a reduction in oral corticosteroid dosage in the subject from ≥ 10 mg/day to ≤ 7.5 mg/day.

In particular embodiments provided herein is a method of treating systemic lupus erythematosus in a subject in need thereof, wherein the subject is being treated with oral corticosteroids, comprising administering to the subject a therapeutically effective amount of anifrolumab, wherein the treatment results in a reduction in oral corticosteroid dosage in the subject to at least ≤ 5.5 mg/day, ≤ 6.5 mg/day of prednisone or prednisone equivalent dose, ≤ 7.5 mg/day of prednisone or prednisone equivalent dose, or ≤ 8.5 mg/day of prednisone or prednisone equivalent dose. In particular embodiments, the reduction in oral corticosteroid dosage in the subject is reduced to at least ≤ 7.5 mg/day. In particular embodiments, the treatment results in a reduction in oral corticosteroid dosage in the subject from ≥ 10 mg/day to ≤ 7.5 mg/day of prednisone or prednisone equivalent dose.

Examples of equivalent doses of oral prednisone are shown in Table 2.

**Table 2: Examples of equivalent doses of oral prednisone**

| **Oral Prednisone and Equivalents** | **Equivalent Dose** | | | | |
|---|---|---|---|---|---|
| **Oral Prednisone** | **7.5 mg** | **10 mg** | **20 mg** | **30 mg** | **40 mg** |
| Cortisone | 37.5 mg | 50 mg | 100 mg | 150 mg | 200 mg |
| Hydrocortisone | 30 mg | 40 mg | 80 mg | 120 mg | 160 mg |
| Methylprednisolone | 6 mg | 8 mg | 16 mg | 24 mg | 32 mg |
| Prednisolone | 7.5 mg | 10 mg | 20 mg | 30 mg | 40 mg |
| Triamcinolone | 6 mg | 8 mg | 16 mg | 24 mg | 32 mg |

### Tender and Swollen Joints

The swollen and tender joint count is based on left and right shoulder, elbow, wrist, metacarpophalangeal (MCP) 1, MCP2, MCP3, MCP4, MCP5, proximal interphalangeal (PIP) 1, PIP2, PIP3, PIP4, PIP5 joints of the upper extremities and left and right knee of the lower extremities. An active joint for the joint count assessment is defined as a joint with tenderness and swelling.

In particular embodiments provided herein is method of treating systemic lupus erythematosus in a subject in need thereof, comprising administering to the subject a therapeutically effective amount of anifrolumab, wherein the treatment results in at least 50% improvement from baseline value of tender joint count and swollen joint count compared to a patient receiving placebo.

### Dosage regimes

The dose of the anifrolumab to be administered to the subject will vary depending, in part, upon the size (body weight, body surface, or organ size) and condition (the age and general health) of the subject.

In particular embodiments, the subject is administered one or more fixed doses of anifrolumab, wherein the dose is 150 mg, 200 mg, 250 mg, 300 mg, or 350 mg. In some embodiments, the subject is administered one or more fixed doses of anifrolumab wherein the dose is 300 mg.

In particular embodiments, anifrolumab is administered over a two-week treatment period, over a four-week treatment period, over a six-week treatment period, over an eight-week treatment period, over a twelve-week treatment period, over a twenty-four-week treatment period, or over a one-year or more treatment period. In particular embodiments, anifrolumab is administered over a three-week treatment period, over a six-week treatment period, over a nine-week treatment period, over a twelve-week treatment period, over a twenty-four-week treatment period, or over a one-year or more treatment period. In particular embodiments, anifrolumab is administered for at least 52 weeks.

In particular embodiments, anifrolumab is administered every week, every two weeks, every four weeks, every six weeks, every eight weeks, every ten weeks, or every twelve weeks.

### Administration methods

When used for *in vivo* administration, the formulations of the disclosure should be sterile. The formulations of the disclosure may be sterilized by various sterilization methods, including, for example, sterile filtration or radiation. In one embodiment, the formulation is filter sterilized with a presterilized 0.22-micron filter. Sterile compositions for injection can be formulated according to conventional pharmaceutical practice as described in "Remington: The Science & Practice of Pharmacy," 21st ed., Lippincott Williams & Wilkins, (2005).

In some embodiments, type I IFN inhibitor can be formulated for particular routes of administration, such as oral, nasal, pulmonary, topical (including buccal and sublingual), rectal, vaginal, and/or parenteral administration. The terms "parenteral administration" and "administered parenterally" as used herein refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection, and infusion.

In some embodiments, anifrolumab can be formulated for particular routes of administration, such as oral, nasal, pulmonary, topical (including buccal and sublingual), rectal, vaginal, and/or parenteral administration. The terms "parenteral administration" and "administered parenterally" as used herein refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection, and infusion.

### Formulations

The formulations can be presented in unit dosage form and can be prepared by any method known in the art of pharmacy. Actual dosage levels of the active ingredients in the formulation of the present disclosure may be varied so as to obtain an amount of the active ingredient which is effective to achieve the desired therapeutic response for a particular subject, composition, and mode of administration, without being toxic to the subject (e.g., "a therapeutically effective amount"). Dosages can also be administered via continuous infusion (such as through a pump). The administered dose may also depend on the route of administration.

The pharmaceutical composition may comprise about 150 mg/mL anifrolumab.

The pharmaceutical composition may comprise 50 mM lysine HCl.

The pharmaceutical composition may comprise 130 mM trehalose dihydrate.

The pharmaceutical composition may comprise 0.05% polysorbate 80.

The pharmaceutical composition may comprise 25 mM histidine/histidine HCl.

The pharmaceutical composition may have a pH of 5.9.

### Interferon test

Type I IFN is considered important in SLE disease pathogenesis and inhibition of this pathway is targeted by anifrolumab. To understand the relationship between type I IFN expression and response to anti-IFN therapy, it is necessary to know if a subject's disease is driven by type I IFN activation. However, direct measurement of the target protein remains a challenge. As such, a transcript-based marker was developed to evaluate the effect of over expression of the target protein on a specific set of mRNA markers. The expression of these markers is easily detected in whole blood and demonstrates a correlation with expression in diseased tissue such as skin in SLE. The bimodal distribution of the transcript scores for SLE subjects supports defining an IFN test high and low subpopulation. The type I IFN test is described in WO2011028933 A1.

### Anifrolumab

Anifrolumab is a monoclonal antibody targeting IFNAR1 (the receptor for α, β, and ω interferons). Disclosure related to anifrolumab can be found in U.S. Patent No. 7,662,381 and U.S. Patent No. 9,988,459.

Anifrolumab is a monoclonal antibody which binds to IFNAR with high affinity and specificity. The antibody is an IFNAR-blocking (antagonistic) antibody, and blocks the activity of the receptor's ligands, namely type I interferons such as interferon-a and interferon-β. Anifrolumab thus provides for downregulation of IFNAR signaling, and thus suppression of IFN-inducible genes.

**Table 3: Anifrolumab sequences**

| | |
|---|---|
| Anifrolumab VH (SEQ ID NO: 1) | |
| Anifrolumab VL (SEQ ID NO: 2) | |
| HCDR1 (SEQ ID NO: 3) | NYWIA |
| HCDR2 (SEQ ID NO: 4) | IIYPGDSDIRYSPSFQG |
| HCDR3 (SEQ ID NO: 5) | HDIEGFDY |
| LCDR1 (SEQ ID NO: 6) | RASQSVSSSFFA |
| LCDR2 (SEQ ID NO: 7) | GASSRAT |
| LCDR3 (SEQ ID NO: 8) | QQYDSSAIT |

Thus, anifrolumab is an antibody comprising an HCDR1, HCDR2 and HCDR3 of SEQ ID NO: 3, SEQ ID NO: 4, and SEQ ID NO: 5, respectively (or functional variant thereof); and an LCDR1 , LCDR2 and LCDR3 of SEQ ID NO: 6, SEQ ID NO: 7, and SEQ ID NO: 8, respectively (or functional variant thereof). In more detail, anifrolumab as referred to herein is an antibody comprising a VH of SEQ ID NO: 1 and a VL of SEQ ID NO: 2 (or functional variant thereof).

The present invention encompasses the antibodies defined herein having the recited CDR sequences or variable heavy and variable light chain sequences (reference (anifrolumab) antibodies), as well as functional variants thereof. A "functional variant" binds to the same target antigen as the reference (anifrolumab) antibody. The functional variants may have a different affinity for the target antigen when compared to the reference antibody, but substantially the same affinity is preferred.

In one embodiment functional variants of a reference (anifrolumab) antibody show sequence variation at one or more CDRs when compared to corresponding reference CDR sequences. Thus, a functional antibody variant may comprise a functional variant of a CDR. Where the term "functional variant" is used in the context of a CDR sequence, this means that the CDR has at most 2, preferably at most 1 amino acid differences when compared to a corresponding reference CDR sequence, and when combined with the remaining 5 CDRs (or variants thereof) enables the variant antibody to bind to the same target antigen as the reference (anifrolumab) antibody, and preferably to exhibit the same affinity for the target antigen as the reference (anifrolumab) antibody.

Without wishing to be bound by theory, since anifrolumab targets (e.g. blocks or antagonizes) IFNAR, it is believed that anifrolumab treats a disease (such as SLE) by blocking signaling initiated by type I interferons (IFNs). Type I IFNs are known to be important drivers of inflammation (e.g. by coordinating the type I interferon response), and thus play a pivotal role in the immune system. However, dysregulation of type I IFN-signaling can lead to aberrant (e.g. aberrantly high) levels of inflammation, and autoimmunity. Such dysregulation of type I IFN interferons has been reported in numerous autoimmune diseases.

For example, a variant of the reference (anifrolumab) antibody may comprise:
- a heavy chain CDR1 having at most 2 amino acid differences when compared to SEQ ID NO: 3;
- a heavy chain CDR2 having at most 2 amino acid differences when compared to SEQ ID NO: 4;
- a heavy chain CDR3 having at most 2 amino acid differences when compared to SEQ ID NO: 5;
- a light chain CDR1 having at most 2 amino acid differences when compared to SEQ ID NO: 6;
- a light chain CDR2 having at most 2 amino acid differences when compared to SEQ ID NO: 7; and
- a light chain CDR3 having at most 2 amino acid differences when compared to SEQ ID NO: 8;
wherein the variant antibody binds to the target of anifrolumab (e.g. IFNAR) and preferably with the same affinity.

Preferably, a variant of the reference (anifrolumab) antibody may comprise:
- a heavy chain CDR1 having at most 1 amino acid difference when compared to SEQ ID NO: 3;
- a heavy chain CDR2 having at most 1 amino acid difference when compared to SEQ ID NO: 4;
- a heavy chain CDR3 having at most 1 amino acid difference when compared to SEQ ID NO: 5;
- a light chain CDR1 having at most 1 amino acid differences when compared to SEQ ID NO: 6;
- a light chain CDR2 having at most 1 amino acid difference when compared to SEQ ID NO: 7; and
- a light chain CDR3 having at most 1 amino acid difference when compared to SEQ ID NO: 8;
wherein the variant antibody binds to the target of anifrolumab (e.g. IFNAR) and preferably with the same affinity.

In one embodiment a variant antibody may have at most 5, 4 or 3 amino acid differences total in the CDRs thereof when compared to a corresponding reference (anifrolumab) antibody, with the proviso that there is at most 2 (preferably at most 1) amino acid differences per CDR. Preferably a variant antibody has at most 2 (more preferably at most 1) amino acid differences total in the CDRs thereof when compared to a corresponding reference (anifrolumab) antibody, with the proviso that there is at most 2 amino acid differences per CDR. More preferably a variant antibody has at most 2 (more preferably at most 1) amino acid differences total in the CDRs thereof when compared to a corresponding reference (anifrolumab) antibody, with the proviso that there is at most 1 amino acid difference per CDR.

The amino acid difference may be an amino acid substitution, insertion or deletion. In one embodiment the amino acid difference is a conservative amino acid substitution as described herein.

In one embodiment a variant antibody may have at most 5, 4 or 3 amino acid differences total in the framework regions thereof when compared to a corresponding reference (anifrolumab) antibody, with the proviso that there is at most 2 (preferably at most 1) amino acid differences per framework region. Preferably a variant antibody has at most 2 (more preferably at most 1) amino acid differences total in the framework regions thereof when compared to a corresponding reference (anifrolumab) antibody, with the proviso that there is at most 2 amino acid differences per framework region. More preferably a variant antibody has at most 2 (more preferably at most 1) amino acid differences total in the framework regions thereof when compared to a corresponding reference (anifrolumab) antibody, with the proviso that there is at most 1 amino acid difference per framework region.

Thus, a variant antibody may comprise a variable heavy chain and a variable light chain as described herein, wherein:
- the heavy chain has at most 14 amino acid differences (at most 2 amino acid differences in each CDR and at most 2 amino acid differences in each framework region) when compared to a heavy chain sequence herein; and
- the light chain has at most 14 amino acid differences (at most 2 amino acid differences in each CDR and at most 2 amino acid differences in each framework region) when compared to a light chain sequence herein;
wherein the variant antibody binds to the same target antigen as the reference (anifrolumab) antibody (e.g. IFNAR) and preferably with the same affinity.

The variant heavy or light chains may be referred to as "functional equivalents" of the reference heavy or light chains.

In one embodiment a variant antibody may comprise a variable heavy chain and a variable light chain as described herein, wherein:
- the heavy chain has at most 7 amino acid differences (at most 1 amino acid difference in each CDR and at most 1 amino acid difference in each framework region) when compared to a heavy chain sequence herein; and
- the light chain has at most 7 amino acid differences (at most 1 amino acid difference in each CDR and at most 1 amino acid difference in each framework region) when compared to a light chain sequence herein;
wherein the variant antibody binds to the same target antigen as the reference (anifrolumab) antibody (e.g. IFNAR) and preferably with the same affinity.

Thus, in one embodiment the type I interferon receptor inhibitor is anifrolumab or a functional variant thereof.

### SRI (Systemic Lupus Erythematosus Responder Index of ≥4)

A subject achieves SRI(4) if all of the following criteria are met:
- Reduction from baseline of ≥4 points in the SLEDAI-2K;
- No new organ system affected as defined by 1 or more BILAG-2004 A or 2 or more
- BILAG-2004 B items compared to baseline using BILAG-2004;
- No worsening from baseline in the subjects' lupus disease activity defined by an increase ≥0.30 points on a 3-point PGA VAS.

SRI(X) (X=5, 6, 7, or 8) is defined by the proportion of subjects who meet the following criteria:
- Reduction from baseline of ≥X points in the SLEDAI-2K;
- No new organ systems affected as defined by 1 or more BILAG-2004 A or 2 or
- more BILAG-2004 B items compared to baseline using BILAG-2004;
- No worsening from baseline in the subjects' lupus disease activity defined by an
- increase ≥0.30 points on a 3-point PGA VAS

### BILAG-2004, British Isles Lupus Assessment Group-2004

The BILAG-2004 is a translational index with 9 organ systems (General, Mucocutaneous, Neuropsychiatric, Musculoskeletal, Cardiorespiratory, Gastrointestinal, Ophthalmic, Renal and Haematology) that is able to capture changing severity of clinical manifestations. It has ordinal scales by design and does not have a global score; rather it records disease activity across the different organ systems at a glance by comparing the immediate past 4 weeks to the 4 weeks preceding them. It is based on the principle of physicians' intention to treat and categorises disease activity into 5 different levels from A to E:
- Grade A represents very active disease requiring immunosuppressive drugs and/or a prednisone dose of >20 mg/day or equivalent
- Grade B represents moderate disease activity requiring a lower dose of corticosteroids, topical steroids, topical immunosuppressives, antimalarials, or NSAIDs
- Grade C indicates mild stable disease
- Grade D implies no disease activity but the system has previously been affected
- Grade E indicates no current or previous disease activity

Although the BILAG-2004 was developed based on the principle of intention to treat, the treatment has no bearing on the scoring index. Only the presence of active manifestations influences the scoring.

### BICLA: BILAG-Based Composite Lupus Assessment (BICLA)

BICLA is a composite index that was originally derived by expert consensus of disease activity indices. BICLA response is defined as (1) at least one gradation of improvement in baseline BILAG scores in all body systems with moderate or severe disease activity at entry (e.g., all A (severe disease) scores falling to B (moderate), C (mild), or D (no activity) and all B scores falling to C or D); (2) no new BILAG A or more than one new BILAG B scores; (3) no worsening of total SLEDAI score from baseline; (4) no significant deterioration (≤10%) in physicians global assessment; and (5) no treatment failure (initiation of non-protocol treatment).

Particularly, a subject is a BICLA responder if the following criteria are met:
- Reduction of all baseline BILAG-2004 A to B/C/D and baseline BILAG-2004 B to C/D, and no BILAG-2004 worsening in other organ systems, as defined by 1 new BILAG-2004 A or more than 1 new BILAG-2004 B item;
- No worsening from baseline in SLEDAI-2K as defined as an increase from baseline of >0 points in SLEDAI-2K;
- No worsening from baseline in the subjects' lupus disease activity defined by an increase ≥0.30 points on a 3-point PGA VAS;
- No discontinuation of investigational product or use of restricted medications beyond the protocol-allowed threshold before assessment

In particular embodiments, treatment using anifrolumab improves a subject's BICLA response rate by at least week 8, week 12, week 24, week 36, week 48, or week 52 of treatment. In particular embodiments, treatment using anifrolumab improves a subject's BICLA response rate by at least week 8.

In particular embodiments, while the subject shows improvement in the BICLA response, the subject does not show improvement in the Systemic Lupus Erythematosus Responder Index (SRI)4 score.

In particular embodiments provided herein is a method of treating systemic lupus erythematosus in a subject in need thereof, comprising administering to the subject a therapeutically effective amount of anifrolumab, wherein the treatment results in an improvement of the BILAG-Based Composite Lupus Assessment (BICLA) response rate compared to a patient receiving placebo. The improvement of the BILAG response rate may be statistically significant. The improvement of the BILAG response rate may be statistically significant after multiplicity adjustment. The improvement of the BILAG response rate may be statistically significant, wherein statistical significance is determined by p <0.05 or p<0.005.

### Patient reported outcomes (PROs)

In particular embodiments, treatment using anifrolumab results in an MCR. In particular embodiments, treatment using anifrolumab results in a PCR.

The SF-36-v2 (acute) is a multipurpose, 36-item survey that measures 8 domains of health: physical functioning, role limitations due to physical health, bodily pain, general health perceptions, vitality, social functioning, role limitations due to emotional problems, and mental health. It yields scale scores for each of these 8 health domains, and summary measures of physical and mental health: the Physical Component Summary and Mental Component Summary.

The FACIT-F is a 13-item subject-completed questionnaire to assess the impact of fatigue over the previous 7 days. The responses range from 0 (Not at All) to 4 (Very Much). Final scores are the sum of the responses and range from 0 to 52; higher scores indicate better QoL (Yellen et al, 1997). Changes in scores >3 points are considered to be clinically meaningful.

The PtGA is a single-item question that takes into account all the ways in which illness and health conditions may affect the patient at this time. The patient should consider the previous week when answering this question. Responses range from very well to very poor on a 100 mm VAS. The physician and subject must complete the PGA and PtGA, respectively, independently of each other.

A Major Clinical Response (MCR) includes BICLA scores C or better at Week 24, maintained with no new A or B scores between Week 24-52. A Partial Clinical Response (PCR) includes a maximum of 1 BICLA score at Week 24, maintained without new A or >1 new B domain score through Week 52.

Physician Global Assessment (PGA) of Disease Activity refers to an assessment wherein a physician evaluates the status of a subject's psoriatic arthritis (PsA) by means of a visual analog scale (VAS). The subject is assessed according to how their current arthritis is. The VAS is anchored with verbal descriptors of "very good" to "very poor."

The method may comprise measuring PROs in the subject before and after administration of anifrolumab. The PRO's may comprise the subject's Functional Assessment of Chronic Illness Therapy-Fatigue (FACIT-F), Short Form 36 Health Survey version 2 (SF-36-v2), mental component summary (MCS), and/or SF-36, physical component summary (PCS) score.

### EXAMPLES

The Examples that follow are illustrative of specific embodiments of the disclosure, and various uses thereof. They are set forth for explanatory purposes only and should not be construed as limiting the scope of the disclosure in any way.

### Example 1: Type I Interferon Inhibition in Active Systemic Lupus Erythematosus

In TULIP-1 (NCT02446912), a phase 3, double-blind, randomized controlled study of adults with moderate to severe SLE despite standard-of-care (SOC) treatment, patients received (2:1:2) placebo or anifrolumab (150 or 300 mg) intravenously every 4 weeks for 48 weeks. Stable SOC continued except for mandatory attempts at oral corticosteroid (OCS) tapering for patients receiving prednisone/equivalent ≥10 mg/day at baseline. The primary endpoint was the difference between the SLE Responder Index (SRI[4]) response rate for anifrolumab 300 mg and placebo at week 52. Safety was also assessed.

Patients were aged 18-70 years and fulfilled the American College of Rheumatology classification criteria for SLE (Hochberg MC. Arthritis Rheum 1997; 40(9): 1725; Tan EM, et al. Arthritis Rheum 1982; 25(11): 1271-7). Requirements included an SLE Disease Activity Index 2000 (SLEDAI-2K) (Gladman DD, et al. J Rheumatol 2002; 29(2): 288-91) ≥6 (excluding points from fever, lupus headache, or organic brain syndrome), a clinical SLEDAI-2K ≥4 (excluding points from laboratory results); British Isles Lupus Assessment Group (BILAG)-2004 (Isenberg DA, et al. Rheumatology (Oxford) 2005; 44(7): 902-6) organ domain scores of ≥1 A and/or ≥2 B items (Yee CS, Cresswell L, et al. Rheumatology (Oxford) 2010; 49(9): 1665-9); and Physician's Global Assessment (PGA) (Petri M, et al. N Engl J Med 2005; 353(24): 2550-8) of disease activity score ≥1 (0-3 scale). Patients were seropositive for antinuclear antibodies or anti-double-stranded DNA (anti-dsDNA) or anti-Smith antibodies and were receiving stable treatment with at least one of the following: prednisone or equivalent, an antimalarial, azathioprine, mizoribine, mycophenolate mofetil/mycophenolic acid, or methotrexate.

To allow adequate time for the investigational product to achieve clinical benefit, Investigators were permitted to administer 1 burst and taper of corticosteroids between week 0 (day 1) and week 12 for increased SLE disease activity/non-SLE activity. Patients who received more than 1 steroid burst and taper from week 0 (day 1) to week 12 or who violated any of the criteria above could continue in the study but were considered nonresponders for subsequent assessments of disease activity, regardless of whether the OCS burst was administered for increased SLE activity or non-SLE causes. Between week 12 and week 40, an increase in corticosteroid dose for increased SLE activity was not allowed. A patient receiving a steroid dose above his or her week 0 (day 1) dose could continue in the study, but was considered a nonresponder for subsequent assessments of disease activity. No increase in OCS was allowed after week 40 (except for the management of adverse events (AEs) or as a prophylaxis for adrenal insufficiency). Patients who received an increase in their OCS after week 40 were considered nonresponders for subsequent assessments of disease activity.

Beginning at week 8 and continuing through week 40, steroid tapering to an OCS dose of ≤7.5 mg/day was required to be attempted in all patients with OCS dose ≥10.0 mg/day at baseline, unless at least 1 of the following criteria were met:
SLEDAI-2K activity that worsened compared with baseline in major organ systems (renal, central nervous system, cardiopulmonary, vasculitis, fever, thrombocytopenia, or hemolytic anemia, or gastrointestinal activity)
Newly affected organ system(s) based on the Systemic Lupus Erythematosus Disease Activity Index 2000 (SLEDAI-2K), excluding serological abnormalities (double-stranded DNA [dsDNA] antibodies, hypocomplementemia)
Moderate to severe skin disease as reflected by a Cutaneous Lupus Erythematosus Disease Area and Severity Index activity score of ≥10
Moderate to severe arthritis disease as reflected by an active joint count of ≥8 tender and/or swollen joints
Type I IFNGS classification at screening was determined by a central laboratory with an analytically validated 4-gene (*IFI27, IFI44*, *IFI44L, RSAD2*) quantitative polymerase chain reaction-based test from whole blood (Furie R, Arthritis Rheum 2017; 69(2): 376-86; Yao Y, et al. Arthritis Res Ther 2010; 12 Suppl 1: S6). Based on phase 2 pharmacokinetic/pharmacodynamic modeling and benefit-risk profile, anifrolumab 300 mg was the chosen therapeutic dosage; a 150-mg dosage was included to elucidate dose-response. Infusions were administered every 4 weeks through week 48, with final assessments at week 52.

The primary efficacy evaluation was the difference in percentages of patients receiving anifrolumab 300 mg or placebo who achieved SLE Responder Index (SRI)(4) ³⁰ responses at week 52. SRI(4) response is a landmark assessment defined as a ≥4-point reduction in SLEDAI-2K, <1 new BILAG-2004 A or <2 new BILAG-2004 B organ domain scores, <0·3-point increase in PGA from baseline, no use of restricted medications beyond protocol-allowed thresholds, and no discontinuation of investigational product.

Key secondary endpoints were adjusted for multiplicity and included the percentages of patients with week-52 SRI(4) responses in the IFNGS test-high subgroup; sustained OCS dosage reduction to ≤7.5 mg/day from week 40 to 52 among patients with baseline dosage ≥10 mg/day; **≥**50% reduction in week-12 Cutaneous Lupus Erythematosus Disease Area and Severity Index (CLASI) ³¹ activity in patients with baseline CLASI ≥10; and week-24 SRI(4) responses; as well as annualized flare rates through week 52 (≥1 new BILAG-2004 A or ≥2 new BILAG-2004 B organ domain scores versus the previous visit ³².

Prespecified secondary endpoints not adjusted for multiplicity included the following: week 52 BILAG-based Composite Lupus Assessment (BICLA) response, higher thresholds of week-52 SRI (5-9), change in Physician's Global Assessment (PGA) from baseline to week 52, change in SLEDAI-2K from baseline to week 52, change in BILAG global score, **≥**50% reduction in CLASI activity score from baseline to week 52, and reductions in active joint counts (≥50% reduction and change joint count from baseline to week 52). In addition, week 52 SRI(4) responses in the anifrolumab 150 mg group were assessed.

Safety assessments included adverse events, laboratory assessments, and vital signs. A 21-gene assay assessed pharmacodynamic neutralization of type I IFNGS. Anti-dsDNA, C3, C4, CH50, and anti-drug antibodies (ADA) were measured.

Efficacy analyses included patients who were randomized and received ≥1 dose of study treatment; patients were analyzed according to randomized treatment (modified intention-to-treat). Planned efficacy analyses compared anifrolumab 300 mg with placebo. The primary endpoint compared anifrolumab 300 mg and placebo week-52 SRI(4) responses using a stratified Cochran-Mantel-Haenszel (CMH) test with the same stratification factors used at randomization. To adjust for stratification factors, the CMH method uses a weighted average across strata of the stratum-specific difference in proportions, where the strata are defined based on the 8 possible combinations of the 3 factors. Key secondary endpoints were analyzed similarly, except flare rate, which was analyzed using a negative binomial regression model. A weighted Holm procedure with predetermined weights was used to control the family-wise type I error rate at 0.05 across the primary and key secondary endpoints. This procedure splits the alpha of 0.05 according to predefined weights and, after initial null hypothesis rejections, recycles the corresponding alpha in proportion to these weights.

SRI(4) response rates were compared for anifrolumab 150 mg versus placebo using the CMH analysis strategy described previously. Outcomes that were continuous variables were modeled using repeated measures and adjusting for baseline value, treatment group, visit, treatment by visit interaction, and stratification factors.

Rules specifying restricted medications were prospectively defined and incorporated in the efficacy endpoints. The original rules used to classify responders based on nonsteroidal anti-inflammatory drug (NSAID) use were inconsistent with the intention of the protocol and inappropriately classified patients who used new NSAID or had an increase in NSAID dosage as nonresponders for all binary response endpoints, even if NSAID use was transient or early in the year-long trial. These rules did not have any impact on study conduct (i.e., did not affect medical decisions, treatment of patients, or data collection; they affected only analysis of the data). After unblinding, a group of SLE experts and the sponsor evaluated the clinical appropriateness of all restricted medication rules and revised them. Key analyses were repeated (post hoc) using the amended restricted medication rules and are presented alongside the original analyses. An additional post hoc analysis exploring the time to first BICLA response sustained through week 52 was performed using a Cox proportional hazards model.

The original restricted medication rules indicated new initiation or increases in doses of antimalarials and/or immunosuppressants after day 1 led to nonresponse (NR) classification for all subsequent efficacy assessments. For corticosteroids the following criteria applied:
Dosages above a protocol-defined maximum at any time always led to classification as NR post-receipt for all subsequent efficacy assessments.
Patients receiving more than one steroid burst during the first 12 weeks of treatment or who violated any of the criteria defining a burst and taper were classified as NR for subsequent efficacy assessments, regardless of the reason for the burst (SLE or non-SLE activity).
Between week 12 and week 40, one steroid burst was allowed only to treat non-SLE symptoms. Patients violating this criterion were classified as NR for subsequent efficacy assessments.
Increases in dosages after week 40 led to classification as NR for subsequent efficacy assessments.
Patients were classified as NR for all subsequent efficacy for NSAIDS.

The overall intent of the revised medication rules used in the post hoc analysis was to align medication rules with clinically appropriate use early in the trial, with a stricter interpretation closer to the primary endpoint. This revision would prevent clinically appropriate medication use early in the trial from leading to classification as a nonresponder (NR) at week 52, while ensuring that if patients took medications that could confound efficacy assessment at week 52, they would be classified as NR at week 52. For antimalarials and/or immunosuppressants, increases in dosage (or new initiation) at any time led to NR imputation for the duration of the trial. For oral corticosteroids the following criteria was applied:
Dosages above a protocol-defined maximum dosage at any time always led to classification as NR post-receipt
One steroid burst and taper was allowed before week 12; exceeding this led to classification as NR for the subsequent 12 weeks

In general, dosages above baseline were not allowed between weeks 12 and 40; exceeding baseline dosage led to classification as NR for the remainder of the trial

After week 40, dosages greater than the week-40 dosage or use of moderately to highly potent topical corticosteroids led to classification as NR

For NSAIDs, initiation of a new NSAID within 14 days of week 52 and documented use on the day before the week-52 assessment led to classification as NR at the week-52 visit. NSAID use prior to week 50 did not lead to classification as NR.

457 patients were randomized (anifrolumab 300 mg, n=180; 150 mg, n=93; placebo, n=184). Week 52 SRI(4) attainment was similar for anifrolumab 300 mg (36.2%, 65/180) and placebo (40.4%, 74/184; P=0.41) (FIG 1A; Table 1). Similarly, for SRI(4) at week 24 and SRI(4) in the high IFNGS patients, the anifrolumab and placebo groups did not differ. In patients with baseline OCS ≥10 mg/day, sustained dosage reduction to ≤7.5 mg/day was achieved by 41.0% (42/103) for anifrolumab 300 mg and 32.1% (33/102) for placebo (difference 8.9 [95% Cl: -4.1, 21.9]). In patients with CLASI activity ≥10 at baseline, ≥50% reduction in CLASI at week 12 was achieved by 41.9% (24/58) of anifrolumab 300 mg patients and 24.9% (14/54) of placebo patients (difference 17.0 [95% Cl: (-0.3, 34.3]). Annualized flare rates were 0.60 for anifrolumab and 0.72 for placebo (rate ratio 0.83 [95% Cl: (0.60, 1.14]). British Isles Lupus Assessment Group-based Composite Lupus Assessment (BICLA) response was achieved by 37.1% (67/180) of patients receiving anifrolumab 300 mg versus 27.0% (49/184) for placebo (difference 10.1 [95% confidence interval (Cl): 0.6, 19.7]).

**Table 1. Primary, Key Secondary, and Supporting Secondary Efficacy Outcomes, Including Outcomes Analyzed With Amended Restricted Medication Rules to Determine Nonresponse**

| **Endpoint** | | **Prespecified Analysis** | | | | **Analysis With Amended Rules for Restricted Medications** | | | |
|---|---|---|---|---|---|---|---|---|---|
| | | **Placebo (n=184)** | **Anifrolumab 300 mg (n=180)** | **Difference (95% CI)*** | **Nomin al P-value** | **Placebo (n=184)** | **Anifrolum ab 300 mg (n=180)** | **Difference (95% CI)*** | **Nomin al P-value** |
| **Primary and key secondary outcomes** | | | | -4.2 (-14.2, 5.8) | | | | | |
| | SRI(4) wk 52, n/N (%)^{†} | 74/184 (40.4) | 65/180 (36.2) | P=0.41^{‡} | 0.41 | 79/184 (43.0) | 84/180 (46.9) | 3.9 (-6.3, 14.1) | 0.455 |
| | SRI(4) wk 52 in IFNGS test-high patients, n/N (%)^{§} | 59/151 (39.3) | 53/148 (35.9) | -3.4 (-14.4, 7.6) | 0.55 | 63/151 (41.8) | 71/148 (48.2) | 6.4 (-4.8, 17.7) | 0.261 |
| | SRI(4) wk 24, n/N (%)^{§} | 75/184 (40.9) | 74/180 (41.5) | 0.6 (-9.4, 10.6) | 0.905 | 79/184 (43.1) | 83/180 (46.4) | 3.3 (-6.7, 13.4) | 0.515 |
| | Sustained OCS reduction to target at wk 52, n/N (%)^{§, ∥} | 33/102 (32.1) | 42/103 (41.0) | 8.9 (-4.1, 21.9) | 0.180 | 33/102 (32.1) | 50/103 (48.8) | 16.7 (3.5, 29.8) | 0.013 |
| | ≥50% reduction in CLASI activity score from BL to wk 12, n/N (%)^{§,¶} | 14/54 (24.9) | 24/58 (41.9) | 17.0 (-0.3, 34.3) | 0.054 | 14/54 (24.9) | 25/58 (43.6) | 18.7 (1.4, 36.0) | 0.034 |
| | Annualized flare rate through wk 52^{§,††, ‡‡} | 0.72 | 0.60 | 0.83 (0.60, 1.14) | 0.258 | 0.72 | 0.60 | 0.83 (0.60, 1.14) | 0.258 |

| **Supporting disease outcomes** | | | | | | | | | |
|---|---|---|---|---|---|---|---|---|---|
| | BICLA response at wk 52, n/N (%) | 49/184 (27.0) | 67/180 (37.1) | 10.1 (0.6, 19.7) | N/A | 54/184 (29.6) | 83/180 (46.1) | 16.4 (6.7, 26.2) | N/A |
| | SRI(5) wk 52, n/N (%) | 55/184 (30.2) | 54/179 (30.2) | 0 (-9.5, 9.6) | N/A | 58/184 (31.7) | 72/179 (40.4) | 8·6 (-1.2, 18.5) | N/A |
| | SRI(6) wk 52, n/N (%) | 55/184 (30.2) | 51/179 (28.6) | -1.6 (-11.1, 7.8) | N/A | 58/184 (31.7) | 69/179 (38.7) | 7.0 (-2.8, 16.8) | N/A |
| | SRI(7) wk 52, n/N (%) | 29/176 (16.5) | 37/173 (21.6) | 5.1 (-3.7, 13.8) | N/A | 31/176 (17.6) | 50/173 (29.0) | 11.5 (2.4, 20.6) | N/A |
| | SRI(8) wk 52, n/N (%) | 26/174 (14.9) | 36/173 (21.0) | 6.1 (-2.5, 14.7) | N/A | 29/174 (16.5) | 49/173 (28.5) | 11.9 (2.9, 21.0) | N/A |
| | PGA change from BL to wk 52, LS mean (SE)^{§§} | n=149 | n=143 | | | | | | |
| | | -0.89 (0.052) | -1.11 (0.053) | -0.22 (-0.36, - 0.08) | N/A | N/A | N/A | N/A | N/A |
| | SLEDAI-2K change from BL to wk 52, LS mean (SE)^{§§} | n=149 | n=143 | | | | | | |
| | | -5.3 (0.33) | -6.0 (0.34) | -0.7 (-1.6, 0.2) | N/A | N/A | N/A | N/A | N/A |
| | BILAG global score change from BL to wk 52, mean (SD)^{§§} | -10.7 (7.72) | -13.0 (8.01) | N/A | N/A | N/A | N/A | N/A | N/A |
| | **≥**50% reduction in CLASI activity score from BL to wk 52, n/N (%)^{¶} | 23/54 (43.1) | 33/58 (57.3) | 14.2 (-3.7, 32.2) | -3.7, N/A | 32.2 24/54 (44.6) | 38/58 (66.0) | 21.4 (3.7, 39.1) | N/A |
| | **≥**50% reduction in active (swollen and tender) joints at wk 52, n/N (%)** | 22/68 (32.3) | 33/70 (47.0) | 14.7 (-1.4, 30.8) | N/A | 22/68 (32.3) | 37/70 (53.0) | 20.7 (4.7, 36.7) | N/A |
| | Active joint count (swollen plus tender) change from BL to wk 52, LS mean (SE)^{§§} | n=150 | n=142 | | | | | | |
| | | -4.8 (0.24) | -5.2 (0.24) | -0.4 (-1.0, 0.2) | N/A | N/A | N/A | N/A | N/A |

| | | | | | | | | | |
|---|---|---|---|---|---|---|---|---|---|
| *For responder rates, the difference in response rates and associated 95% Cls are weighted and calculated using a stratified Cochran-Mantel-Haenszel approach. ^{†}Primary endpoint. SRI(4) response was defined as a ≥4-point reduction in SLEDAI-2K score, <1 new BILAG-2004 A or <2 new BILAG-2004 B organ domain scores, <0·3-point (10%) increase in PGA score from baseline, and no discontinuation of investigational product and no use of restricted medications beyond the protocol-allowed threshold. ^{‡}Because the primary endpoint was not statistically significant, per the prespecified analysis plan, all other comparisons are nonsignificant. ^{§}Key secondary endpoint. ^{∥}In patients with baseline OCS ≥10 mg/day (prednisone or equivalent). ^{¶}In patients with CLASI activity score ≥10 at baseline. **In patients with ≥8 swollen and ≥8 tender joints at baseline. ^{††}A flare is defined as either ≥1 new BILAG-2004 A or ≥2 new BILAG-2004 B items compared with the previous visit (i.e., a worsening from an E, D, or C score to a B score in at least two organ systems or a worsening from an E, D, C, or B score to an A score in any one organ system compared with the previous visit). ^{‡‡}Calculation of flare rate does not involve restricted medications; therefore, values for the prespecified and post hoc analyses are the same. ^{§§}Modeled using repeated measures and adjusting for baseline value, treatment group, visit, treatment by visit interaction, and stratification factors. | | | | | | | | | |

Abbreviations: BICLA = BILAG-based Composite Lupus Assessment; BILAG = British Isles Lupus Assessment Group; BL = baseline; Cl = confidence interval; CLASI = Cutaneous Lupus Erythematosus Disease Area and Severity Index; IFNGS = interferon gene signature; LS = least squares; N/A = not applicable; OCS = oral corticosteroids; PGA = Physician's Global Assessment; SD = standard deviation; SE = standard error; SLEDAI-2K = Systemic Lupus Erythematosus Disease Activity Index 2000; SRI = Systemic Lupus Erythematosus Responder Index; wk = week.

In the IFNGS test-high subpopulation (375/457, 82.1% of the study population), SRI(4) responses at week 52 were similar for patients who received anifrolumab 300 mg (35.9%, 53/148) and placebo (39.3%, 59/151; difference -3.4 [95% Cl: -14.4, 7.6]).

Of patients receiving prednisone or equivalent ≥10 mg/day at randomization, a numerically greater percentage of the anifrolumab 300-mg group (41.0%, 42/103) than the placebo group (32.1%, 33/102) achieved OCS dosage reduction to the target (≤7.5 mg/day), sustained from week 40 to 52 (difference 8.9 [95% CI: -4.1, 21.9]) **(FIG's 3A and 3B).** Of patients with CLASI activity scores ≥10 at baseline, ≥**50%** reduction at week 12 was achieved by 41.9% (24/58) in the anifrolumab 300-mg group versus 24.9% (14/54) in the placebo group (difference 17.0 [95% Cl: -0.3, 34.3]) **(****FIG 1C****, Table** 1). SRI(4) results at week 24 were similar to results at week 52. The BILAG-based annualized flare rate was numerically lower for anifrolumab than placebo (0.60 versus 0.72, respectively; rate ratio 0.83 [95% Cl: 0.60, 1.14]).

Supporting secondary measures of disease improvement are shown in the lower section of Table 1. BICLA response, a rigorous composite global disease measure, was achieved by numerically more patients at week 52 in the anifrolumab 300-mg group (37.1%, 67/180) than the placebo group (27.0%, 49/184; difference 10.1 [95% Cl: 0.6, 19.7]; **FIG 1E****, Table 1).** Although SRI(4) responses appeared similar across treatment groups, higher thresholds of SRI slightly favored anifrolumab **(Table 1).** SLEDAI-2K and BILAG global scores both had greater numeric change from baseline to week 52 in the anifrolumab group than the placebo group **(Table 1).** Likewise, numerically greater improvement in PGA scores occurred in the anifrolumab group than in the placebo group (LS mean change from baseline of -1.11 [SE=0.053] and - 0.89 [SE=0.052], respectively, difference -0.22 [95% Cl: -0.36, -0.08]). Numeric differences in favor of anifrolumab were also observed for supporting measures of CLASI and 2 other joint count measures (Table 1).

In the anifrolumab 150-mg group, week-52 SRI(4) responses were similar to placebo (37.6% [35/93] and 40.4% [74/184], respectively; difference -2.6 [95% Cl: -14.7, 9.6]). Additional efficacy outcomes for the anifrolumab 150-mg group are presented in **Table 2.**

**Table 2. Anifrolumab 150-mg Group: Efficacy Outcomes, Including Those Using Prespecified and Amended Rules for Determining Nonresponse Due to Restricted Medication Use**

| Endpoint* | Prespecified Analysis | Analysis With Amended Rules for Restricted Medications |
|---|---|---|
| | Anifrolumab 150 mg (n=93) | Anifrolumab 150 mg (n=93) |
| SRI(4) wk 52, n/N (%)^{†} | 35/93 (37.6) | 45/93 (48.4) |
| SRI(4) wk 52 in IFNGS test-high patients, n/N (%) | 30/76 (39.5) | 40/76 (52.6) |
| SRI(4) wk 24, n/N (%) | 34/93 (36.6) | 40/93 (43.1) |
| Sustained OCS reduction to target wk 52, n/N (%)^{‡} | 17/48 (35.2) | 24/48 (49.6) |
| **≥**50% reduction in CLASI activity score from BL to wk 12, n/N (%)^{§} | 15/30 (50) | 16/30 (53.3) |
| Annualized flare rate through wk 52 (95% Cl)^{∥, ¶} | 0.62 (0.44, 0.87) | 0.62 (0.44, 0.87) |
| BICLA response wk 52, n/N (%) | 27/93 (29.0) | 35/93 (37.7) |
| SRI(5) wk 52, n/N (%) | 29/93 (31.2) | 39/93 (42.0) |
| SRI(6) wk 52, n/N (%) | 28/93 (30.1) | 36/93 (38.7) |
| SRI(7) wk 52, n/N (%) | 23/85 (27.0) | 29/85 (34.0) |
| SRI(8) wk 52, n/N (%) | 21/85 (24.6) | 27/85 (31.6) |
| PGA change from BL to wk 52, LS mean (SE)^{†} | n=77 -1.07 (0.072) | N/A |
| SLEDAI-2K change from BL to wk 52, LS mean (SE)^{§§} | n=78 | N/A |
| | -5.5 (0.46) | |
| BILAG global score change from BL to wk 52, mean (SD)^{§§} | n=78 | N/A |
| | -11.0 (8.43) | |
| **≥**50% reduction in CLASI activity score from BL to wk 52 in patients with CLASI score ≥10 at BL, n/N (%)^{§} | 12/30 (40.0) | N/A |
| ≥50% reduction in active (swollen and tender) joints at wk 52, n/N (%)^{**} | 17/36 (47.6) | 21/36 (59.1) |
| Active joint count change from BL to wk 52, LS mean (SE)^{†} | n=78 | N/A |
| | -5.3 (0.32) | |

| | | |
|---|---|---|
| *For responder rates, the difference in response rates and associated 95% Cls are weighted and calculated using a stratified Cochran-Mantel-Haenszel approach. ^{†}SRI(4) response was defined as a ≥4-point reduction in SLEDAI-2K score, <1 new BILAG-2004 A or <2 new BILAG-2004 B organ domain scores, <0·3-point (10%) increase in PGA score from baseline, and no discontinuation of investigational product and no use of restricted medications beyond the protocol-allowed threshold. ^{‡}In patients with baseline OCS ≥10 mg/day (prednisone or equivalent). ^{§}In patients with CLASI activity score ≥10 at baseline. ^{∥}A flare is defined as either ≥1 new BILAG-2004 A or ≥2 new BILAG-2004 B items compared with the previous visit (ie, a worsening from an E, D, or C score to a B score in at least two organ systems or a worsening from an E, D, C, or B score to an A score in any one organ system compared with the previous visit). ^{¶}Calculation of flare rate does not involve restricted medications; therefore, values for the prespecified and post hoc analyses are the same. **In patients with ≥8 swollen and ≥8 tender joints at baseline. | | |

Abbreviations: BICLA = BILAG-based Composite Lupus Assessment; BILAG = British Isles Lupus Assessment Group; BL = baseline; Cl = confidence interval; CLASI = Cutaneous Lupus Erythematosus Disease Area and Severity Index; IFNGS = interferon gene signature; OCS = oral corticosteroids; PGA = Physician's Global Assessment; SLEDAI-2K = Systemic Lupus Erythematosus Disease Activity Index 2000; SRI = Systemic Lupus Erythematosus Responder Index; wk = week.

In the prespecified SRI(4) analysis, the original restricted medication rules classified approximately 8% of the study population as nonresponders because of new or increased NSAID use. Applying the posthoc amended restricted medication rules decreased the number of patients classified as nonresponders on this basis **(Table 1,** right panel). Achievement of the primary endpoint, SRI(4), was numerically higher under these rules, but the difference in response rates between anifrolumab and placebo remained similar (anifrolumab 300 mg 46.9%; placebo 43.0%; difference 3.9 [95% Cl: -6.3, 14.1]; **FIG 1B****, Table 1).** Similar patterns occurred for SRI(4) response at week 24 and SRI(4) at week 52 in the IFNGS high subgroup.

In patients receiving prednisone or equivalent ≥10 mg/day at randomization, sustained OCS reduction to ≤7.5 mg/day was achieved by more patients treated with anifrolumab (48.8%, 50/103) than placebo (32.1%, 33/102; difference 16.7 [95% Cl: 3.5, 29.8]; **Table 1;** **FIG 3****).** CLASI responses in the predefined subset of patients were more frequent in those treated with anifrolumab (43.6%, 25/58) than those receiving placebo (24.9%, 14/54; difference 18.7 [95% Cl: 1.4, 36.0]; **FIG 1D****, Table 1).**

Although SRI(4) responses were similar across treatment groups, greater differences were observed between anifrolumab and placebo using modified SRIs that incorporated higher thresholds of SLEDAI-2K response (Table 1). For example, SRI(7) response was achieved by 29.0% of anifrolumab-treated patients versus 17.6% of patients receiving placebo (difference 11.5 [95% Cl: 2.4, 20.6]). BICLA responses were achieved by numerically more patients treated with anifrolumab (46.1%) than placebo (29.6%; difference 16.4 [95% Cl: 6.7, 26.2]; **FIG 1F****, Table 1).** Differences in the time to attainment of sustained BICLA responses suggest that patients receiving anifrolumab had a 93% higher likelihood of achieving response that was sustained to week 52 than patients receiving placebo (hazard ratio 1.93; 95% Cl: 1.38, 2.73; FIG 2A). Supporting endpoints for skin and joint responses also had numerically greater improvements in the anifrolumab group (Table 1). For example, **≥**50% joint count improvements in the predefined subset of patients were more common with anifrolumab (53.0%, 37/70) than placebo (32.3%, 22/68; difference 20.7 [95% Cl: 4.7, 36.7]).

In patients with high type I IFN pharmacodynamic signatures at baseline (fold change >2) who received anifrolumab 300 mg, neutralization of the IFNGS was seen early in treatment (median percentage of baseline signature at week 12, 12.6% [median absolute deviation=6.5], i.e., 87.4% suppression of IFNGS) and maintained through week 52 **(****FIG 2B****).** No IFNGS neutralization was observed with placebo, and minimal suppression was observed with anifrolumab 150 mg. Anti-dsDNA and C3 levels trended toward normal with anifrolumab 300-mg treatment **(Table 3; FIG's 4A-4B).** The percentages of patients who were ADA negative at baseline and ADA positive at any time post baseline were small and similar across treatment groups (anifrolumab 300 mg, 5/164 patients [3.0%]; placebo, 7/171 patients [4.1%]). The numbers of patients who were persistently ADA positive (defined as ADA negative at baseline and ADA positive at ≥2 assessments with ≥16 weeks between the first and last positive test) were 3/164 (1.8%) in the anifrolumab 300-mg group and 4/171 (2.3%) in the placebo group.

Greater percentages of patients had at least one adverse event in the anifrolumab 300-mg and 150-mg groups (161/180, 89.4% and 79/93 84.9%, respectively) than in the placebo group (144/184, 78.3%). The frequencies of serious adverse events were similar across treatment groups, with no events predominating.

**Table 3 Interferon Gene Signature Suppression, Immune Biomarkers, and Immunogenicity Results**

| **Biomarker*** | | | **Placebo (n=184)** | **Anifrolumab 150 mg (n=93)** | **Anifrolumab 300 mg (n=180)** |
|---|---|---|---|---|---|
| Percentage of baseline IFN 21-gene pharmacodynamic signature in patients with elevated 21-gene PD signature at baseline | | | n=114 | n=56 | n=97 |
| | Mean (SD) | | 115·343 (136·4202) | 122·841 (349·6918) | 30·322 (55·6731) |
| | Median (minimum, maximum) | | 89·178 (3·80, 1304·72) | 63·216 (4·82, 2656·28) | 11·771 (0.99, 324-75) |
| Change from BL in anti-dsDNA antibodies in patients with elevated dsDNA at baseline, U/mL | | | n=62 | n=35 | n=62 |
| | Mean (SD) | | 64·0 (407·98) | -101-4 (525 87) | -63·8 (158·28) |
| | Median (minimum, maximum) | | -6·6 (-1090·17, 2299·00) | -21·0 (-2026·52, 1702·60) | -19·6 (-1101.07, 72·10) |
| Change from BL complement concentration in patients with abnormal complement levels at baseline | | | | | |
| | C3, g/L | | n=49 | n=29 | n=48 |
| | | Mean (SD) | 0.0381 (0·16987) | 0.1099 (0·21515) | 0.1505 (0·16111) |
| | | Median (minimum, maximum) | 0·0410 (-0·311, 0·392) | 0·0760 (-0·195, 0·604) | 0·1305 (-0·176, 0·796) |
| | C4, g/L | | n=30 | n=15 | n=28 |
| | | Mean (SD) | 0.0255 (0·04803) | 0·0173 (0·03976) | 0.0236 (0·02889) |
| | | Median (minimum, maximum) | 0·0125 (-0·013, 0·229) | 0·0030 (-0·027, 0·120) | 0·0190 (-0·015, 0·118) |
| | CH50, CH50 units | | n=8 | n=12 | n=12 |
| | | Mean (SD) | 104·9 (84·50) | 114.6 (102·81) | 130·3 (114·48) |
| | | Median (minimum, maximum) | 89·0 (20, 235) | 93 5 (-43, 348) | 112·5 (-23, 380) |
| ADA positive any visit, n/N (%) | | | 15/184 (8.2) | 7/93 (7.5) | 17/179 (9.5) |
| ADA positive only post-baseline, n/N (%) | | | 7/171 (4.1) | 2/85 (2.4) | 5/164 (3.0) |
| Persistently ADA positive,^{†} n/N (%) | | | 4/171 (2.3) | 0/85 | 3/164 (1.8) |

| | | | | | |
|---|---|---|---|---|---|
| *Measured at wk 52 unless otherwise indicated. ^{†}Treatment-induced ADA detected at two or more assessments (with ≥16 weeks between first and last positive) or detected at last assessment (in patients who are ADA negative at baseline). | | | | | |

Abbreviations: ADA = anti-drug antibody; BL = baseline; C3 = third complement; C4 = fourth complement; CH50 = total hemolytic complement levels; dsDNA = double-stranded DNA; IFNGS = interferon gene signature; SD = standard deviation, wk = week.

The SRI(4) was chosen as TULIP-1's primary endpoint because of its wide use and the positive outcome in the anifrolumab phase 2 MUSE trial. In TULIP-1, SRI(4) response rates were no different between the placebo and anifrolumab groups; however, results of other endpoints, including another composite endpoint, BICLA, suggest clinical benefit of anifrolumab. Although SRI(4) and BICLA comprise the same components, each of these composite endpoints may be optimal in different situations³³. SRI(4) is based on the SLEDAI-2K, which requires complete resolution of a manifestation before that item's score will change. Therefore, SRI(4) cannot capture partial resolution within an organ domain, even if such improvement is clinically meaningful³⁴. In contrast, BICLA is based on improvements in BILAG-2004, which registers graded changes within an organ domain, and therefore, is more sensitive to detecting improvement. For example, rash in the SLEDAI-2K mucocutaneous domain has a weight of 2 points, making it impossible to achieve SRI(4) response based on this manifestation alone even if a patient experiences complete resolution of rash; BICLA, in contrast, captures partial or complete resolution of rash as a response, as does CLASI. Another distinction between BICLA and SRI is that BICLA reflects only clinical improvements, whereas SRI(4) response can be achieved with serologic improvements alone. Impact on serologies is more likely to be observed with therapies that target antibody-producing cells more directly.

The potential for reduction in OCS use during anifrolumab treatment is a particularly important outcome of TULIP-1. OCS are commonly used for SLE management, despite a range of serious adverse effects associated with their use that increase with longer duration and higher dosages³⁵. In the current study, of the anifrolumab-treated patients who were taking high-dosage OCS (prednisone or equivalent ≥10 mg/day) at baseline, 48.8% were able to achieve sustained dosage reduction to a target of ≤7.5 mg/day, compared with 32.1% of patients receiving placebo (using amended restricted medication rules). Skin and joint disease are among the most common SLE manifestations, as reflected at baseline in the TULIP-1 population, and the data suggest that treatment with anifrolumab may improve both of these manifestations. Of patients with greater baseline skin disease activity (CLASI ≥10), more anifrolumab-treated patients achieved **≥**50% reduction in CLASI activity scores than placebo-treated patients (44% versus 25%, respectively, using amended medication rules). Similarly, among patients with greater baseline joint disease activity (≥8 swollen and ≥8 tender joints), more anifrolumab-treated patients achieved **≥**50% decreases in swollen and tender joint counts than placebo-treated patients (53% versus 33%, respectively, using amended medication rules).

Although the trial did not achieve its primary endpoint, SRI(4), anifrolumab 300 mg resulted in numeric improvements relative to placebo in attaining multiple other global and organ-specific endpoints, including BICLA, Cutaneous Lupus Erythematosus Disease Area and Severity Index (CLASI), joint count, and corticosteroid tapering.

### Example 2: Efficacy and Safety of Anifrolumab in Active Systemic Lupus Erythematosus

TULIP-2, a phase 3, randomized, double-blind, placebo-controlled trial, evaluated efficacy and safety of intravenous anifrolumab 300 mg vs placebo (1:1) every 4 weeks for 48 weeks in patients with moderate to severe SLE despite standard-of-care (SOC) treatment. Week-52 BICLA response was the primary endpoint. SOC was stable except for mandatory attempts at oral corticosteroid (OCS) tapering to prednisone equivalent ≤7.5 mg/day for patients receiving ≥10 mg/day at entry.

Patients were 18-70 years of age and fulfilled American College of Rheumatology classification criteria for SLE³⁶. Patients had moderate to severe SLE as measured by SLEDAI 2000 (SLEDAI-2K³⁷ score ≥6 (excluding points attributable to fever, lupus headache, or organic brain syndrome) and clinical SLEDAI-2K score ≥4 (excluding points from laboratory results). They also had either severe disease activity in ≥1 organ or moderate activity in ≥2 organs (measured by BILAG-2004 ³⁸) organ domain scores of ≥1 A item or ≥2 B items³⁹ and Physician's Global Assessment (PGA) of disease activity ≥1 on a visual analog scale from 0 (none) to 3 (severe disease). At screening, patients were seropositive for antinuclear antibodies, anti-double-stranded DNA (anti-dsDNA) antibodies, or anti-Smith antibodies and had received stable standard-of-care (SOC) treatment for SLE with at least 1 of the following: prednisone or equivalent, an antimalarial, azathioprine, mizoribine, mycophenolate mofetil/mycophenolic acid, or methotrexate. Type I interferon gene signature (IFNGS) classification at screening was determined by a central laboratory with an analytically validated 4-gene (*IFI27, IFI44*, *IFI44L, RSAD2*) quantitative polymerase chain reaction-based test from whole blood ⁴⁰. Patients with active severe lupus nephritis or neuropsychiatric SLE were excluded.

To allow adequate time for the investigational product to achieve clinical benefit, Investigators were permitted to administer 1 burst and taper of corticosteroids between week 0 (day 1) and week 12 for increased SLE disease activity/non-SLE activity. Patients who received more than 1 steroid burst and taper from week 0 (day 1) to week 12 or who violated any of the criteria above could continue in the study but were considered nonresponders for subsequent assessments of disease activity, regardless of whether the OCS burst was administered for increased SLE activity or non-SLE causes. Between week 12 and week 40, an increase in corticosteroid dose for increased SLE activity was not allowed. A patient receiving a steroid dose above his or her week 0 (day 1) dose could continue in the study, but was considered a nonresponder for subsequent assessments of disease activity. No increase in OCS was allowed after week 40 (except for the management of AEs or as a prophylaxis for adrenal insufficiency). Patients who received an increase in their OCS after week 40 were considered nonresponders for subsequent assessments of disease activity.

Beginning at week 8 and continuing through week 40, steroid tapering to an OCS dose of ≤7.5 mg/day was required to be attempted in all patients with OCS dose ≥10.0 mg/day at baseline, unless at least 1 of the following criteria were met:
- SLEDAI-2K activity that worsened compared with baseline in major organ systems (renal, central nervous system, cardiopulmonary, vasculitis, fever, thrombocytopenia, or hemolytic anemia, or gastrointestinal activity)
- Newly affected organ system(s) based on the Systemic Lupus Erythematosus Disease Activity Index 2000 (SLEDAI-2K), excluding serological abnormalities (double-stranded DNA [dsDNA] antibodies, hypocomplementemia)
- Moderate to severe skin disease as reflected by a Cutaneous Lupus Erythematosus Disease Area and Severity Index activity score of ≥10
- Moderate to severe arthritis disease as reflected by an active joint count of ≥8 tender and/or swollen joints

The primary efficacy evaluation was the difference between percentages of patients who achieved BICLA response at week 52 in the anifrolumab 300 mg and placebo groups. BICLA response is defined as all of (i) reduction of all severe or moderately severe baseline disease activity (BILAG-2004 A or B) to lower levels (B/C/D or C/D, respectively) and no worsening in other organ systems (defined as ≥1 new BILAG-2004 A or ≥2 new BILAG-2004 B items); (ii) confirmation of no worsening in disease activity measured by SLEDAI-2K (no increase from baseline); (iii) <0.3-point increase in PGA from baseline; (iv) no use of restricted medications beyond protocol-allowed thresholds, and (v) no discontinuation of investigational product. BICLA response, rather than SRI(4) response, was designated as the primary endpoint in a protocol amendment, prior to unblinding, because of its metric properties and consistent discrimination between treatment arms in previous anifrolumab trials.

Key secondary endpoints were adjusted for multiplicity and included the percentages of patients achieving a) BICLA response at week 52 among patients who were IFNGS test-high at baseline; b) OCS dosage reduction to ≤7.5 mg/day sustained from week 40 to week 52 among patients with baseline dosage ≥10 mg/day; c) **≥**50% reduction in Cutaneous Lupus Erythematosus Disease Area and Severity Index (CLASI) ³¹ at week 12 among patients with moderate to severe cutaneous activity (CLASI ≥10) at baseline; and d) ≥50% reduction in both swollen and tender joint counts at Week 52 among patients with ≥6 swollen and ≥6 tender joints at baseline. Annualized flare rate through 52 weeks (≥1 new BILAG-2004 A or ≥2 new BILAG-2004 B scores compared with the previous visit)³² was also a key secondary endpoint.

Exploratory endpoints that were not adjusted for multiplicity included SRI(4) to SRI(8) responses, time to first flare, and time to onset of sustained BICLA response.

Efficacy analyses included all patients who were randomized and received ≥1 dose of study treatment. The primary endpoint compared BICLA response rates at week 52 for the anifrolumab and placebo groups using a stratified Cochran-Mantel-Haenszel (CMH) test with the same stratification factors as for randomization (SLEDAI-2K, OCS dosage at baseline, and type I IFNGS). Raw numbers of responders are reported alongside response percentages and Cls that are adjusted using CMH. Key secondary endpoints and other response endpoints were analyzed similarly, except flare rate, which was analyzed using a negative binomial regression model. A weighted Holm procedure with predetermined weights was used to control the family-wise type I error rate at 0.05 across the primary and key secondary endpoints. Weights were chosen based on estimated power and relative clinical importance: BICLA in patients with high baseline IFNGS (0.8), OCS reduction (0.06), CLASI response (0.06), reduction in joint counts (0.06), and flare rate (0.02). Other prespecified exploratory outcomes were not controlled for multiplicity. Time to onset of BICLA response sustained through week 52, and time to flare, were evaluated using a Cox proportional hazards model.

BICLA response at week 52 was achieved in more patients on anifrolumab 300 mg (47.8%, 86/180) than placebo (31.5%, 57/182; difference 16.3 [95% confidence interval [Cl]: 6.3, 26.3; P=0.001]) **(Table 4).** Exploring BICLA response overtime, a numerically greater proportion of patients treated with anifrolumab achieved BICLA at all assessed timepoints **(****FIG 5A****),** and a sustained BICLA response was achieved earlier in anifrolumab-treated patients **(****FIG 5A****).**

**Table 4. Primary, Key Secondary, and Other Secondary Efficacy Outcomes**

| Endpoint | | Placebo (n=182) n/N (%)* | Anifrolumab 300 mg (n=180) n/N (%)* | Difference (95% CI)* | Adjusted P-value /Unadjusted *P*-value^{‡} | Significant after multiplicity adjustment |
|---|---|---|---|---|---|---|
| **Primary endpoint:** | | | | | | |
| | BICLA response at wk 52^{†} | 57/182 (31.5) | 86/180 (47.8) | 16.3 (6.3, 26.3) | 0.001/ 0.001 | NA |

| **Key secondary endpoints:** | | | | | | |
|---|---|---|---|---|---|---|
| | BICLA response at wk 52 in type I IFNGS test-high patients | 46/151 (30.7) | 72/150 (48.0) | 17.3 (6.5, 28.2) | 0.002/ 0.002 | Yes |
| | OCS reduction to target sustained from wk 40 to wk 52^{∥} | 25/83 (30.2) | 45/87 (51.5) | 21.2 (6.8, 35.7) | 0.014/ 0.004 | Yes |
| | ≥50% reduction in CLASI activity from BL to wk 12 ^{¶} | 10/40 (25.0) | 24/49 (49.0) | 24.0 (4.3, 43.6) | 0.039/ 0.017 | Yes |
| | ≥50% reduction in both swollen and tender joints from BL to wk 52** | 34/90 (37.5) | 30/71 (42.2) | 4.7 (-10.6, 20.0) | 0.547/ 0.547 | No |
| | Annualized flare rate through wk 52^{††} | 0.64 | 0.43 | 0.67^{∥∥} (0.48, 0.94) | 0.081/ 0.020 | No |

| **Other secondary endpoints:** | | | | | | |
|---|---|---|---|---|---|---|
| | SRI(4) at wk 52^{‡‡} | 68/182 (37.3) | 100/180 (55.5) | 18.2 (8.1, 28.3) | NA | NA |
| | SRI(5) at wk 52^{‡‡} | 51/181 (28.1) | 79/180 (44.0) | 15.9 (6.1, 25.8) | NA | NA |
| | SRI(6) at wk 52^{‡‡} | 48/181 (26.4) | 79/180 (44.0) | 17.6 (7.8, 27.3) | NA | NA |
| | SRI(7) at wk 52^{‡‡} | 34/169 (20.0) | 56/167 (33.6) | 13.6 (4.0, 23.2) | NA | NA |
| | SRI(8) at wk 52^{‡‡} | 33/167 (19.6) | 50/166 (30.2) | 10.7 (1.2, 20.2) | NA | NA |

| | | | | | | |
|---|---|---|---|---|---|---|
| *Percentage response, the difference in response estimates, and the associated 95% CIs are weighted and are calculated using a stratified Cochran-Mantel-Haenszel approach with stratification factors (SLEDAI-2K score at screening [<10 points vs ≥10 points], Week 0 OCS dose [<10 mg/day vs ≥10 mg/day prednisone or equivalent] and type I IFN gene signature test result at screening [high vs low]). ^{†}BICLA response is defined as reduction of all baseline BILAG-2004 A to B/C/D, baseline BILAG-2004 B to C/D, and no BILAG-2004 worsening in other organ systems (≥1 new BILAG-2004 A or ≥2 new BILAG-2004 B items); no worsening (increase >0 points) from baseline SLEDAI-2K; <0.3-point increase in PGA from baseline, no use of restricted medications beyond protocol-allowed thresholds, and no discontinuation of investigational product. ^{‡}Adjusted P-value for comparison of treatment groups using a stratified Cochran-Mantel-Haenszel approach/Nominal P-value for treatment comparison. ^{∥}In patients with baseline OCS ≥10 mg/day prednisone or equivalent. ^{¶}In patients with CLASI activity score ≥10 at baseline. ^{**}In patients with ≥6 swollen and ≥6 tender joints at baseline. ^{††}Values are annualized flare rates rather than responder percentages. A flare is defined as either ≥1 new BILAG-2004 A or ≥2 new BILAG-2004 B items compared with the previous visit (i.e., a worsening from an E, D, or C score to a B score in at least 2 organ systems or a worsening from an E, D, C, or B score to an A score in any 1 organ system compared with the previous visit). ^{‡‡}SRI(4) response was defined as a ≥4-point reduction in SLEDAI-2K score, <1 new BILAG-2004 A or <2 new BILAG-2004 B organ domain scores, <0.3-point (10%) increase in PGA score from baseline, and no discontinuation of investigational product and no use of restricted medications beyond the protocol-allowed threshold. SRI(5), SRI(6), SRI(7), and SRI(8) responses are defined similarly to SRI(4) response with the exception that the point reduction in SLEDAI-2K score is ≥5, ≥6, ≥7, or ≥8 for SRI(5), (6), (7), and (8), respectively. ^{∥∥}Calculated as a rate ratio (anifrolumab/placebo). | | | | | | |

In the IFNGS test-high subpopulation (301/362, 83.1% of patients overall), the BICLA response rate at Week 52 was greater in the anifrolumab group than in the placebo group (difference 17.3 [95% Cl: 6.5, 28.2], adjusted *P*=0.002]) **(Table 4).** Among patients receiving prednisone or equivalent ≥10 mg/day at randomization (47.0%, 170/362), a difference in the proportion of patients who achieved sustained reduction to ≤7.5 mg/day was seen (anifrolumab 51.5%, 45/87; placebo 30.2%, 25/83; difference 21.2 [95% Cl: 6.8, 35.7], adjusted P=0.014) (**Table 4;** **FIG 5B**). Among patients with at least moderately active skin disease (CLASI ≥10) at baseline, ≥50% CLASI reduction at week 12 was achieved by more anifrolumab-treated patients (49.0%, 24/49) than placebo-treated patients (25.0%, 10/40; difference 24.0 [95% Cl: 4.3, 43.6], adjusted P=0.039) (**FIG 5C**). The percentages of patients with ≥6 swollen and ≥6 tender joints at baseline who achieved a ≥50% decrease in both swollen and tender joint counts at 52 weeks were similar for anifrolumab (42.2%, 30/71) and placebo (37.5%, 34/90) groups (difference 4.7 [95% Cl: -10.6, 20.0], adjusted P=0.55). BILAG-based annualized flare rate was numerically lower for anifrolumab than for placebo (0.43 vs. 0.64, respectively; rate ratio 0.67 [95% Cl: 0.48, 0.94]) (adjusted P=0.081), and exploratory analysis of time to first flare favored anifrolumab (hazard ratio, 0.65; 95% Cl: 0.46, 0.91) (**FIG 6B**).

SRI(4) response at week 52 was numerically greater in the anifrolumab group (55.5%, 100/180) than the placebo group (37.3%, 68/182; difference 18.2 [95% Cl: 8.1, 28.3]) (**Table 4**)**.** For each of the higher thresholds of SRI (5-8), numeric differences in favor of anifrolumab were observed.

In patients who were type I IFNGS test-high at baseline who received anifrolumab (150/180, 83.3%), neutralization of the IFNGS was achieved early in treatment (median percentage neutralization at week 12 = 88.0%) and was maintained through week 52 (**Table 5;** **FIG 7**). No IFNGS neutralization was observed with placebo treatment. Among patients with abnormal anti-dsDNA at baseline, levels tended toward normalization with anifrolumab treatment. Among patients with abnormal (low) complement levels at baseline, numerically greater increases were observed in the anifrolumab. Among anifrolumab-treated patients who were ADA negative at baseline, few were ADA positive at any time post baseline (1/170 patients [0.6%]).

Adverse events were reported for 159/180 (88.3%) anifrolumab-treated patients and 153/182 (84.1%) placebo-treated patients.

**Table 5. Interferon Gene Signature Suppression, Immune Biomarkers, and Immunogenicity Results**

| Biomarker* | | | Placebo (n=182) | Anifrolumab 300 mg (n=180) |
|---|---|---|---|---|
| Percentage of baseline IFN 21-gene pharmacodynamic signature in patients with elevated 21-gene PD signature at baseline | | | n=111 | n=121 |
| | Mean (SD) | | 153.64 | 25.66 |
| | Median | | 99.93 | 9.94 |
| Change from BL in anti-dsDNA antibodies in patients with elevated dsDNA at baseline, U/mL | | | n=49 | n=71 |
| | Mean (SD) | | -17.60 | 8.89 |
| | Median | | -4.65 | -12.56 |
| Change from BL complement concentration in patients with abnormal complement levels at baseline | | | | |
| | C3, g/L | | n=58 | n=64 |
| | | Mean (SD) | 0.0448 (0.16194) | 0.1132 (0.19471) |
| | | Median | 0.0475 | 0.0890 |
| | C4, g/L | | n=33 | n=43 |
| | | Mean (SD) | 0.0071 (0.01854) | 0.0225 (0.03761) |
| | | Median | 0.0060 | 0.0100 |
| | CH50, CH50 units | | n=13 | n=14 |
| | | Mean (SD) | 87.6 (67.1) | 95.1 (79.7) |
| | | Median | 75.0 | 106.0 |
| ADA positive any visit, n/N (%) | | | 20/182 (11.0) | 8/180 (4.4) |
| ADA positive only post-baseline, n/N (%) | | | 9/167 (5.4) | 1/170 (0.6) |
| Persistently ADA positive, n/N (%) | | | 3/167 (1.8) | 1/170 (0.6) |

| | | | | |
|---|---|---|---|---|
| *Measured at wk 52 unless otherwise indicated. ^{†}Treatment-induced ADA detected at 2 or more assessments (with ≥16 weeks between first and last positive) or detected at last assessment (in patients who are ADA negative at baseline). | | | | |

Abbreviations: ADA = anti-drug antibody; BL = baseline; C3 = third complement; C4 = fourth complement; CH50 = total hemolytic complement levels; dsDNA = double-stranded DNA; IFNGS = interferon gene signature; SD = standard deviation, wk = week.

### Example 2. Early sustained response

Early and Sustained Responses With Anifrolumab Treatment in Patients With Active Systemic Lupus Erythematosus (SLE) in 2 Phase 3 Trials

### Background

In the phase 3 TULIP-2 and TULIP-1 trials in SLE, treatment with the type I interferon receptor antibody anifrolumab resulted in higher percentages of patients with BICLA responses vs placebo at Week 52, with differences of 16.3% (primary endpoint; P=0.001, 95% CI 6.3-26.3) and 16.4% (secondary endpoint; 95% CI 6.7-26.2), respectively.

### Objective

To better understand the time course of BICLA responses to anifrolumab, we examined responses over time compared with placebo in TULIP-2 and TULIP-1, including those that were sustained from attainment through Week 52. Major clinical response (MCR) and partial clinical response (PCR) were also assessed as alternative outcome measures. In particular, to compare BICLA responses to anifrolumab vs placebo over time in TULIP-1, TULIP-2, and pooled TULIP data at early time points, the time to onset of response sustained to Week 52 and the major and partial clinical response. The Major clinical response is defined as all BILAG-2004 scores C or better at Week 24, maintained through Week 52, with no new A or B scores between Weeks 24-52. The Partial clinical response is defined as a maximum of 1 BILAG-2004 B score at Week 24, maintained through Week 52, with no new A or >1 new B domain score through Week 52.

### Methods

The TULIP-2 and TULIP-1 randomized, double-blind, placebo-controlled trials evaluated the efficacy and safety of anifrolumab (300 mg Q4W) over 52 weeks in patients with moderately to severely active SLE who were receiving standard-of-care treatment. Time to onset of BICLA response that was sustained from attainment through Week 52 was evaluated using a Cox proportional hazards model. MCR was defined as all BILAG-2004 scores C or better at Week 24, maintained with no new A or B scores between Weeks 24-52. PCR was defined as ≤1 BILAG-2004 B score at Week 24 maintained without a new A or >1 new B domain score through Week 52. For TULIP-1, BICLA response rate and time to onset of BICLA response were analyzed using the amended restricted medication rules; MCR and PCR were analyzed using the prespecified analysis plan.

### Results

There were more BICLA responders with anifrolumab vs placebo from early time points (**FIG. 9****,** **FIG. 11****,** **FIG. 12****).** The time to onset of sustained BICLA response favoured anifrolumab (**FIG. 11****,** **FIG. 12**). There were numerical differences favouring anifrolumab in the percentage of patients with sustained BICLA responses and the percentage of patients with PCR and MCR (FIG 13). Overall, 180 patients each in TULIP-2 and TULIP-1 received anifrolumab compared with 182 and 184 patients in the placebo arms, respectively. At the first 3 assessments in TULIP-2 (Weeks 4, 8, and 12), numerically greater percentages of patients treated with anifrolumab (26.8%, 35.3%, and 42.9%, respectively) were classified as having a BICLA response compared with placebo (21.3%, 21.6%, and 31.8%). A similar trend was observed in TULIP-1 with anifrolumab (23.3%, 34.2%, and 36.5%) vs placebo (18.3%, 23.2%, and 27.5%). The time to onset of BICLA response sustained from onset through Week 52 favoured anifrolumab in both TULIP-2 (HR 1.55, 95% CI 1.11-2.18) and TULIP-1 (HR 1.93, 95% Cl 1.38-2.73). In TULIP-2, 86 (47.8%) patients treated with anifrolumab had BICLA responses that were sustained from time of onset through Week 52 compared with 57 (31.3%) patients in the placebo group. In TULIP-1, 85 (47.2%) patients in the anifrolumab treatment arm had BICLA responses that were sustained from time of onset through Week 52 compared with 55 (29.9%) patients in the placebo group. In TULIP-2 and TULIP-1, MCR was observed in 20.8% and 22.1% of patients treated with anifrolumab, respectively, compared with 10.9% and 15.8% of those who received placebo. PCR was observed in 46.8% and 45.4% of anifrolumab-treated patients compared with 38.4% and 40.2% in the placebo groups, respectively.

### Conclusion

Rapid and durable BICLA responses support the clinical benefit of anifrolumab for patients with moderately to severely active SLE. In 2 Phase 3 studies, a greater proportion of patients achieved BICLA responses sustained from onset through Week 52 with anifrolumab treatment compared with placebo. Anifrolumab resulted in numerically favorable differences in time to onset of BICLA responses maintained through Week 52 across the TULIP studies. MCR and PCR also favored anifrolumab. These data support the sustainability of clinical benefit derived from anifrolumab treatment of patients with active SLE.

### Example 3: Efficacy in patient subgroups

### Objective

To compare BICLA responses to anifrolumab vs placebo through Week 52 in protocol-defined subgroups of patients in TULIP-1 and TULIP-2 and across pooled TULIP-1 and TULIP-2 data. Baseline characteristics are shown in Tables 6 and 7.

### Results

There was a robust BICLA response rates observed at Week 52 with anifrolumab across prespecified subgroups in TULIP-1, TULIP-2, and pooled TULIP data. There was no substantive impact on effect size of demographics (**FIG 14-16**), baseline disease activity (FIG 17), baseline OCS dosage (FIG 18), baseline Type I IFNGS test status (**FIG 19**). The response rate to anifrolumab was similar in IFNGS test-high and - low patients (**FIG 19**).

**Table 6: Baseline Patient Demographics**

| **Baseline patient demographics** | | **Pooled TULIP^{a}** | |
|---|---|---|---|
| | | **Placebo (n=366)** | **Anifrolumab 300 mg (n=360)** |
| **Age, mean (SD), years** | | **41.0 (11.9)** | **42.6 (12.0)** |
| **Female, n (%)** | | **341 (93.2)** | **333 (92.5)** |
| **BMI, n (%)** | **≤28 kg/m²** | 223 (60.9) | 205 (56.9) |
| | **>28 kg/m²** | 143 (39.1) | 155 (43.1) |
| **Race,^{b} n (%)** | **White** | **244 (66.7)** | **235 (65.3)** |
| | **Black/African American** | **48 (13.1)** | **46 (12.8)** |
| | **Asian** | **35 (9.6)** | **41 (11.4)** |
| | **Other** | 31 (8.5) | 30 (8.3) |
| **Geographic region,^{c} n (%)** | **Asia-Pacific** | 32 (8.7) | 38 (10.6) |
| | **Europe** | 122 (33.3) | 115 (31.9) |
| | **Latin America** | 57 (15.6) | 59 (16.4) |
| | **USA/Canada** | 140 (38.3) | 139 (38.6) |
| | **Rest of world** | 15 (4.1) | 9 (2.5) |

BMI, body mass index; IFNGS, type I interferon gene signature; OCS, oral corticosteroid; SD, standard deviation; SLEDAI-2K, Systemic Lupus Erythematosus Disease Activity Index 2000; qPCR, quantitative polymerase chain reaction.

**Table 7: Baseline Disease Characteristics**

| **Baseline disease characteristics** | | | **Pooled TULIP^{a}** | |
|---|---|---|---|---|
| | | | **Placebo (n=366)** | **Anifrolumab 300 mg (n=360)** |
| | **SLEDAI-2K (screening)** | **Mean (SD)** | **11.3 (3.6)** | **11.4 (3.9)** |
| | | **≥10, n (%)** | 260 (71.0) | 251 (69.7) |
| | | **<10 mg/day** | 181 (49.5) | 170 (47.2) |
| | **OCS dosage, n (%)** | **≥10 mg/day** | **185 (50.5)** | **190 (52.8)** |
| | **Type** I **IFNGS status,^{d} n (%)** | **High** | **302 (82.5)** | **298 (82.8)** |
| | | **Low** | 64 (17.5) | 62 (17.2) |

### Example 4: Flare assessments

### Background

Anifrolumab treatment resulted in improved British Isles Lupus Assessment Group (BILAG)-based Composite Lupus Assessment (BICLA) response rates in patients with systemic lupus erythematosus (SLE) in the phase 3 TULIP-2 and TULIP-1 trials. In addition, annualized flare rates were lower among the groups treated with anifrolumab compared with placebo

### Objective

TULIP-2 and TULIP-1 data were analyzed to assess the effects of anifrolumab on the number of SLE flares and time to first flare during 52 weeks of treatment.

### Methods

The randomized, double-blind, placebo-controlled TULIP-2 and TULIP-1 trials evaluated efficacy and safety of intravenous anifrolumab 300 mg vs placebo every 4 weeks for 48 weeks, with the primary endpoints assessed at Week 52, in patients with moderate to severe SLE despite standard-of-care treatment. Flares were defined as ≥1 new BILAG-2004 A or ≥2 new (worsening) BILAG-2004 B domain scores compared with the prior month's visit. Time to first flare was evaluated using a Cox proportional hazards model. Annualized flare rate was analyzed using a negative binomial regression model.

### Results

In TULIP-2 (anifrolumab, n=180; placebo, n=182) and TULIP-1 (anifrolumab, n=180; placebo, n=184), fewer patients experienced ≥1 BILAG-2004 flare in the anifrolumab groups (TULIP-2: 31.1%, n=56; TULIP-1: 36.1%, n=65) compared with the placebo groups (TULIP-2: 42.3%, n=77; TULIP-1: 43.5%, n=80; **FIG** 20). Results favoring anifrolumab were observed in time to first flare (TULIP-2: hazard ratio [HR] 0.65, 95% confidence interval [CI] 0.46-0.91 and TULIP-1: HR 0.76, 95% CI 0.55-1.06; **FIG 21**) and BILAG-based annualized flare rates (TULIP-2: adjusted rate ratio 0.67, 95% CI 0.48-0.94 and TULIP-1: rate ratio 0.83, 95% CI 0.60-1.14) across both trials. Annualized BILAG flare rates (vs prior visit) were significantly lower in the anifrolumab group compared with the placebo group in TULIP-2 (**FIG 22**). Fewer patients experienced ≥1 BILAG flare vs the prior visit in the anifrolumab group in TULIP-1, TULIP-2 and pooled TULIP (36.1%, 31.1%, and 33.6%, respectively) compared with placebo group (43.5%, 42.3%, and 42.9%, respectively; **FIG 23**)

### Conclusions

Across 2 phase 3 trials, we observed reductions in the total number of flares and annualized flare rates, as well as prolongation of time to first flare with anifrolumab treatment compared with placebo. These results support the potential of anifrolumab to reduce disease activity and reduce flares, benefiting patients with SLE. The results of the TULIP trials support the capacity of anifrolumab to not only reduce disease activity but also to reduce flares in the presence of OCS taper, an attribute that is vital to the long-term management of patients with SLE.

### Example 5: Understanding BICLA

### Background

The BILAG-based Composite Lupus Assessment (BICLA) is a validated composite global measure of SLE disease activity that registers both partial and complete improvement within organ systems. BICLA has been used as an endpoint in the phase 3 TULIP-1 and TULIP-2 trials of anifrolumab. This study investigated the relationship between BICLA response and clinical/laboratory assessments of SLE in TULIP-1 and TULIP-2, irrespective of treatment.

### Methods

This was a post-hoc analysis of the randomized, double-blind TULIP-1 and TULIP-2 trials. Patients with moderately to severely active SLE, despite standard-of-care, were randomized to receive anifrolumab (150 or 300 mg IV Q4W) or placebo for 48 weeks, with the primary endpoint assessed at Week 52. BICLA responses were defined by all of the following: reduction of baseline BILAG-2004 A and B domain scores to B/C/D and C/D, respectively, and no worsening in any organ system; no worsening of the SLE Disease Activity Index 2000 (SLEDAI-2K) score; no worsening of ≥0.3 points in the Physician's Global Assessment (PGA; range 0-3). Maintained oral corticosteroid (OCS) dosage reduction was defined as OCS dosage of ≤7.5 mg/day achieved by Week 40 and maintained through Week 52. Cutaneous Lupus Erythematosus Disease Area and Severity Index activity (CLASI-A) response was defined as ≥50% reduction of CLASI-A score from baseline for patients with CLASI-A ≥10 at baseline.

### Results

Baseline characteristics were generally comparable in BICLA responders (N=318) and non-responders (N=501). Overall, improved outcomes were observed in BICLA responders vs nonresponders, including numerically greater improvements in SLEDAI-2K (-7.4 [SD: 3.64] vs -4.2 [4.28]) from baseline to Week 52. Greater mean OCS daily dose reduction was observed in BICLA responders vs nonresponders (-5.4 [SD:6.84] vs -1.7 [8.08]) from baseline to Week 52 and maintained OCS dosage reduction was achieved by more BICLA responders vs nonresponders (79.2% vs 19.1%) at Week 52. Reduction in CLASI-A score was achieved by more BICLA responders vs nonresponders (92.0% vs 23.2%) at Week 52. Greater reduction of mean anti-dsDNA levels was observed in BICLA responders vs nonresponders (-46.1 [SD: 335.69] vs 15.8 [450.92]) from baseline to Week 52; numeric improvements were also observed for the complement proteins, C3 and C4.

### Conclusions

BICLA response is associated with improvements in multiple clinical and laboratory measures of SLE, strengthening its value as a clinically meaningful primary endpoint in SLE trials. The treatment of subjects with SLE with the type I IFN inhibitor anifrolumab in two Phase III clinical trial resulted in a BICLA response in patients associated with other improvements in multiple clinical and laboratory measures of SLE.

### Example 5: Early and sustained reduction in severity of skin disease as measured by CLASI

### Background

Skin is the second most commonly involved organ in SLE, with up to 85% of patients experiencing skin disease. The Cutaneous Lupus Erythematosus Disease Area and Severity Index (CLASI) is a validated index to measure skin disease severity with activity scores (CLASI-A) ranging from 0 (mild) to 70 (severe). CLASI-A includes measures for erythema, scale/hypertrophy, mucous membrane lesions, recent hair loss, and nonscarring alopecia. In the phase 3 TULIP-1 and -2 trials of patients with SLE, a greater proportion of patients with CLASI-A at baseline ≥10 achieved a ≥50% reduction of CLASI-A at Week 12 with anifrolumab compared with placebo. We further evaluated the effect of anifrolumab on skin-specific SLE disease activity using data pooled from TULIP-1 and -2.

### Methods

TULIP-1 and -2 were 52-week randomized, double-blind, placebo-controlled trials that evaluated the efficacy and safety of anifrolumab (300 mg IV every 4 weeks for 48 weeks) in patients with moderately to severely active SLE despite standard-of-care treatment. TULIP-1 and -2 were analyzed separately using restricted medication rules per the TULIP-2 protocol, and data from both trials were pooled. We compared skin responses over time in patients receiving anifrolumab vs placebo. A CLASI-A response was defined as ≥50% reduction of CLASI-A score from baseline for patients with CLASI-A ≥10. Time to CLASI-A response was evaluated using a Cox proportional hazards model.

### Results

In total, 360 patients received anifrolumab and 366 received placebo. At baseline, mean (SD) CLASI-A score was 8.1 (7.41); 95.9% (696/726) of patients had baseline CLASI-A >0 and 27.7% (201/726) had baseline CLASI-A ≥10. In the subgroup of patients with baseline CLASI-A ≥10, CLASI-A response (≥50% reduction) was achieved by Week 8 for 36.0% (38/107) of patients receiving anifrolumab vs 21.7% (21/94) receiving placebo (difference 14.3; 95% CI 1.8%, 26.9%). (FIG 24). Results favoring anifrolumab were observed in time to CLASI-A response sustained to Week 52 in TULIP-1 (hazard ratio [HR] 1.91; 95% CI 1.14, 3.27) and TULIP-2 (HR 1.55; 95% CI 0.87, 2.85) (**FIG 25**). Of the subgroup of patients with baseline CLASI-A >0, a greater number of patients achieved a CLASI-A response (≥50% reduction) by Week 12 in the anifrolumab vs placebo groups in both TULIP-1 and -2 (nominal P<0.05) (**FIG 26**); a similar effect was observed in the subgroup of patients with baseline CLASI-A ≥10 in both TULIP-1 and -2 (nominal P<0.05) (**FIG 26**). An example from one patient following treatment with anifrolumab (300 mg) is shown in **FIG 27****.**

### Conclusions

Anifrolumab treatment was associated with rapid and durable improvements in skin-specific SLE disease activity, as assessed by CLASI, in a subgroup of patients with mild to severe cutaneous activity at baseline. These findings demonstrate the ability of anifrolumab to reduce skin disease activity in patients with moderately to severely active SLE.

### Example 6: Clinical relevance of BILCA

### Background

The British Isles Lupus Assessment Group-based Composite Lupus Assessment (BICLA) is a validated global measure of treatment response in systemic lupus erythematosus (SLE) clinical trials. To understand the relevance of BICLA to clinical practice, the relationship between BICLA response and routine SLE assessments and patient-reported outcomes (PROs) was investigated.

The BICLA was developed following an expert panel review of disease activity indices used in SLE clinical trials. A BICLA response requires improvement in all domains affected at baseline, assessed by BILAG-2004, no worsening of other BILAG-2004 domains, no worsening versus baseline of both SLEDAI-2K and PGA, no initiation of nonprotocol treatment or use beyond protocol-allowed thresholds, and no discontinuation of investigational product. Thus, in contrast to the SRI, the driver of efficacy in the BICLA is BILAG-2004, whereas worsening is assessed with SLEDAI-2K and PGA in addition to BILAG. BILAG-2004-based BICLA weighs organ systems equally and distinguishes between inactive disease, partial and complete improvement, and deterioration of disease activity, whereas SLEDAI-2K-based SRI assigns weighting to organ systems and requires complete resolution of disease activity in the involved organ system to capture improvement.

Composite SLE assessments are not routinely used in clinical practice. The relevance of treatment response assessed in this way thus may not be appreciated by clinicians. We therefore investigated the relationship between BICLA response and other SLE disease measures that are meaningful in real-world clinical practice, including flares, oral glucocorticoid daily dosage and sustained oral glucocorticoid taper, PROs, medical resource utilization, and clinical and laboratory measures of global and organ-specific disease. These relationships were assessed between BICLA responders and nonresponders using pooled data from the phase 3 TULIP-1 and TULIP-2 trials of anifrolumab, regardless of treatment group assignment.

### Methods

### Patients and Study Design

This was a *post hoc* analysis of pooled data from the phase 3 randomized, placebo-controlled, double-blind, 52-Week TULIP-1 and TULIP-2 trials. In brief, eligible patients were aged 18 to 70 years, fulfilled the American College of Rheumatology revised classification criteria for SLE (13), and had seropositive moderate to severe SLE despite standard of care treatment. Patients with active severe lupus nephritis or neuropsychiatric SLE were excluded. Patients were randomized to receive intravenous infusions of placebo or anifrolumab every 4 weeks for 48 weeks in addition to standard-of-care treatment (TULIP-1: placebo, anifrolumab 150 mg, or anifrolumab 300 mg [2:1:2]; TULIP-2: placebo or anifrolumab 300 mg [1 :1]). Primary endpoints were assessed at Week 52. Other treatments were stable throughout the trial except those resulting from protocol-determined intent-to-taper oral glucocorticoids. For patients receiving oral glucocorticoid ≥10 mg/day at baseline, an attempt to taper oral glucocorticoid to ≤7.5 mg/day was required between Weeks 8 and 40; tapering was also permitted for patients receiving oral glucocorticoid <10 mg/day at baseline. Stable oral glucocorticoid dosage was required between Weeks 40 and 52.

### Study Endpoints and Assessments

BICLA response was defined as all of the following: reduction of all baseline BILAG-2004 A and B domain scores to B/C/D and C/D, respectively, and no worsening in other BILAG-2004 organ systems as defined by ≥1 new BILAG-2004 A or ≥2 new BILAG-2004 B domain scores; no increase in SLEDAI-2K score (from baseline); no increase in PGA score (≥0.3 points from baseline); no discontinuation of investigational product; and no use of restricted medications beyond protocol-allowed thresholds. Pooled data were analyzed according to the TULIP-2 restricted medication analytical rules to classify responders/nonresponders.

Clinical outcome measures were compared between BICLA responders and nonresponders at Week 52, regardless of treatment group assignment, and results are presented in a hierarchy of clinical relevance, agreed by consensus between the authors. Outcome measures include the percentage of patients with flares (defined as ≥1 new BILAG-2004 A or ≥2 new BILAG-2004 B domain scores compared with the prior visit) through Week 52, annualized flare rates, percentage of patients achieving sustained oral glucocorticoid taper (defined as oral glucocorticoid dosage reduction to ≤7.5 mg/day prednisone or equivalent, achieved by Week 40 and sustained through Week 52, in patients receiving ≥10 mg/day at baseline), and change in daily oral glucocorticoid dosage from baseline to Week 52. Changes in PROs were assessed from baseline to Week 52, including responses in Functional Assessment of Chronic Illness Therapy-Fatigue [FACIT-F] (defined as a >3-point improvement), responses in Short Form 36 Health Survey version 2 [SF-36-v2] [acute] physical component summary [PCS] and mental component summary [MCS] (defined as an improvement of >3.4 in the PCS and >4.6 in the MCS), and changes from baseline in Patient's Global Assessment [PtGA]). Medical resource utilization (health care visits, emergency department [ED] use, and hospital visits) was also assessed. Other indices compared between BICLA responders and nonresponders included changes from baseline to Week 52 in SLEDAI-2K, PGA, joint counts (active, swollen, tender), and the Cutaneous Lupus Erythematosus Disease Area and Severity Index Activity (CLASI-A) responses (defined as ≥50% reduction in CLASI-A score among patients with CLASI-A score ≥10 at baseline). Serologies (anti-double-stranded DNA [anti-dsDNA] antibodies and complement C3 and C4) were evaluated; anti-dsDNA antibody levels were classified as 'positive' (>15 U/mL) or 'negative' (≤15 U/mL), and complement levels were classified as 'abnormal' (C3, <0.9 g/L; C4, <0.1 g/L) or 'normal' (C3, ≥0.9 g/L; C4, ≥0.1 g/L). Adverse events (AEs) were also assessed.

### Statistical Analyses

The similar designs of the TULIP-1 and TULIP-2 studies allowed for the results to be pooled. Sample sizes were selected for TULIP-1 and TULIP-2 to acquire adequate safety database sizes and to assess key secondary endpoints. In TULIP-1 and TULIP-2, 180 subjects/arm yielded >99% and 88% power, respectively, to reject the hypothesis (no difference in the primary endpoint) using a 2-sided alpha of 0.05. Responder versus nonresponder rates were calculated using a stratified Cochran-Mantel-Haenszel approach, which included stratification factors of SLEDAI-2K score at screening (<10 or ≥1 0), baseline oral glucocorticoid dosage (<10 mg/day or ≥10 mg/day), and type I IFNGS test status at screening (test-low or test-high). Study was also included in the model. For all responder analyses, patients were considered nonresponders if they used restricted medications beyond the protocol-allowed thresholds or discontinued investigational product before the assessment. Comparison of estimated change from baseline to Week 52 between BICLA responders and nonresponders was assessed using a mixed repeated measures model with fixed effects for baseline value, group, visit, study, and the stratification factors used at screening; a group-by-visit interaction term was used and visit was a repeated variable in the model. Missing data were imputed using the last observation carried forward for the first visit with missing data; subsequent visits with missing data were not imputed. For responder analyses, if any component of the variable could not be derived owing to missing data, the patient was classified as a nonresponders for that visit.

### Results

### Trial Populations

Data were pooled for 457 patients in TULIP-1 and 362 patients in TULIP-2 (N=819). Across both trials, 360 patients received anifrolumab 300 mg, 93 patients received anifrolumab 150 mg, and 366 patients received placebo. Regardless of treatment group assignment, there were 318 BICLA responders and 501 BICLA nonresponders at Week 52. Patient demographics and baseline clinical characteristics were generally balanced across BICLA responders and nonresponders (**Table 8** and **Table 9**). The majority of patients were female (92.5%, responders; 93.0%, nonresponders) and the mean (standard deviation [SD]) age was 41.5 (11.67) years for responders and 41.7 (12.13) years for nonresponders. Similar proportions of BICLA responders and nonresponders were white (67.0% vs 65.9%), Black/African American (14.2% vs 12.6%), or Asian (9.1% vs 11.0%).

Overall, improved outcomes were observed in BICLA responders versus nonresponders.

**Table 8. Patient demographics and baseline clinical characteristics**

| **Baseline characteristic** | | **BICLA responders at Week 52 (n=318)** | **BICLA nonresponders at Week 52 (n=501)** |
|---|---|---|---|
| Age, mean (SD), years | | 41.5 (11.67) | 41.7 (12.13) |
| Female, n (%) | | 294 (92.5) | 466 (93.0) |
| Time from SLE diagnosis to randomization, median (range), months | | 85.5 (0-555) | 84.0 (4-503) |
| IFNGS test-high at screening, n (%) | | 261 (82.1) | 415 (82.8) |
| BILAG-2004 score, mean (SD) | | 18.9 (5.20) | 19.2 (5.59) |
| | ≥1 A item, n (%) | 171 (53.8) | 222 (44.3) |
| | No A and ≥2 B items, n (%) | 126 (39.6) | 254 (50.7) |
| | No A and <2 B items, n (%) | 21 (6.6) | 25 (5.0) |
| SLEDAI-2K score, mean (SD) | | 10.8 (3.19) | 11.7 (4.01) |
| <10, n (%) | | 104 (32.7) | 127 (25.3) |
| ≥10, n (%) | | 214 (67.3) | 374 (74.7) |
| PGA score, mean (SD) | | 1.76 (0.425) | 1.81 (0.396) |
| Oral glucocorticoid use,^{a} n (%) | | 263 (82.7) | 410 (81.8) |
| | <10 mg/day | 98 (30.8) | 152 (30.3) |
| | ≥10 mg/day | 165 (51.9) | 258 (51.5) |
| Antimalarials | | 225 (70.8) | 361 (72.1) |
| Immunosuppressants | | 158 (49.7) | 230 (45.9) |
| CLASI activity score, mean (SD) | | 8.5 (7.56) | 7.8 (7.18) |
| | <10, n (%) | 215 (67.6) | 373 (74.5) |
| | ≥10, n (%) | 103 (32.4) | 128 (25.5) |
| | 0, n (%) | 13 (4.1) | 20 (4.0) |
| | >0, n (%) | 305 (95.9) | 481 (96.0) |
| SDI global score, mean (SD) | | 0.6 (1.08) | 0.5 (0.89) |
| Swollen joint count, mean (SD) | | 6.5 (5.27) | 7.4 (6.08) |
| Tender joint count, mean (SD) | | 9.8 (6.94) | 11.1 (7.85) |
| Active joint count,^{b} mean (SD) | | 6.1 (5.22) | 6.9 (5.97) |
| Anti-dsDNA positive,^{c} n (%) | | 142 (44.7) | 224 (44.7) |
| | Median (min, max),^{d} U/mL | 48.2 (15, 3790) | 57.0 (15, 4404) |
| | Mean (SD),^{c} U/mL | 142.5 (401.84) | 220.4 (526.38) |
| Abnormal C3,^{e} n (%) | | 123 (38.7) | 178 (35.5) |
| | Median (min, max),^{c} g/L | 0.729 (0.36, 0.90) | 0.715 (0.18, 0.90) |
| | Mean (SD),^{c} g/L | 0.711 (0.1279) | 0.685 (0.1603) |
| Abnormal C4,^{f} n (%) | | 72 (22.6) | 118 (23.6) |
| | Median (min, max),^{c} g/L | 0.080 (0.05, 0.10) | 0.072 (0.05, 0.10) |
| | Mean (SD),^{c} g/L | 0.075 (0.0168) | 0.071 (0.0145) |

| | | | |
|---|---|---|---|
| BICLA, BILAG-based Composite Lupus Assessment; BILAG-2004, British Isles Lupus Assessment Group-2004; CLASI, Cutaneous Lupus Erythematosus Disease Area and Severity Index; dsDNA, double-stranded DNA; IFNGS, interferon gene signature; max, maximum; min, minimum; PGA, Physician's Global Assessment; SD, standard deviation; SDI, Systemic Lupus International Collaborating Clinics/American College of Rheumatology Damage Index; SLE, systemic lupus erythematosus; SLEDAI-2K, SLE Disease Activity Index 2000. ^{a}Oral glucocorticoid includes prednisone or equivalent; ^{b}An active joint is defined as a joint with swelling and tenderness; ^{c}Anti-dsDNA antibody 'positive' defined as a result of >15 U/mL. ^{d}Only patients with anti-dsDNA antibodies and abnormal complement levels at baseline are included in the summary statistics for the respective variables. ^{e}Complement C3 'abnormal' levels defined as a result of <0.9 g/L. ^{f}Complement C4 'abnormal' levels defined as a result of <0.1 g/L. | | | |

**Table 9. Patient demographics and baseline SLE medications for BICLA responders and nonresponders**

| **Baseline demographics and SLE medication** | | **BICLA responders at Week 52 (n=318)** | **BICLA nonresponders at Week 52 (n=501)** |
|---|---|---|---|
| Race,^{a} n (%) | | | |
| | White | 213 (67.0) | 330 (65.9) |
| | Black/African American | 45 (14.2) | 63 (12.6) |
| | Asian | 29 (9.1) | 55 (11.0) |
| | Other | 23 (7.2) | 45 (9.0) |
| Hispanic or Latino ethnic group, n (%) | | 80 (25.2) | 115 (23.0) |

| Geographic region, n (%) | | | |
|---|---|---|---|
| | | | |
| | United States/Canada | 92 (28.9) | 226 (45.1) |
| | Europe | 126 (39.6) | 144 (28.7) |
| | Latin America | 66 (20.8) | 63 (12.6) |
| | Asia Pacific | 28 (8.8) | 49 (9.8) |
| | Rest of world | 6 (1.9) | 19 (3.8) |

| Baseline treatment for SLE, n (%) | | | |
|---|---|---|---|
| | Oral glucocorticoid^{b} | 263 (82.7) | 410 (81.8) |
| | Antimalarial | 225 (70.8) | 361 (72.1) |
| | Immunosuppressant^{c} | 158 (49.7) | 230 (45.9) |

| | | | |
|---|---|---|---|
| BICLA, British Isles Lupus Assessment Group-based Composite Lupus Assessment;; SD, standard deviation; SLE, systemic lupus erythematosus. ^{a}Race data were missing from 16 patients (8 each in the responder and nonresponder groups); "Oral glucocorticoid included prednisone or equivalent; ^{c}Immunosuppressant agents included azathioprine, methotrexate, mycophenolate mofetil, mycophenolic acid, and mizoribine. | | | |

### Flares

More BICLA responders than nonresponders were flare free over the 52-week treatment period (76.1% vs 52.2%), meaning that fewer BICLA responders than nonresponders experienced ≥1 flare over the 52-week period (23.9% vs 47.8%; difference -23.9%; 95% confidence interval [Cl] -30.4 to -17.5; nominal P<0.001) (FIG 28A). Fewer patients experienced 1, 2, or ≥3 flares and the annualized flare rate was lower for BICLA responders versus nonresponders (rate ratio [RR] 0.36, 95% CI 0.29 to 0.47; nominal P<0.001) (**Table 10**).

**Table 10. SLE flares in BICLA responders and nonresponders**

| | **BICLA responders at Week 52 (n=318)** | **BICLA nonresponders at Week 52 (n=501)** | |
|---|---|---|---|
| **Flares per patient through Week 52** | | | |
| Patients with 0 flares, n (%)^{a} | 242 (76.1) | 263 (52.2) | |
| Patients with ≥1 flare, n (%) | | 76 (23.9) | 238 (47.8) |
| | Comparison between groups, difference (95% CI), nominal *P* value^{a} | -23.9 (-30.4 to -17.5), < 0.001 | |
| | 1 | 52 (16.4) | 132 (26.3) |
| | 2 | 18 (5.7) | 69 (13.8) |
| | ≥3 | 6 (1.9) | 37 (7.4) |

| **Flare rate through Week 52^{b}** | | | |
|---|---|---|---|
| Total number of flares | | 107 | 401 |
| Total follow-up time, years | | 318.8 | 440.6 |
| Annualized rate (95% CI) | | 0.30 (0.24 to 0.38) | 0.83 (0.70 to 0.98) |
| | Comparison between groups, rate ratio (95% CI), nominal P value | 0.36 (0.29 to 0.47), < 0.0001 | |

| | | | |
|---|---|---|---|
| BICLA, BILAG-based Composite Lupus Assessment; BILAG-2004, British Isles Lupus Assessment Group-2004; Cl, confidence interval; CMH, Cochran-Mantel-Haenszel; PGA, Physician's Global Assessment; SD, standard deviation; SLE, systemic lupus erythematosus; SLEDAI-2K, SLE Disease Activity Index 2000. BILAG-2004 flares were defined by ≥1 new BILAG-2004 A or ≥2 new BILAG-2004 B domain scores compared with the prior visit. ^{a}Percentages, difference, Cl, and nominal P values are weighted and calculated using a stratified CMH approach. "Flare rates calculated using a negative binomial regression model which included covariates of group and stratification factors. The logarithm to the (base e) of the follow-up time is used as an offset variable in the model to adjust for different exposure times. | | | |

### Oral Glucocorticoid Use and Steroid Sparing

Similar percentages of BICLA responders and nonresponders were receiving oral glucocorticoid at any dosage, and at ≥10 mg/day, at baseline. BICLA responders versus nonresponders had greater reductions in daily oral glucocorticoid dosage from baseline to Week 52 (least squares [LS] mean difference -4.29 mg/day, 95% CI -5.37 to -3.20, nominal P<0.001) (**FIG 28B**). The secondary endpoint of sustained oral glucocorticoid dosage reduction to ≤7.5 mg/day among patients who were receiving oral glucocorticoid ≥10 mg/day at baseline was achieved by more BICLA responders than nonresponders (79.2% vs 19.1%; difference 60.1%, 95% CI 52.1% to 68.1%, nominal P<0.001) (**FIG 28C**). Mean (SD) cumulative oral glucocorticoid dose through Week 52 was 31.3% lower in BICLA responders versus nonresponders (2159.20 [1661.39] mg vs 3140.81 [3081.19] mg) (**FIG 28D**).

### PROs

FACIT-F, SF-36 MCS, and SF-36 PCS scores were similar for BICLA responders and nonresponders at baseline (**Table 11**). Improvement in FACIT-F was reported in more BICLA responders than nonresponders (55.6% vs 15.7%; difference 40.0%, 95% CI 33.6% to 46.3%, nominal P<0.001) (**FIG 29A**). Similarly, more BICLA responders than nonresponders had improvement above the predefined threshold in SF-36 PCS (57.9% vs 12.8%; difference 45.1%, 95% CI 38.9% to 51.3%, nominal P<0.001) and SF-36 MCS (42.6% vs 12.3%; difference 30.3%, 95% Ct 24.1% to 36.5%, nominal P<0.001) (**FIG 29A-C**).

**Table 11. PRO scores at baseline in BICLA responders and nonresponders**

| **PRO** | | **BICLA responders (n=318)** | **BICLA nonresponders (n=501)** |
|---|---|---|---|
| FACIT-F | | | |
| | n | 311 | 467 |
| | Mean (SD) | 26.3 (12.63) | 25.3 (11.99) |
| SF-36 PCS | | | |
| | n | 312 | 469 |
| | Mean (SD) | 38.3 (9.20) | 37.0 (9.25) |
| SF-36 MCS | | | |
| | n | 312 | 469 |
| | Mean (SD) | 43.8 (11.35) | 44.0 (11.24) |
| PtGA | | | |
| | n | 311 | 467 |
| | Mean (SD) | 54.7 (23.36) | 55.8 (21.44) |

| | | | |
|---|---|---|---|
| BICLA, British Isles Lupus Assessment Group-based Composite Lupus Assessment; FACIT-F Functional Assessment of Chronic Illness Therapy-Fatigue; MCS, mental component score; PCS, physical component score; PRO, patient-reported outcome; PtGA, Patient's Global Assessment; SD, standard deviation; SF-36-v2, Short Form 36 Health Survey version 2 (acute recall). | | | |

### PtGA

PtGA scores were similar for BICLA responders and nonresponders at baseline. Greater improvements in PtGA scores from baseline to Week 52 were reported for BICLA responders than nonresponders (LS mean difference -11.1, 95% CI -14.9 to -7.3, nominal P < 0.001) (**FIG 29D**).

### Medical Resource Utilization

During the 52-week trials, fewer BICLA responders than nonresponders had health care visits (62.5% vs 70.7%; difference -8.3%, 95% CI -14.9% to -1.6%, nominal P=0.015) (**Table 12**). Fewer BICLA responders required emergency department (ED) visits compared with nonresponders (11.9% vs 21.8%; difference -9.9%, 95% CI -15.2% to -4.5%, nominal P=0.001), and fewer ED visits were related to increased SLE activity (2.6% vs 24.0%; difference -21.4%, 95% CI -35.3% to -7.5%, nominal P=0.003). Similarly, fewer BICLA responders than nonresponders had hospital visits (4.5% vs 14.4%; difference - 10.0%, 95% Cl -14.3% to -5.7%, nominal P<0.001), and no hospital visits were related to increased SLE activity among BICLA responders, compared with 38.5% among BICLA nonresponders (difference -38.5%, 95% CI -58.8% to -18.2%, nominal P<0.001).

**Table 12. Medical resource utilization for BICLA responders and nonresponders**

| **Medical resource utilization** | | | **BICLA responders at Week 52 (n=318)** | **BICLA nonresponders at Week 52 (n=501)** |
|---|---|---|---|---|
| **Health care visits (specialist and primary care)** | | | | |
| | Patients with ≥1 health care visit,^{a} n (%) | | 198 (62.5) | 348 (70.7) |
| | | Comparison between groups, difference (95% CI), nominal *P* value | -8.3 (-14.9 to -1.6), 0.015 | |

| **Emergency department visits** | | | | |
|---|---|---|---|---|
| | Patients with ≥1 ED visit,^{a} n (%) | | 38 (11.9) | 107 (21.8) |
| | | Comparison between groups, difference (95% CI), nominal *P* value | -9.9 (-15.2 to -4.5), < 0.001 | |
| | Visit related to increase in SLE activity,^{a} n (%) | | 1 (2.6) | 25 (24.0) |
| | | Comparison between groups, difference (95% CI), nominal *P* value | -21.4 (-35.3 to -7.5), 0.003 | |
| | Number of ED visits per patient,^{b} mean (SD) | | 1.4 (0.86) | 1.7 (1.48) |

| **Hospitalizations** | | | | |
|---|---|---|---|---|
| | Patients with ≥1 hospital visits,^{a} n (%) | | 14 (4.5) | 71 (14.4) |
| | | Comparison between groups, difference (95% CI), nominal *P* value | -10.0 (-14.3 to -5.7), < 0.001 | |
| | Visit related to increase in SLE activity,^{a} n (%) | | 0 | 25 (38.5) |
| | | Comparison between groups, difference (95% CI), nominal *P* value | -38.5 (-58.8 to -18.2), < 0.001 | |
| | Number of hospital visits per patient,^{b} mean (SD) | | 1.6(2.13) | 1.4 (0.72) |
| | Length of hospital stay,^{b} mean (SD), days | | 5.7 (2.64) | 7.4 (8.02) |
| | Total days in ICU,^{b} n | | 0 | 5 |
| | | Number of days in ICU, mean (SD) | - | 1.8 (0.45) |

| | | | | |
|---|---|---|---|---|
| BICLA, British Isles Lupus Assessment Group-based Composite Lupus Assessment; Cl, confidence interval; ED, emergency department; ICU, intensive care unit; SD, standard deviation; SLE, systemic lupus erythematosus. ^{a}Percentages, differences, CIs, and nominal P values were calculated using a stratified CMH approach. "Data on hospital visits and emergency department visits were missing from 8 patients in the BICLA nonresponders group. | | | | |

### SLEDAI-2K and PGA

Mean (SD) SLEDAI-2K and PGA scores were similar between responders and nonresponders at baseline (Table 1). From baseline to Week 52, greater improvements were observed for BICLA responders versus nonresponders in total SLEDAI-2K (LS mean difference -3.5, 95% CI -4.1 to -3.0, nominal P<0.001) (**FIG 30A**) and PGA scores (LS mean difference -0.59, 95% CI -0.67 to -0.51, nominal P<0.001) (**FIG 30B**).

### CLASI Activity

Overall, 32.4% of BICLA responders and 25.5% of nonresponders had a baseline CLASI-A ≥10 (Table 1). Among these patients, more BICLA responders achieved a ≥50% reduction in CLASI-A at Week 52 versus nonresponders (92.0% vs 23.2%; difference 68.8%, 95% CI 59.2% to 78.3%, nominal P<0.001) (FIG 31A).

### Joint Counts

Mean (SD) active joint counts (defined as joints with swelling and tenderness) were 6.1 (5.22) and 6.9 (5.97) in BICLA responders and nonresponders, respectively, at baseline. Mean (SD) swollen joint counts were 6.5 (5.27) and 7.4 (6.08), respectively, and tender joint counts were 9.8 (6.94) and 11.1 (7.85), respectively. From baseline to Week 52, joint counts improved more for BICLA responders versus nonresponders for active (LS mean difference -1.9, 95% CI -2.4 to -1.4, nominal P < 0.001), tender (LS mean difference - 3.6, 95% Cl -4.4 to -2.8, nominal P < 0.001), and swollen (LS mean difference -2.1, 95% CI -2.7 to -1.6, nominal P < 0.001) joints (**FIG 31B**).

### Serologies

Equal percentages of patients were anti-dsDNA antibody positive at baseline between BICLA responders and nonresponders. Improvement from positive to negative anti-dsDNA antibody status \\was observed in similar percentages of BICLA responders and nonresponders (5.0% vs 4.4%) (**Table 13**).

**Table 13. Serology changes from baseline to Week 52 for BICLA responders and nonresponders**

| **Change from baseline to Week 52** | | **BICLA responders at Week 52 (n=318)** | **BICLA nonresponders** at **Week 52 (n=501)** | |
|---|---|---|---|---|
| **Anti-dsDNA^{a}** | | | | |
| | n | 132 | 147 | |
| | Shift positive to negative, n (%) | | 16 (5.0) | 22 (4.4) |
| | Shift negative to positive, n (%) | | 8 (2.5) | 16 (3.2) |
| | Remained negative, n (%) | | 114 (35.8) | 142 (28.3) |
| | Remained positive, n (%) | | 116 (36.5) | 125 (25.0) |

| **Complement C3^{b}** | | | | |
|---|---|---|---|---|
| | n | | 121 | 127 |
| | Shift abnormal to normal, n (%) | | 33 (10.4) | 35 (7.0) |
| | Shift normal to abnormal, n (%) | | 14 (4.4) | 30 (6.0) |
| | Remained normal, n (%) | | 167 (52.5) | 193 (38.5) |
| | Remained abnormal, n (%) | | 88 (27.7) | 92 (18.4) |
| | LS mean change, % (95% CI) | | 15.7 (9.489 to 21.926) | 12.9 (6.852 to 18.929) |
| | | LS mean difference, % (95% CI), nominal *P* value | 2.8 (-4.19 to 9.82), 0.429 | |

| **Complement C4^{c}** | | | | |
|---|---|---|---|---|
| | n | | 70 | 79 |
| | Shift abnormal to normal, n (%) | | 24 (7.5) | 24 (4.8) |
| | Shift normal to abnormal, n (%) | | 9 (2.8) | 20 (4.0) |
| | Remained normal, n (%) | | 222 (69.8) | 251 (50.1) |
| | Remained abnormal, n (%) | | 46 (14.5) | 55 (11.0) |
| | LS mean change, % (95% CI), g/L | | 19.5 (5.664 to 33.336) | 29.1 (16.066 to 42.198) |
| | | LS mean difference, % (95% CI), nominal *P* value | -9.6 (-25.17 to 5.91), 0.223 | |

| | | | | |
|---|---|---|---|---|
| BICLA, British Isles Lupus Assessment Group-based Composite Lupus Assessment; Cl, confidence interval; dsDNA, double-stranded DNA; max, maximum; min, minimum; SD, standard deviation. ^{a}Anti-dsDNA antibody 'positive' or 'negative' defined as a result of >15U/mL or ≤15 U/mL, respectively. ^{b}Complement C3 'abnormal' or 'normal' levels defined as a result of <0.9 g/L or C3, <0.9 g/L, respectively. ^{c}Complement C4 'abnormal' or 'normal' levels defined as a result of <0.1 g/L or C3, <0.1 g/L, respectively. | | | | |

Only patients with baseline positive anti-dsDNA or abnormal complement C3 or C4 are included in the analysis. Percentage change, difference, Cl, and nominal *P* values calculated using a repeated measures model with fixed effects for baseline value, group, visit, study, and stratification factors. A visit-by-group interaction term was used to account for different relationships across groups and visit was a repeated variable in the model. Percentages do not equal 100% owing to missing data.

Similar proportions of BICLA responders and nonresponders had abnormal C3 and C4 levels at baseline. Percentage changes from baseline to Week 52 in complement levels did not differ between BICLA responders versus nonresponders for C3 (LS mean difference 2.82, 95% CI -4.185 to 9.819, nominal P = 0.429) or C4 (LS mean difference -9.63, 95% Cl -25.174 to 5.910, nominal P = 0.223) (**Table 13**). More BICLA responders than nonresponders had improvements from abnormal to normal C3 (10.4% vs 7.0%) and C4 (7.5% vs 4.8%).

### Safety

AE frequencies were similar between BICLA responders and nonresponders (83.6% and 85.2%) (**Table 14**). Mild and moderate AEs were reported by similar percentages of BICLA responders and nonresponders, whereas fewer BICLA responders than nonresponders experienced severe AEs (3.8% vs 9.4%). There were no AEs leading to discontinuation (DAE) in BICLA responders compared with 8.2% DAEs in nonresponders. Fewer BICLA responders than nonresponders experienced serious AEs (5.0% vs 19.0%). Fewer BICLA responders than nonresponders had non-opportunistic serious infections (2.2% vs 6.8%). The percentage of patients with herpes zoster was similar in BICLA responders and nonresponders (4.7% vs 3.6%), as was the percentage of patients with influenza (1.9% vs 2.0%) or malignancy (0.6% vs 1.0%).

**Table 14. AEs during treatment in BICLA responders and nonresponders**

| **AEs, n (%)** | | **BICLA responders at Week 52 (n=318)** | **BICLA nonresponders at Week 52 (n=501)** |
|---|---|---|---|
| | Patients with any AE | 266 (83.6) | 427 (85.2) |
| Any AE with outcome of death | | 0 | 2 (0.4) |
| Any SAE (including events with outcome of death) | | 16 (5.0) | 95 (19.0) |
| Lupus-related SAEs | | 1 (0.3) | 15 (0.3) |
| | Lupus nephritis | 0 | 1 (0.2) |
| | SLE | 1 (0.3) | 14 (2.8) |
| Any DAE | | 0 | 41 (8.2) |
| Any AE related to investigational product | | 101 (31.8) | 160 (31.9) |
| Any AE by maximum reported intensity | | | |
| | Mild | 126 (39.6) | 183 (36.5) |
| | Moderate | 128 (40.3) | 197 (39.3) |
| | Severe | 12 (3.8) | 47 ( 9.4) |
| Any AESI | | 27 (8.5) | 67 (13.4) |
| | Non-opportunistic serious infection | 7 (2.2) | 34 (6.8) |
| | Opportunistic infections | 0 | 1 (0.2) |
| | Anaphylaxis | 0 | 1 (0.2) |
| | Malignancy | 2 (0.6) | 5 (1.0) |
| | Herpes zoster | 15 (4.7) | 18 (3.6) |
| | Tuberculosis (including latent TB) | 2 (0.6) | 1 (0.2) |
| | Influenza | 6 (1.9) | 10 (2.0) |
| | Non-SLE vasculitis | 0 | 0 |
| | Major adverse cardiovascular event | 1 (0.3) | 1 (0.2) |

| | | | |
|---|---|---|---|
| AE, adverse event; AESI, adverse event of special interest; DAE, adverse event leading to discontinuation; SAE, serious adverse event; SLE, systemic lupus erythematosus; TB, tuberculosis. | | | |

### Conclusions

BICLA is a dichotomous SLE outcome measure that classifies a patient as a responder or nonresponder based on changes in organ domain activity. As BICLA is primarily used in the clinical trial setting, the aim of this study was to assess the meaningfulness of BICLA response in terms of outcomes that are relevant to patients and physicians. In this post hoc analysis of pooled data acquired from 819 patients enrolled in the TULIP-1 and TULIP-2 trials, BICLA response was significantly associated with improved clinical outcomes across a range of SLE assessments, key PROs, and medical resource utilization measures.

Flares, with or without an increase in glucocorticoid dose, pose significant risks to patients with SLE. In the long term, both disease flares and oral glucocorticoid use have been linked to organ damage, which itself increases mortality risk. Flares also associate with reduced health-related quality of life, and flare severity and oral glucocorticoid use correlates with health care costs. A key SLE treatment goal therefore is to prevent flares while minimizing oral glucocorticoid exposure, which in turn is expected to reduce medical resource utilization. We observed that BICLA responders had fewer disease flares together with a lower daily oral glucocorticoid dose. A greater percentage of BICLA responders achieved sustained oral glucocorticoid reduction to target dose. They also had fewer hospitalizations and ED visits than did nonresponders, including those related to increased SLE activity. Greater improvements in global and tissue-specific disease activity were also observed in responders versus nonresponders, as measured by PGA, SLEDAI-2K, CLASI-A, and joint counts. As improved patient outcomes in disease activity and oral glucocorticoid exposure have been shown to associate with reduced health care costs, BICLA responders may incur lower health care costs than nonresponders.

We also assessed adverse events in BICLA responders and nonresponders. Consistent with the lower flare rates, reduced medical resource utilization, and fewer SLE-related ED visits and hospitalizations associated with BICLA response, there were fewer SAEs in BICLA responders versus nonresponders. While discontinuation of investigational product for any reason led to a patient being classified as a BICLA nonresponder by definition, of note, BICLA nonresponders had a greater propensity to discontinue due to an AE than BICLA responders.

PROs have been incorporated into nearly all SLE clinical trials. However, analyses have often yielded discordance between clinical outcomes and PROs, as patient perceptions of disease activity and illness are heavily impacted by fatigue and quality of life and are not captured by the results of formal measures of disease activity. In the TULIP trials, BICLA responders had improvements in validated PROs, including the physical and mental components of the SF-36 health survey and the FACIT assessment of fatigue. Fatigue, a common symptom in patients with SLE, interferes with daily life, and more than one half of patients with BICLA responses experienced improvement in fatigue in the TULIP trials. PtGA and PGA scores showed concordance in improvement, and greater degrees of improvement among BICLA responders than nonresponders. Our results demonstrate that BICLA response translates to general improvements in the physical and mental wellbeing of patients with SLE.

Investigation of correlations of SRI(4) response to clinical outcomes in pooled data from 2 phase 2b trials (sifalimumab and anifrolumab), as well as 2 phase 3 trials of belimumab, also demonstrated improved clinical outcomes in SRI(4) responders compared with nonresponders. Whereas changes in serologic outcomes were not significantly different between BICLA responders and nonresponders in the TULIP trials, SRI(4) response was associated with significant improvements in anti-dsDNA antibody and complement C3 levels (but not C4 levels) in the belimumab phase 3 trials. This discordance may be a reflection of the different mechanisms of action of the 2 evaluated drugs, and/or because the BILAG-2004, which measures improvement in BICLA, does not include serology in its scoring system.

The data confirm the value of BICLA as a clinical trial endpoint and that a BICLA response correlates with improvements in a range of other outcomes that resonate with the priorities of both clinicians and patients in everyday practice.

**Table 8. PRO scores at baseline in BICLA responders and nonresponders**

| **PRO** | | **BICLA responders (n=318)** | **BICLA nonresponders (n=501)** |
|---|---|---|---|
| FACIT-F | | | |
| | n | 311 | 467 |
| | Mean (SD) | 26.3 (12.63) | 25.3 (11.99) |
| SF-36 PCS | | | |
| | n | 312 | 469 |
| | Mean (SD) | 38.3 (9.20) | 37.0 (9.25) |
| SF-36 MCS | | | |
| | n | 312 | 469 |
| | Mean (SD) | 43.8 (11.35) | 44.0 (11.24) |
| PtGA | | | |
| | n | 311 | 467 |
| | Mean (SD) | 54.7 (23.36) | 55.8 (21.44) |

| | | | |
|---|---|---|---|
| BICLA, British Isles Lupus Assessment Group-based Composite Lupus Assessment; FACIT-F Functional Assessment of Chronic Illness Therapy-Fatigue; MCS, mental component score; PCS, physical component score; PRO, patient-reported outcome; PtGA, Patient's Global Assessment; SD, standard deviation; SF-36-v2, Short Form 36 Health Survey version 2 (acute recall). | | | |

### REFERENCES

(1) Bruce, I. N.; O'Keeffe, A. G.; Farewell, V.; Hanly, J. G.; Manzi, S.; Su, L.; Gladman, D. D.; Bae, S.-C.; Sanchez-Guerrero, J.; Romero-Diaz, J.; Gordon, C.; Wallace, D. J.; Clarke, A. E.; Bernatsky, S.; Ginzler, E. M.; Isenberg, D. A.; Rahman, A.; Merrill, J. T.; Alarcón, G. S.; Fessler, B. J.; Fortin, P. R.; Petri, M.; Steinsson, K.; Dooley, M. A.; Khamashta, M. A.; Ramsey-Goldman, R.; Zoma, A. A.; Sturfelt, G. K.; Nived, O.; Aranow, C.; Mackay, M.; Ramos-Casals, M.; van Vollenhoven, R. F.; Kalunian, K. C.; Ruiz-lrastorza, G.; Lim, S.; Kamen, D. L.; Peschken, C. A.; Inane, M.; Urowitz, M. B. Factors Associated with Damage Accrual in Patients with Systemic Lupus Erythematosus: Results from the Systemic Lupus International Collaborating Clinics (SLICC) Inception Cohort. Ann. Rheum. Dis. 2015, 74 (9), 1706-1713. https://doi.org/10.1136/annrheumdis-2013-205171.
(2) Petri, M. Long-Term Outcomes in Lupus. Am. J. Manag. Care 2001, 7 (16 Suppl), S480-485.
(3) Patel, D. D.; Antoni, C.; Freedman, S. J.; Levesque, M. C.; Sundy, J. S. Phase 2 to Phase 3 Clinical Trial Transitions: Reasons for Success and Failure in Immunologic Diseases. J. Allergy Clin. Immunol. 2017, 140 (3), 685-687. https://doi.org/10.1016/j.jaci.2017.04.029.
(4) Dowden, H.; Munro, J. Trends in Clinical Success Rates and Therapeutic Focus. Nat. Rev. Drug Discov. 2019, 18 (7), 495-496. https://doi.org/10.1038/d41573-019-00074-z.
(5) Eisenberg, R. WHY CAN'T WE FIND A NEW TREATMENT FOR SLE? J. Autoimmun. 2009, 32 (3-4), 223-230. https://doi.org/10.1016/j.jaut.2009.02.006.
(6) Hahn, B. H. Targeted Therapies in Systemic Lupus Erythematosus: Successes, Failures and Future. Ann. Rheum. Dis. 2011, 70 Suppl 1, i64-i66. https://doi.org/10.1136/ard.2010.142208.
(7) Isenberg, D. A.; Petri, M.; Kalunian, K.; Tanaka, Y.; Urowitz, M. B.; Hoffman, R. W.; Morgan-Cox, M.; likuni, N.; Silk, M.; Wallace, D. J. Efficacy and Safety of Subcutaneous Tabalumab in Patients with Systemic Lupus Erythematosus: Results from ILLUMlNATE-1, a 52-Week, Phase III, Multicentre, Randomised, Double-Blind, Placebo-Controlled Study. Ann. Rheum. Dis. 2016, 75 (2), 323-331. https://doi.org/10.1136/annrheumdis-2015-207653.
(8) Isenberg, D.; Merrill, J.; Hoffman, R.; Linnik, M.; Morgan-Cox, M.; Veenhuizen, M.; likuni, N.; Dickson, C.; Silk, M.; Wallace, D.; Dörner, T. OP0184 Efficacy and Safety of Tabalumab in Patients with Systemic Lupus Erythematosus (SLE): Results from 2 Phase 3, 52-Week, Multicenter, Randomized, Placebo-Controlled Trials. Ann. Rheum. Dis. 2015, 74 (Suppl 2), 141-141. https://doi.org/10.1136/annrheumdis-2015-eular.1195.
(9) Furie, R. A.; Leon, G.; Thomas, M.; Petri, M. A.; Chu, A. D.; Hislop, C.; Martin, R. S.; Scheinberg, M. A.; PEARL-SC Study. A Phase 2, Randomised, Placebo-Controlled Clinical Trial of Blisibimod, an Inhibitor of B Cell Activating Factor, in Patients with Moderate-to-Severe Systemic Lupus Erythematosus, the PEARL-SC Study. Ann. Rheum. Dis. 2015, 74 (9), 1667-1675. https://doi.org/10.1 136/annrheumdis-2013-205144.
(10) Merrill, J. T.; Shanahan, W. R.; Scheinberg, M.; Kalunian, K. C.; Wofsy, D.; Martin, R. S. Phase III Trial Results with Blisibimod, a Selective Inhibitor of B-Cell Activating Factor, in Subjects with Systemic Lupus Erythematosus (SLE): Results from a Randomised, Double-Blind, Placebo-Controlled Trial. Ann. Rheum. Dis. 2018, 77 (6), 883-889. https://doi.org/10.1136/annrheumdis-2018-213032.
(11) Isenberg, D.; Gordon, C.; Lieu, D.; Copt, S.; Rossi, C. P.; Wofsy, D. Efficacy and Safety of Atacicept for Prevention of Flares in Patients with Moderate-to-Severe Systemic Lupus Erythematosus (SLE): 52-Week Data (APRIL-SLE Randomised Trial). Ann. Rheum. Dis. 2015, 74 (11), 2006-2015. https://doi.org/10.1136/annrheumdis-2013-205067.
(12) Alarcón-Segovia, D.; Tumlin, J. A.; Furie, R. A.; McKay, J. D.; Cardiel, M. H.; Strand, V.; Bagin, R. G.; Linnik, M. D.; Hepburn, B.; LJP 394 Investigator Consortium. LJP 394 for the Prevention of Renal Flare in Patients with Systemic Lupus Erythematosus: Results from a Randomized, Double-Blind, Placebo-Controlled Study. Arthritis Rheum. 2003, 48 (2), 442-454. https://doi.org/10.1002/art.10763.
(13) Mahieu, M. A.; Strand, V.; Simon, L. S.; Lipsky, P. E.; Ramsey-Goldman, R. A Critical Review of Clinical Trials in Systemic Lupus Erythematosus. Lupus 2016, 25 (10), 1122-1140. https://doi.org/10.1177/0961203316652492.
(14) Rovin, B. H.; Furie, R.; Latinis, K.; Looney, R. J.; Fervenza, F. C.; Sanchez-Guerrero, J.; Maciuca, R.; Zhang, D.; Garg, J. P.; Brunetta, P.; Appel, G.; LUNAR Investigator Group. Efficacy and Safety of Rituximab in Patients with Active Proliferative Lupus Nephritis: The Lupus Nephritis Assessment with Rituximab Study. Arthritis Rheum. 2012, 64 (4), 1215-1226. https://doi.org/10.1002/art.34359.
(15) Merrill, J. T.; Neuwelt, C. M.; Wallace, D. J.; Shanahan, J. C.; Latinis, K. M.; Oates, J. C.; Utset, T. O.; Gordon, C.; Isenberg, D. A.; Hsieh, H.-J.; Zhang, D.; Brunetta, P. G. Efficacy and Safety of Rituximab in Moderately-to-Severely Active Systemic Lupus Erythematosus: The Randomized, Double-Blind, Phase II/III Systemic Lupus Erythematosus Evaluation of Rituximab Trial. Arthritis Rheum. 2010, 62 (1), 222-233. https://doi.org/10.1002/art.27233.
(16) Daikh, D. I.; Wofsy, D. Cutting Edge: Reversal of Murine Lupus Nephritis with CTLA4Ig and Cyclophosphamide. J. Immunol. 2001, 166 (5), 2913-2916. https://doi.org/10.4049/jimmunol.166.5.2913.
(17) Merrill, J. T.; Burgos-Vargas, R.; Westhovens, R.; Chalmers, A.; D'Cruz, D.; Wallace, D. J.; Bae, S. C.; Sigal, L.; Becker, J.-C.; Kelly, S.; Raghupathi, K.; Li, T.; Peng, Y.; Kinaszczuk, M.; Nash, P. The Efficacy and Safety of Abatacept in Patients with Non-Life-Threatening Manifestations of Systemic Lupus Erythematosus: Results of a Twelve-Month, Multicenter, Exploratory, Phase IIb, Randomized, Double-Blind, Placebo-Controlled Trial. Arthritis Rheum. 2010, 62 (10), 3077-3087. https://doi.org/10.1002/art.27601.
(18) Strand, V.; Petri, M.; Kalunian, K.; Gordon, C.; Wallace, D. J.; Hobbs, K.; Kelley, L.; Kilgallen, B.; Wegener, W. A.; Goldenberg, D. M. Epratuzumab for Patients with Moderate to Severe Flaring SLE: Health-Related Quality of Life Outcomes and Corticosteroid Use in the Randomized Controlled ALLEVIATE Trials and Extension Study SL0006. Rheumatol. Oxf. Engl. 2014, 53 (3), 502-511. https://doi.org/10.1093/rheumatology/ket378.
(19) Wallace, D. J.; Kalunian, K.; Petri, M. A.; Strand, V.; Houssiau, F. A.; Pike, M.; Kilgallen, B.; Bongardt, S.; Barry, A.; Kelley, L.; Gordon, C. Efficacy and Safety of Epratuzumab in Patients with Moderate/Severe Active Systemic Lupus Erythematosus: Results from EMBLEM, a Phase IIb, Randomised, Double-Blind, Placebo-Controlled, Multicentre Study. Ann. Rheum. Dis. 2014, 73 (1), 183-190. https://doi.org/10.1136/annrheumdis-2012-202760.
(20) Wallace, D. J.; Strand, V.; Merrill, J. T.; Popa, S.; Spindler, A. J.; Eimon, A.; Petri, M.; Smolen, J. S.; Wajdula, J.; Christensen, J.; Li, C.; Diehl, A.; Vincent, M. S.; Beebe, J.; Healey, P.; Sridharan, S. Efficacy and Safety of an Interleukin 6 Monoclonal Antibody for the Treatment of Systemic Lupus Erythematosus: A Phase II Dose-Ranging Randomised Controlled Trial. Ann. Rheum. Dis. 2017, 76 (3), 534-542. https://doi.org/10.1136/annrheumdis-2016-209668.
(21) Psarras, A.; Emery, P.; Vital, E. M. Type I Interferon-Mediated Autoimmune Diseases: Pathogenesis, Diagnosis and Targeted Therapy. Rheumatol. Oxf. Engl. 2017, 56 (10), 1662-1675. https://doi.org/10.1093/rheumatology/kew431.
(22) Kalunian, K. C.; Merrill, J. T.; Maciuca, R.; McBride, J. M.; Townsend, M. J.; Wei, X.; Davis, J. C.; Kennedy, W. P. A Phase II Study of the Efficacy and Safety of Rontalizumab (RhuMAb Interferon-a) in Patients with Systemic Lupus Erythematosus (ROSE). Ann. Rheum. Dis. 2016, 75 (1), 196-202. https://doi.org/10.1136/annrheumdis-2014-206090.
(23) Furie, R.; Khamashta, M.; Merrill, J. T.; Werth, V. P.; Kalunian, K.; Brohawn, P.; Illei, G. G.; Drappa, J.; Wang, L.; Yoo, S.; Investigators, for the C. S. Anifrolumab, an Anti-Interferon-α Receptor Monoclonal Antibody, in Moderate-to-Severe Systemic Lupus Erythematosus. Arthritis Rheumatol. Hoboken Nj 2017, 69 (2), 376. https://doi.org/10.1002/art.39962.
(24) Update on TULIP 1 Phase III trial for anifrolumab in systemic lupus erythematosus https://www.astrazeneca.com/media-centre/press-releases/2018/update-on-tulip-1-phase-iii-trial-for-anifrolumab-in-systemic-lupus-erythematosus-31082018.html (accessed Nov 10, 2020).
(25) Felten, R.; Scher, F.; Sagez, F.; Chasset, F.; Arnaud, L. Spotlight on Anifrolumab and Its Potential for the Treatment of Moderate-to-Severe Systemic Lupus Erythematosus: Evidence to Date. Drug Des. Devel. Ther. 2019, 13, 1535-1543. https://doi.org/10.2147/DDDT.S170969.
(26) Isenberg, D. A.; Merrill, J. T. Why, Why, Why de-Lupus (Does so Badly in Clinical Trials). Expert Rev. Clin. Immunol. 2016, 12 (2), 95-98. https://doi.org/10.1586/1744666X.2016.1112270.
(27) Wallace, D. J. The Evolution of Drug Discovery in Systemic Lupus Erythematosus. Nat. Rev. Rheumatol. 2015, 11 (10), 616-620. https://doi.org/10.1038/nrrheum.2015.86.
(28) Felten, R.; Sagez, F.; Gavand, P.-E.; Martin, T.; Korganow, A.-S.; Sordet, C.; Javier, R.-M.; Soulas-Sprauel, P.; Rivière, M.; Scher, F.; Poindron, V.; Guffroy, A.; Arnaud, L. 10 Most Important Contemporary Challenges in the Management of SLE. Lupus Sci. Med. 2019, 6 (1), e000303. https://doi.org/10.1136/lupus-2018-000303.
(29) Hui-Yuen, J. S.; Reddy, A.; Taylor, J.; Li, X.; Eichenfield, A. H.; Bermudez, L. M.; Starr, A. J.; Imundo, L. F.; Buyon, J.; Furie, R. A.; Kamen, D. L.; Manzi, S.; Petri, M.; Ramsey-Goldman, R.; van Vollenhoven, R. F.; Wallace, D. J.; Askanase, A. Safety and Efficacy of Belimumab in Systemic Lupus Erythematosus Academic Clinical Practices. J. Rheumatol. 2015, 42 (12), 2288-2295. https://doi.org/10.3899/jrheum.150470.
(30) Furie, R. A.; Petri, M. A.; Wallace, D. J.; Ginzler, E. M.; Merrill, J. T.; Stohl, W.; Chatham, W. W.; Strand, V.; Weinstein, A.; Chevrier, M. R.; Zhong, Z. J.; Freimuth, W. W. Novel Evidence-Based Systemic Lupus Erythematosus Responder Index. Arthritis Rheum. 2009, 61 (9), 1143-1151. https://doi.org/10.1002/art.24698.
(31) Albrecht, J.; Taylor, L.; Berlin, J. A.; Dulay, S.; Ang, G.; Fakharzadeh, S.; Kantor, J.; Kim, E.; Militello, G.; McGinnis, K.; Richardson, S.; Treat, J.; Vittorio, C.; Van Voorhees, A.; Werth, V. P. The CLASI (Cutaneous Lupus Erythematosus Disease Area and Severity Index): An Outcome Instrument for Cutaneous Lupus Erythematosus. J. Invest. Dermatol. 2005, 125 (5), 889-894. https://doi.org/10.1111/j.0022-202X.2005.23889.x.
(32) Gordon, C.; Sutcliffe, N.; Skan, J.; Stoll, T.; Isenberg, D. A. Definition and Treatment of Lupus Flares Measured by the BILAG Index. Rheumatol. Oxf. Engl. 2003, 42 (11), 1372-1379. https://doi.org/10.1093/rheumatology/keg382.
(33) Thanou, A.; Chakravarty, E.; James, J. A.; Merrill, J. T. Which Outcome Measures in SLE Clinical Trials Best Reflect Medical Judgment? Lupus Sci. Med. 2014, 1 (1), e000005. https://doi.org/10.1136/lupus-2013-000005.
(34) Mikdashi, J.; Nived, O. Measuring Disease Activity in Adults with Systemic Lupus Erythematosus: The Challenges of Administrative Burden and Responsiveness to Patient Concerns in Clinical Research. Arthritis Res. Ther. 2015, 17 (1), 183. https://doi.org/10.1186/s13075-015-0702-6.
(35) Rice, J. B.; White, A. G.; Scarpati, L. M.; Wan, G.; Nelson, W. W. Long-Term Systemic Corticosteroid Exposure: A Systematic Literature Review. Clin. Ther. 2017, 39 (11), 2216-2229. https://doi.org/10.1016/j.clinthera.2017.09.011.
(36) Hochberg, M. C. Updating the American College of Rheumatology Revised Criteria for the Classification of Systemic Lupus Erythematosus. Arthritis Rheum. 1997, 40 (9), 1725. https://doi.org/10.1002/art.1780400928.
(37) Gladman, D. D.; Ibañez, D.; Urowitz, M. B. Systemic Lupus Erythematosus Disease Activity Index 2000. J. Rheumatol. 2002, 29 (2), 288-291.
(38) Isenberg, D. A.; Rahman, A.; Allen, E.; Farewell, V.; Akil, M.; Bruce, I. N.; D'Cruz, D.; Griffiths, B.; Khamashta, M.; Maddison, P.; McHugh, N.; Snaith, M.; Teh, L. S.; Yee, C. S.; Zoma, A.; Gordon, C. BILAG 2004. Development and Initial Validation of an Updated Version of the British Isles Lupus Assessment Group's Disease Activity Index for Patients with Systemic Lupus Erythematosus. Rheumatol. Oxf. Engl. 2005, 44 (7), 902-906. https://doi.org/10.1093/rheumatology/keh624.
(39) Yee, C.-S.; Cresswell, L.; Farewell, V.; Rahman, A.; Teh, L.-S.; Griffiths, B.; Bruce, I. N.; Ahmad, Y.; Prabu, A.; Akil, M.; McHugh, N.; D'Cruz, D.; Khamashta, M. A.; Isenberg, D. A.; Gordon, C. Numerical Scoring for the BILAG-2004 Index. Rheumatol. Oxf. Engl. 2010, 49 (9), 1665-1669. https://doi.org/10.1093/rheumatology/keq026.
(40) Yao, Y.; Higgs, B. W.; Richman, L.; White, B.; Jallal, B. Use of Type I Interferon-Inducible MRNAs as Pharmacodynamic Markers and Potential Diagnostic Markers in Trials with Sifalimumab, an Anti-IFNα Antibody, in Systemic Lupus Erythematosus. Arthritis Res. Ther. 2010, 12 (Suppl 1), S6. https://doi.org/10.1186/ar2887.

## Claims

1. A type I IFN receptor (IFNR) inhibitor for use in a method of treating systemic lupus erythematosus (SLE) in a subject in need thereof, the method comprising administering to the subject a therapeutically effective amount of the type I interferon (IFN) receptor (IFNR) inhibitor, wherein at baseline the subject has a low type I IFN gene signature (IFNGS), wherein the IFNR inhibitor reduces SLE disease activity in the subject, wherein the IFNR inhibitor is anifrolumab,
and wherein the IFNGS of the subject at baseline is measurable by a 4-gene quantitative polymerase chain reaction-based test, wherein the test measures IFI27, IFI44, lFI44L and RSAD2 expression in the whole blood of the subject.

2. The IFNR inhibitor for the use of any preceding claim, wherein the reducing SLE disease activity in the subject comprises a BILAG-Based Composite Lupus Assessment (BICLA) response in the subject.

3. The IFNR for the use of any preceding claim, wherein the subject has moderate to severe SLE.

4. The IFNR inhibitor for the use of claim 5, wherein the method comprises administering a dose of 300 mg of the IFNR inhibitor to the subject.

5. The IFNR inhibitor for the use of claim 5 or 6, comprising administering a dose of the IFNR inhibitor to the subject every 4 weeks.

6. The IFNR inhibitor for the use of any preceding claim, wherein the IFNR inhibitor is administered to the subject intravenously.

7. The IFNR inhibitor for the use of any of claims 1 to 5, wherein the IFNR inhibitor is administered to the subject subcutaneously.

8. The IFNR inhibitor for the use in any preceding claim, wherein the subject is positive for anti-dsDNA, anti-nuclear or anti-Smith antibodies at baseline.

## Patentansprüche

1. Typ-I-IFN-Rezeptor-Hemmer (IFNR-Hemmer) zur Verwendung in einem Verfahren zum Behandeln von systemischem Lupus erythematodes (SLE) in einem Subjekt, das dies benötigt, das Verfahren umfassend ein Verabreichen einer therapeutisch wirksamen Menge des Typ-I-Interferon(IFN)-Rezeptor-Hemmers (IFNR-Hemmer) an das Subjekt, wobei das Subjekt zu Beginn eine niedrige Typ-I-IFN-Gensignatur (IFNGS) aufweist, wobei der IFNR-Hemmer die SLE-Krankheitsaktivität in dem Subjekt verringert, wobei der IFNR-Hemmer Anifrolumab ist,
und wobei die IFNGS des Subjekts zu Beginn durch einen auf der quantitativen 4-Gen-Polymerase-Kettenreaktion basierenden Test messbar ist, wobei der Test die Expression von IFI27, IFI44, IFI44L und RSAD2 in dem Vollblut des Subjekts misst.

2. IFNR-Hemmer zur Verwendung nach einem der vorstehenden Ansprüche, wobei das Verringern der SLE-Krankheitsaktivität in dem Subjekt eine BILAG-Based Composite Lupus Assessment-Reaktion (BICLA-Reaktion) in dem Subjekt umfasst.

3. IFNR zur Verwendung nach einem der vorstehenden Ansprüche, wobei das Subjekt mittelschweren bis schweren SLE aufweist.

4. IFNR-Hemmer zur Verwendung nach Anspruch 5, wobei das Verfahren das Verabreichen einer Dosis von 300 mg des IFNR-Hemmers an das Subjekt umfasst.

5. IFNR-Hemmer zur Verwendung nach Anspruch 5 oder 6, umfassend das Verabreichen einer Dosis des IFNR-Hemmers an das Subjekt alle 4 Wochen.

6. IFNR-Hemmer zur Verwendung nach einem der vorstehenden Ansprüche, wobei der IFNR-Hemmer dem Subjekt intravenös verabreicht wird.

7. IFNR-Hemmer zur Verwendung nach einem der Ansprüche 1 bis 5, wobei der IFNR-Hemmer dem Subjekt subkutan verabreicht wird.

8. IFNR-Hemmer zur Verwendung nach einem der vorstehenden Ansprüche, wobei das Subjekt zu Beginn positiv auf Anti-dsDNA-, Anti-Nuklear- oder Anti-Smith-Antikörper ist.

## Revendications

1. Inhibiteur du récepteur de l'IFN (IFNR) de type I pour une utilisation dans un procédé de traitement du lupus érythémateux disséminé (LED) chez un sujet qui en a besoin, le procédé comprenant l'administration au sujet d'une quantité thérapeutiquement efficace de l'inhibiteur du récepteur de l'interféron (IFN) (IFNR) de type I, dans lequel, au départ, le sujet présente une faible signature génétique de l'IFN (IFNGS) de type I, dans lequel l'inhibiteur de l'IFNR réduit l'activité de la maladie de LED chez le sujet, dans lequel l'inhibiteur de l'IFNR est l'anifrolumab,
et dans lequel l'IFNGS du sujet au départ est mesurable par un test quantitatif d'amplification en chaîne par polymérase à 4 gènes, dans lequel le test mesure l'expression de l'IFI27, de l'IFI44, de l'IF144L et de la RSAD2 dans le sang total du sujet.

2. Inhibiteur de l'IFNR pour l'utilisation selon l'une quelconque revendication précédente, dans lequel la réduction de l'activité de la maladie de LED chez le sujet comprend une réponse de l'évaluation composite du lupus basée sur le BILAG (BICLA) chez le sujet.

3. IFNR pour l'utilisation selon l'une quelconque revendication précédente, dans lequel le sujet est atteint de LED modéré à sévère.

4. Inhibiteur de l'IFNR pour l'utilisation selon la revendication 5, dans lequel le procédé comprend l'administration d'une dose de 300 mg de l'inhibiteur de l'IFNR au sujet.

5. Inhibiteur de l'IFNR pour l'utilisation selon la revendication 5 ou 6, comprenant l'administration d'une dose de l'inhibiteur de l'IFNR au sujet toutes les 4 semaines.

6. Inhibiteur de l'IFNR pour l'utilisation selon l'une quelconque revendication précédente, dans lequel l'inhibiteur de l'IFNR est administré au sujet par voie intraveineuse.

7. Inhibiteur de l'IFNR pour l'utilisation selon l'une quelconque des revendications 1 à 5, dans lequel l'inhibiteur de l'IFNR est administré au sujet par voie sous-cutanée.

8. Inhibiteur de l'IFNR pour l'utilisation selon l'une quelconque revendication précédente, dans lequel le sujet est positif pour les anticorps anti-dsDNA, antinucléaires ou anti-Smith au départ.
